# EUROPÄISCHE PATENTSCHRIFT

(11) **EP 2 552 914 B1**
(45) Veröffentlichungstag und Bekanntmachung des Hinweises auf die Patenterteilung: **11.11.2015**
(21) Anmeldenummer: 11706181.2
(22) Anmeldetag: 28.02.2011
(51) Int. Cl.: C07D 471/04, A61K 31/4375, A61P 35/00

(54) **BENZONAPHTHYRIDINAMINE ALS AUTOTAXIN-INHIBITOREN**
BENZONAPHTHYRIDINAMINES AS AUTOTAXIN INHIBITORS
BENZONAPHTHYRIDINAMINES EN TANT QU'INHIBITEURS D'AUTOTAXINE

(30) Priorität: 26.03.2010 EP 10003282
(43) Veröffentlichungstag der Anmeldung: 06.02.2013
(73) Patentinhaber: Merck Patent GmbH, 64293 Darmstadt (DE)
(72) Erfinder: STAEHLE, Wolfgang, 55218 Ingelheim (DE); SCHULTZ, Melanie, 64287 Darmstadt (DE); SCHIEMANN, Kai, 64342 Seeheim-Jugenheim (DE)
(86) Internationale Anmeldenummer: PCT/EP2011/000964
(87) Internationale Veröffentlichungsnummer: WO 2011/116867

(56) Entgegenhaltungen:
- EP-A1- 0 997 462
- GB-A- 1 186 061
- FRANCO GATTA ET AL: "Synthesis of 10-Amino-1,2,3,4-tetrahydrobenzo[b][1,6]na phthyridines and Related Derivatives", JOURNAL OF HETEROCYCLIC CHEMISTRY, WILEY-BLACKWELL PUBLISHING, INC, US, Bd. 33, 1. Januar 1996 (1996-01-01), Seiten 1807-1814, XP009132640, ISSN: 0022-152X, DOI: DOI:10.1002/JHET.5570330642
- HOEGLUND ADRIENNE B ET AL: "Characterization of non-lipid autotaxin inhibitors.", BIOORGANIC & MEDICINAL CHEMISTRY 15 JAN 2010 LNKD- PUBMED:20005724, Bd. 18, Nr. 2, 15. Januar 2010 (2010-01-15), Seiten 769-776, XP002637116, ISSN: 1464-3391

## Beschreibung

### Technisches Gebiet

Die vorliegende Erfindung betrifft Benzonaphthyridinamine als Autotaxin-Inhibitoren und ihre Verwendung in der Behandlung und/oder Prophylaxe von physiologischen und/oder pathophysiologischen Zuständen, bei denen die Hemmung, Regulierung und/oder Modulation der Phosphodiesterase bzw. Lysophospholipase Autotaxin eine Rolle spielt, insbesondere von verschiedenen Krebsarten sowie Autoimmun- und Entzündungskrankheiten.

### Stand der Technik

Autotaxin (ATX) ist eine Enzym, welches für die Erhöhung des Lysophosphatsäurespiegel in Ascites und Plasma verantwortlich ist (Xu et al., Clinical Cancer Research 1995, 1: 1223 und Xu et al., Biochem. J. 1995, 309: 933). ATX setzt Lysophatidylcholin (LPC) zu Lysophosphatsäure um (Tokumura et al., J. Biol. Chem. 2002, 277: 39436 und Umezu-Gozo et al., J. Biol. Chem. 2002, 158: 227).

LPA ist ein interzellularer Lipid Mediator der eine Vielzahl von biologischen und biochemischen Prozessen wie beispielsweise glatte Muskelkontraktion, Thrombozyten Aggregation und Apoptose beeinflusst (Tigyi et al., Prog. Lipid Res 2003, 42: 498 und Mills et al., Nat. Rev. Cancer 2003, 3: 582 und Lynch et al., Prost. Lipid Med 2001, 64: 33). Außerdem ist LPA in erhöhten Konzentrationen in Plasma und Ascites Flüssigkeit von Ovarial Krebs Patienten der frühen und späten Phase zu finden. LPA spielt dort eine Rolle bei der Tumorzellproliferation und deren Invasion in benachbarte Gewebe, welche zur Metastasierung führen kann (Xu et al., Clinical Cancer Research 1995, 1: 1223 und Xu et al., Biochem. J. Vol. 1995, 309: 933). Diese biologischen und pathobiologischen Prozesse werden durch die Aktivierung durch LPA von G-Protein gekoppelten Rezeptoren angeschaltet (Contos et al., Mol. Pharm. 2000, 58: 1188). Aus diesem Grunde ist es zur Behandlung von Tumor Patienten wünschenswert, den LPA Spiegel zu senken. Dies kann durch die Hemmung von Enzymen erreicht werden, die an der LPA Biosynthese beteiligt sind, wie beispielsweise Autotaxin (ATX, Sano et al., J. Biol. Chem. 2002, 277: 21197 und Aoki et al., J. Biol. Chem. 2003, 277: 48737).

Autotaxin gehört zu der Enzymfamilie der Nukleotide Pyrophosphatasen und Phosphodiesterasen (Goding et al., Immunol. Rev. 1998, 161: 11) und stellt einen wichtigen Ansatzpunkt bei der antitumoralen Therapie dar (Mills et al., Nat. Rev. Cancer 2003, 3: 582 and Goto et al., J. Cell. Biochem. 2004, 92: 1115), da es in Tumoren verstärkte expremiert wird und Tumorzellproliferation und -invasion in benachbarte Gewebe bewirkt, was zur Metastasenbildung führen kann (Nam et al., Oncogene 2000, 19: 241). Außerdem bewirkt Autotaxin zusammen mit anderen angiogenetischen Faktoren Blutgefäßformation im Rahmen der Angiogenese (Nam et al., Cancer Res. 2001, 61: 6938). Angiogenese ist ein wichtiger Vorgang beim Tumorwachstum, der die Versorgung des Tumors mit Nährstoffen sichert. Aus diesem Grunde ist die Hemmung der Angiogenese ein wichtiger Ansatzpunkt der Krebs- und Tumortherapie, mit dem der Tumor gewissermaßen ausgehungert werden kann (Folkman, Nature Reviews Drug Discovery 2007, 6: 273-286).

Verbindungen, die zur Hemmung von Autotaxin fähig sind, sind in Peng et al. (Bioorganic & Medicinal Chemistry Letters 2007, 17: 1634-1640) beschrieben. Die dort beschriebenen Verbindungen stellen Lipid Analoga dar, welche strukturell keine Gemeinsamkeiten mit den erfindungsgemäßen Verbindungen aufweisen.

Weitere Stand der Technik Dokumente sind wie folgt:
US 3,637,706, US 3,647,800 und US 3,674,790 beschreiben Benzonaphthyridin-Derivate, die sich von den erfindungsgemäßen Verbindungen in ihrer Substitution in der Position 2 unterscheiden.
Yamato M et al. (Journal of Medicinal Chemistry 1989, 32(6): 1295-1300) beschreiben Benzonaphthyridin-Derivate, die sich von den erfindungsgemäßen Verbindungen in ihrer Substitution in der Position 2 unterscheiden.
US 4,751,305 und US 4,808,612 beschreiben Benzonaphthyridin-Derivate, die sich von den erfindungsgemäßen Verbindungen in ihrer Substitution in der Position 2 unterscheiden.
US 4,816,464 beschreibt spezifische Benzonaphthyridin-Derivate, die sich von den erfindungsgemäßen Verbindungen in ihrer Substitution in der Position 2 unterscheiden.
Yamato M et al. (Chemical & Pharmaceutical Bulletin 1990, 38(11): 3048-3052 beschreiben Benzonaphthyridin-Derivate, die sich von den erfindungsgemäßen Verbindungen in ihrer Substitution in der Position 2 unterscheiden.
JP 3-218359 beschreibt Benzonaphthyridin-Derivate, die sich von den erfindungsgemäßen Verbindungen in ihrer Substitution in der Position 2 unterscheiden.
Skotnicki JS et al. (Medicinal Chemistry Research 1991, 1(4): 254-252) beschreiben Hydrazin-Benzonaphthyridin-Derivate, die sich von den erfindungsgemäßen Verbindungen in ihrer Substitution in der Position 10 unterscheiden.
Pirrung MC et al. (Chemistry & Biology 1995, 2 (9): 621-626) beschreiben ein Benzonaphthyridin-Derivat, das sich von den erfindungsgemäßen Verbindungen in der Position 2 unterscheidet.
Youssef, Khairia M et al. (Bulletin of the Faculty of Pharmacy (Cairo University) 1995, 33(1): 33-39) und Youssef, Khairia M (AI-Azhar Bulletin of Science 1999, 10(1): 99-112) beschreiben Hydrazin-Benzonaphthyridin-Derivate, die sich von den erfindungsgemäßen Verbindungen in ihrer Substitution in der Position 10 unterscheiden.
Neurartige Naphthyridin-Derivate mit antagonistischen Effekt auf Tachykinin-Rezeptoren sind in der WO 99/00388 beschrieben, die sich von den erfindungsgemäßen Verbindungen in ihrer Substitution in der Position 10 unterscheiden.
WO 2000/035918 beschreibt u.a. Benzonaphthyridin-Derivate als Tachykininrezeptor-Antagonisten, die sich von den erfindungsgemäßen Verbindungen in ihrer Substitution in der Position 2 unterscheiden.
JP 2002-088081 beschreibt u.a. Benzonaphthyridin-Derivate als Tachykininantagonisten, die sich von den erfindungsgemäßen Verbindungen in ihrer Substitution in der Position 2 unterscheiden.
WO 2004/067513 beschreibt u.a. ein Benzonaphthyridin-Derivat (Beispiel 25), das sich aber von den erfindungsgemäßen Verbindungen in der Position 2 unterscheidet.
Die internationale Anmeldung PCT/EP2009/007930 **(**WO 2010/060532**)**, angemeldet am 05.11.2009, beschreibt Benzonaphthyridin-Derivate, die sich von den erfindungsgemäßen Verbindungen in ihrer Substitution in der Position 10 unterscheiden.

Durch die Aufnahme einer Literaturstelle in diese Anmeldung ist nicht ausgesagt, dass die Literaturstelle einen relevanten Stand der Technik für diese Anmeldung darstellt.

### Beschreibung der Erfindung

Aufgabe der vorliegenden Erfindung ist die Bereitstellung von neuen Autotaxin-Inhibitoren.

Die Aufgabe der vorliegenden Erfindung wurde in einem Aspekt überraschend gelöst durch Bereitstellen von Verbindungen der Formel (I) 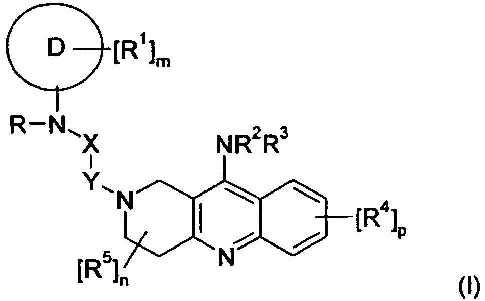 worin:
- D: Ar oder Het bedeutet,
- Ar: unsubstituiertes oder einfach oder mehrfach substituiertes Phenyl, Indanyl, Naphthyl oder Biphenyl bedeutet.
- Het: einen ein- oder zweikernigen gesättigten, partiell ungesättigten oder aromatischen Heterocyclus mit 1, 2, 3 oder 4 N-, O-und/oder S- Atomen, der unsubstituiert oder einfach oder mehrfach substituiert sein kann, bedeutet,
- X, Y: jeweils unabhängig voneinander fehlt, -CH₂-, -(-CH₂)₂-, -C(O)-, -CHOH- oder -CH₂OC(O)- bedeuten, wobei nur einer der Reste X oder Y fehlen darf,
- R: jeweils unabhängig voneinander H, A, Cyc, (CH₂)_{q}Ar oder (CH₂)_{q}Het bedeuten und einfach oder mehrfach mit R⁶ substituiert sein können, wobei bei A und Cyc die C-Kette bzw. der C-Ring auch durch O unterbrochen sein kann,
- R¹: jeweils unabhängig voneinander R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phenyl bedeuten und einfach oder mehrfach mit R⁶ substituiert sein können,
- R², R³: jeweils unabhängig voneinander R bedeuten, wobei R² und R³ alternativ zusammen auch Cyc oder Het bilden können, welche wiederum einfach oder mehrfach mit R⁶ substituiert sein können,
- R⁴: jeweils unabhängig voneinander R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phenyl bedeuten und einfach oder mehrfach mit R⁶ substituiert sein können,
- R⁵: jeweils unabhängig voneinander R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phenyl bedeuten und einfach oder mehrfach mit R⁶ substituiert sein können,
- R⁶: jeweils unabhängig voneinander R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phenyl bedeuten und, sofern eine Substitution chemisch möglich ist, einfach oder mehrfach mit R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phenyl substituiert sein können,
- A: linear oder verzweigtes Alkyl mit 1, 2, 3, 4, 5, 6, 7 oder 8 C-Atomen bedeutet, worin 1, 2, 3, 4, 5, 6 oder 7 H-Atome durch OR, CN, NRR, F und/oder CI ersetzt sein können und/oder worin eine oder zwei nicht-benachbarte CH₂-Gruppen durch O, NH, S, SO, SO₂ und/oder durch CH=CH-Gruppen ersetzt sein können,
- Cyc: cyclisches Alkyl mit 3, 4, 5, 6, 7, 8, 9, 10, 11, 12, 13 oder 14 C-Atomen bedeutet,
- m: 0, 1, 2, 3, 4, oder 5 bedeutet,
- n: 0, 1, 2, oder 3 bedeutet,
- p: 0, 1, 2, 3, oder 4 bedeutet,
- q: 0, 1, oder 2 bedeutet,
sowie ihrer pharmazeutisch verwendbaren Salze, Solvate und Stereoisomere, einschließlich deren Mischungen in allen Verhältnissen.

Die Erfindung betrifft ferner bevorzugte jeweils unabhängige Ausführungsformen von Verbindungen gemäß der Formel (I) wie hier definiert, worin jeweils unabhängig voneinander bedeuten:
Bevorzugte Ausführungsform (A):
   - D: Ar bedeutet,
Bevorzugte Ausführungsform (B):
   - Ar: unsubstituiertes oder einfach oder mehrfach substituiertes Phenyl bedeutet, bevorzugt aber einfach oder mehrfach substituiertes Phenyl bedeutet,
Bevorzugte Ausführungsform (C):
   - X, Y: jeweils unabhängig voneinander -CH₂-, -C(O)- oder - CH₂OC(O)- bedeuten, bevorzugt ist X = -C(O)- und Y = -CH₂-oder -CH₂OC(O)-,
Bevorzugte Ausführungsform (D):
   - R¹: jeweils unabhängig voneinander F, Cl, OA oder OCH₃ bedeuten,
Bevorzugte Ausführungsform (E):
   - R², R³: jeweils unabhängig voneinander H, Ar, Ar einfach substituiert mit OA, Het, Het einfach substituiert mit A, CH₂-Het, A, A einfach substituiert mit OH oder mit NRR oder mit CO-NRR oder mit Het oder mit CO-R bedeuten oder jeweils unabhängig voneinander 1-methyl-piperidin-4-yl, 2-hydroxyethyl, 2-dimethylamino-ethyl, methylcarbamoylmethyl, dimethylcarbamoylmethyl, 1 H-Benzoimidazol-2-ylmethyl, 2-oxo-2-pyrrolidin-1-yl-ethyl, 2-oxo-2-piperidin-1-yl-ethyl, 2-morpholin-4-yl-2-oxo-ethyl, cyclohexylcarbamoylmethyl, 2-(4-methyl-piperazin-1-yl)-2-oxo-ethyl, 2-(4-isopropyl-piperazin-1-yl)-2-oxo-ethyl, diethylcarbamoylmethyl, 2-(4-Benzyl-piperazin-1-yl)-2-oxo-ethyl, 3-oxo-3-piperidin-1-yl-propyl, pyridin-4-yl, pyridin-3-yl, phenyl, pyridin-2-yl oder 4-methoxy-phenyl bedeuten, wobei R² und R³ alternativ zusammen auch 4-(2-hydroxy-ethyl)-piperazin-1-yl bilden können,
Bevorzugte Ausführungsform (F):
   - m: 2 bedeutet,
Bevorzugte Ausführungsform (G):
   - n: 0 bedeutet,
Bevorzugte Ausführungsform (H):
   - p: 0 bedeutet,
Bevorzugte Ausführungsform (I):
   - q: 0 oder 1 bedeutet,
sowie ihre pharmazeutisch verwendbaren Salze, Solvate und Stereoisomere, einschließlich deren Mischungen in allen Verhältnissen.

Die Erfindung betrifft ferner in einer bevorzugten Ausführungsform Verbindungen gemäß Formel (I) wie hier definiert sowie hier dargestellten bevorzugten Ausführungsformen, worin jeweils unabhängig voneinander bedeuten:
- D: Ar bedeutet,
- Ar: unsubstituiertes oder einfach oder mehrfach substituiertes Phenyl bedeutet, bevorzugt aber einfach oder mehrfach substituiertes Phenyl bedeutet,
- X, Y: jeweils unabhängig voneinander -CH₂-, -C(O)- oder - CH₂OC(O)- bedeuten, bevorzugt ist X = -C(O)- und Y = -CH₂-oder -CH₂OC(O)-,
- R¹: jeweils unabhängig voneinander F, Cl, OA oder OCH₃ bedeuten,
- R², R³: jeweils unabhängig voneinander H, Ar, Ar einfach substituiert mit OA, Het, Het einfach substituiert mit A, CH₂-Het, A, A einfach substituiert mit OH oder mit NRR oder mit CO-NRR oder mit Het oder mit CO-R bedeuten oder jeweils unabhängig voneinander 1-methyl-piperidin-4-yl, 2-hydroxyethyl, 2-dimethylamino-ethyl, methylcarbamoylmethyl, dimethylcarbamoylmethyl, 1 H-Benzoimidazol-2-ylmethyl, 2-oxo-2-pyrrolidin-1-yl-ethyl, 2-oxo-2-piperidin-1-yl-ethyl, 2-morpholin-4-yl-2-oxo-ethyl, cyclohexylcarbamoylmethyl, 2-(4-methyl-piperazin-1-yl)-2-oxo-ethyl, 2-(4-isopropyl-piperazin-1-yl)-2-oxo-ethyl, diethylcarbamoylmethyl, 2-(4-Benzyl-piperazin-1-yl)-2-oxo-ethyl, 3-oxo-3-piperidin-1-yl-propyl, pyridin-4-yl, pyridin-3-yl, phenyl, pyridin-2-yl oder 4-methoxy-phenyl bedeuten, wobei R² und R³ alternativ zusammen auch 4-(2-hydroxy-ethyl)-piperazin-1-yl bilden können,
- m: 2 bedeutet,
- n: 0 bedeutet,
- p: 0 bedeutet,
- q: 0 oder 1 bedeutet,
Het, R, R⁶, A und Cyc die hier definierten angegebenen Bedeutungen haben,
sowie ihre pharmazeutisch verwendbaren Salze, Solvate und Stereoisomere, einschließlich deren Mischungen in allen Verhältnissen.

In einem weiteren Aspekt wurde die Aufgabe der Erfindung überraschend gelöst durch Bereitstellen einer Verbindung ausgewählt aus der Gruppe bestehend aus:

| **Compound No.** | **Chemical Structure** | **Chemical Name** |
|---|---|---|
| **1** | 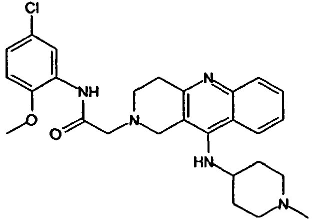 | N-(5-Chloro-2-methoxy-phenyl)-2-[10-(1-methyl-piperidin-4-ylamino)-3,4-dihydro-1H-benzo[b][1,6]naphthyri din-2-yl]-acetamide |
| **2** | 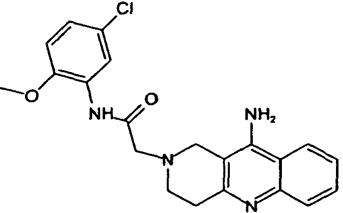 | 2-(10-Amino-3,4-dihydro-1H-benzo[b][1,6]naphthyri din-2-yl)-N-(5-chloro-2-methoxy-phenyl)-acetamide |
| **3** | 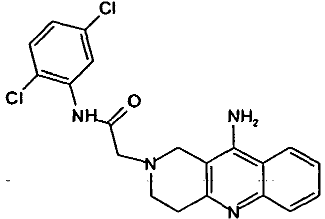 | 2-(10-Amino-3,4-dihydro-1H-benzo[b][1,6]naphthyri din-2-yl)-N-(2,5-dichloro-phenyl)-acetamide |
| **4** | 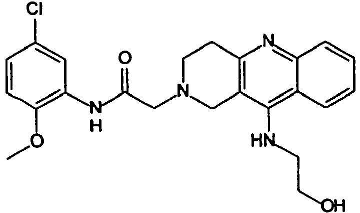 | N-(5-Chloro-2-methoxy-phenyl)-2-[10-(2-hydroxy-ethylamino)-3,4-dihydro-1H-benzo[b][1,6]naphthyri din-2-yl]-acetamide |
| **5** | 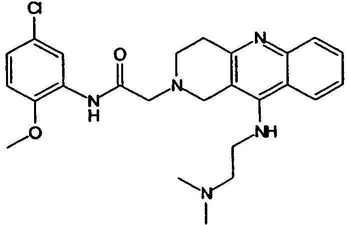 | N-(5-Chloro-2-methoxy-phenyl)-2-[10-(2-dimethylaminoethylamino)-3,4-dihydro-1H-benzo[b][1,6]naphthyri din-2-yl]-acetamide |
| **6** | 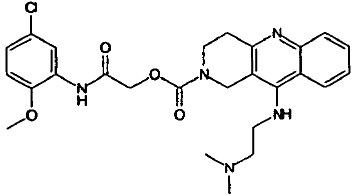 | 10-(2-Dimethylaminoethylamino)-3,4-dihydro-1H-benzo[b][1,6]naphthyri dine-2-carboxylic acid (5-chloro-2-methoxyphenylcarbamoyl)-methyl ester |
| **7** | 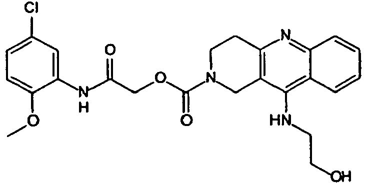 | 10-(2-Hydroxyethylamino)-3,4-dihydro-1H-benzo[b][1,6]naphthyri dine-2-carboxylic acid (5-chloro-2-methoxyphenylcarbamoyl)-methyl ester |
| **8** | 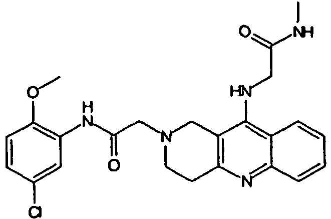 | N-(5-Chloro-2-methoxy-phenyl)-2-[10-(methylcarbamoylmet hyl-amino)-3,4-dihydro-1H-benzo[b][1,6]naphthyri din-2-yl]-acetamide |
| **9** | 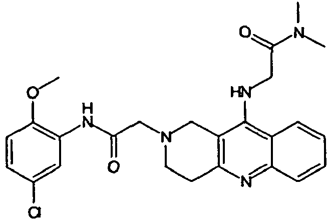 | N-(5-Chloro-2-methoxy-phenyl)-2-[10-(dimethylcarbamoylm ethyl-amino)-3,4-dihydro-1H-benzo[b][1,6]naphthyri din-2-yl]-acetamide |
| **10** | 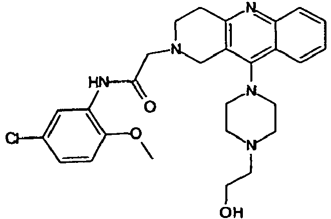 | N-(5-Chloro-2-methoxy-phenyl)-2-{10-[4-(2-hydroxyethyl)-piperazin-1-yl]-3,4-dihydro-1H-benzo[b][1,6]naphthyri din-2-yl}-acetamide |
| **11** | 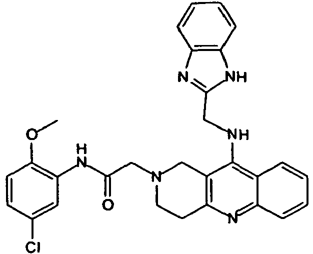 | 2-{10-[(1H-Benzoimidazol-2-ylmethyl)-amino]-3,4-dihydro-1H-benzo[b][1,6]naphthyri din-2-yl}-N-(5-chloro-2-methoxy-phenyl)-acetamide |
| **12** | 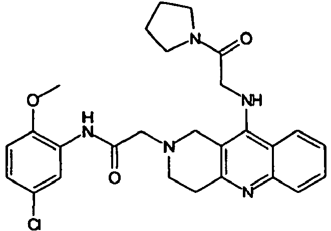 | N-(5-Chloro-2-methoxy-phenyl)-2-[10-(2-oxo-2-pyrrolidin-1-yl-ethylamino)-3,4-dihydro-1H-benzo[b][1,6]naphthyri din-2-yl]-acetamide |
| **13** | 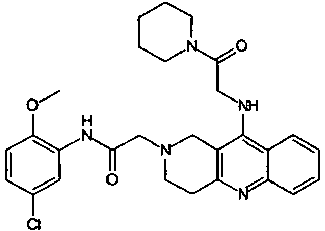 | N-(5-Chloro-2-methoxy-phenyl)-2-[10-(2-oxo-2-piperidin-1-yl-ethylamino)-3,4-dihydro-1H-benzo[b][1,6]naphthyri din-2-yl]-acetamide |
| **14** | 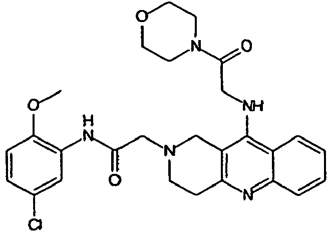 | N-(5-Chloro-2-methoxy-phenyl)-2-[10-(2-morpholin-4-yl-2-oxo-ethylamino)-3,4-dihydro-1H-benzo[b][1,6]naphthyri din-2-yl]-acetamide |
| **15** | 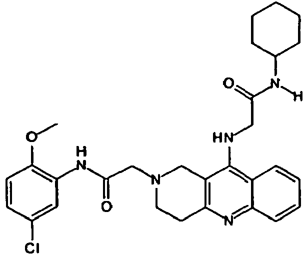 | N-(5-Chloro-2-methoxy-phenyl)-2-[10-(cyclohexylcarbamoyl methyl-amino)-3,4-dihydro-1H-benzo[b][1,6]naphthyri din-2-yl]-acetamide |
| **16** | 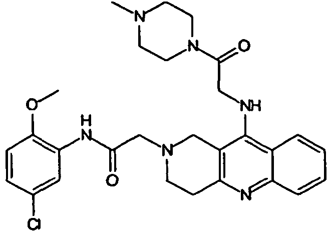 | N-(5-Chloro-2-methoxy-phenyl)-2-{10-[2-(4-methyl-piperazin-1-yl)-2-oxo-ethylamino]-3,4-dihydro-1H-benzo[b][1,6]naphthyri din-2-yl}-acetamide |
| **17** | 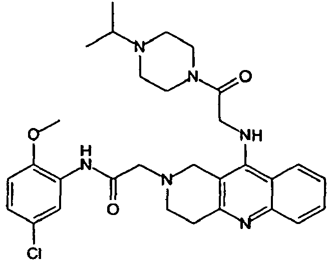 | N-(5-Chloro-2-methoxy-phenyl)-2-{10-[2-(4-isopropyl-piperazin-1-yl)-2-oxo-ethylamino]-3,4-dihydro-1H-benzo[b][1,6]naphthyri din-2-yl}-acetamide |
| **18** | 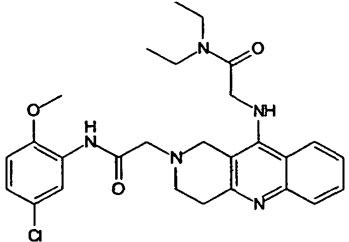 | N-(5-Chloro-2-methoxy-phenyl)-2-[10-(diethylcarbamoylmet hyl-amino)-3,4-dihydro-1H-benzo[b][1,6]naphthyri din-2-yl]-acetamide |
| **19** | 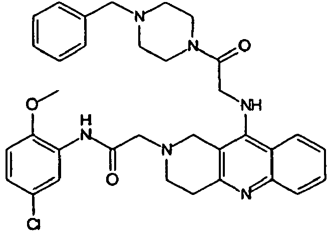 | 2-{10-[2-(4-Benzylpiperazin-1-yl)-2-oxo-ethylamino]-3,4-dihydro-1H-benzo[b][1,6]naphthyri din-2-yl}-N-(5-chloro-2-methoxy-phenyl)-acetamide |
| **20** | 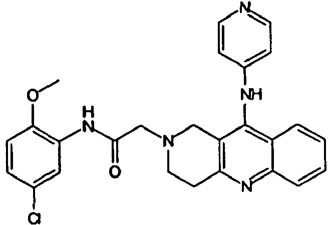 | N-(5-Chloro-2-methoxy-phenyl)-2-[10-(pyridin-4-ylamino)-3,4-dihydro-1H-benzo[b][1,6]naphthyri din-2-yl]-acetamide |
| **21** | 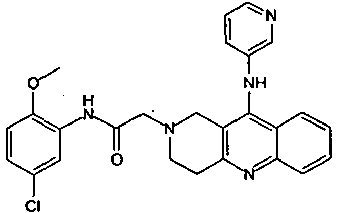 | N-(5-Chloro-2-methoxy-phenyl)-2-[10-(pyridin-3-ylamino)-3,4-dihydro-1H-benzo[b][1,6]naphthyri din-2-yl]-acetamide |
| **22** | 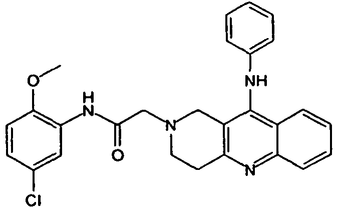 | N-(5-Chloro-2-methoxy-phenyl)-2-(10-phenylamino-3,4-dihydro-1H-benzo[b][1,6]naphthyri din-2-yl)-acetamide |
| **23** | 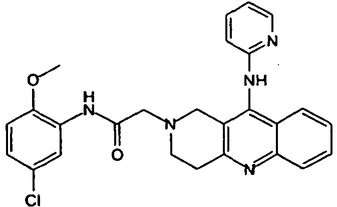 | N-(5-Chloro-2-methoxy-phenyl)-2-[10-(pyridin-2-ylamino)-3,4-dihydro-1H-benzo[b][1,6]naphthyri din-2-yl]-acetamide |
| **24** | 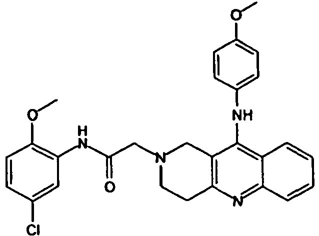 | N-(5-Chloro-2-methoxy-phenyl)-2-[10-(4-methoxy-phenylamino)-3,4-dihydro-1H-benzo[b][1,6]naphthyri din-2-yl]-acetamide |

sowie ihre pharmazeutisch verwendbaren Salze, Solvate und Stereoisomere, einschließlich deren Mischungen in allen Verhältnissen.

Um Zweifel zu vermeiden, gilt, wenn chemische Bezeichnung und chemische Struktur der oben dargestellten Verbindungen versehentlich nicht übereinstimmen, die chemische Struktur als die eindeutige Definition der Verbindung.

Alle der oben generisch oder explizit offenbarten Verbindungen, einschließlich der bevorzugten Untermengen/Ausführungsformen der hier offenbarten Formel (I) und Verbindungen 1 bis 24, werden im Folgenden als Verbindungen der (vorliegenden) Erfindung oder erfindungsgemäße Verbindungen bezeichnet.

Die hier zur Definition von Verbindungen, insbesondere der erfindungsgemäßen Verbindungen, verwendete Nomenklatur stützt sich im Allgemeinen auf die Regeln der IUPAC-Organisation für chemische Verbindungen und insbesondere organische Verbindungen.

Die Ausdrückt, die zur Erläuterung der obigen Verbindungen der Erfindung angegeben sind, besitzen, soweit in der Beschreibung oder den Ansprüchen nicht anders angegeben, stets die folgenden Bedeutungen:
Der Ausdruck "unsubstituiert" bedeutet, dass der entsprechende Rest, die entsprechende Gruppe oder der entsprechende Molekülteil keine Substituenten aufweist.

Der Ausdruck "substituiert" bedeutet, dass der entsprechende Rest, die entsprechende Gruppe oder der entsprechende Molekülteil einen oder mehrere Substituenten aufweist. Weist ein Rest eine Mehrzahl von Substituenten auf, und ist eine Auswahl verschiedener Substituenten angegeben, so sind die Substituenten unabhängig voneinander ausgewählt und brauchen einander nicht gleich zu sein.

Die Ausdrücke "Alkyl" oder "A" sowie andere Gruppen mit der Vorsilbe "Alk" beziehen sich für die Zwecke dieser Erfindung auf acyclische gesättigte oder ungesättigte Kohlenwasserstoffreste, die verzweigt oder geradkettig sein können und vorzugsweise 1 bis 8 Kohlenstoffatome aufweisen, d.h. C₁-C₈-Alkanyle, C₂-C₈-Alkenyle und C₂-C₈-Alkinyle. Alkenyle weisen mindestens eine C-C-Doppelbindung und Alkinyle mindestens eine C-C-Dreifachbindung auf. Alkinyle können zusätzlich auch mindestens eine C-C-Doppelbindung aufweisen. Beispiele geeigneter Alkylreste sind Methyl, Ethyl, n-Propyl, Isopropyl, n-Butyl, Isobutyl, sec-Butyl, tert-Butyl, n-Pentyl, iso-Pentyl, neo-Pentyl, tert-Pentyl, 2- oder 3-Methyl-pentyl, n-Hexyl, 2-Hexyl, Isohexyl, n-Heptyl, n-Octyl, n-Nonyl, n-Decyl, n-Undecyl, n-Dodecyl, n-Tetradecyl, n-Hexadecyl, n-Octadecyl, n-Icosanyl, n-Docosanyl, Ethylenyl (Vinyl), Propenyl (-CH₂CH=CH₂; -CH=CH-CH₃, -C(=CH₂)-CH₃), Butenyl, Pentenyl, Hexenyl, Heptenyl, Octenyl, Octadienyl, Octadecenyl, Octadec-9-enyl, Icosenyl, Icos-11-enyl, (Z)-Icos-11-enyl, Docosenyl, Docos-13-enyl, (Z)-Docos-13-enyl, Ethinyl, Propinyl (-CH₂-C≡CH, -C≡C-CH₃), Butinyl, Pentinyl, Hexinyl, Heptinyl, Octinyl. Besonders bevorzugt ist C₁-₄-Alkyl. Ein C₁-₄-Alkylrest ist beispielsweise ein Methyl, Ethyl, Propyl, Isopropyl, Butyl, Isobutyl, tert-Butyl.

Der Ausdruck "(C₉-C₃₀)Alkyl" bezieht sich für die Zwecke dieser Erfindung auf acyclische gesättigte oder ungesättigte Kohlenwasserstoffreste, die verzweigt oder geradkettig sein können und 9 bis 30 Kohlenstoffatome aufweisen, d.h. C₉-₃₀-Alkanyle, C₉-₃₀-Alkenyle und C₉-₃₀-Alkinyle. C₉-₃₀-Alkenyle weisen mindestens eine C-C-Doppelbindung und C₉-₃₀-Alkinyle mindestens eine C-C-Dreifachbindung auf. C₉-₃₀-Alkinyle können zusätzlich auch mindestens eine C-C-Doppelbindung aufweisen. Beispiele geeigneter (C₉-C₃₀)Alkylreste sind Tetradecyl, Hexadecyl, Octadecyl, Eicosanyl, cis-13-Docosenyl (Erucyl), trans-13-Docosenyl (Brassidyl), cis-15-Tetracosenyl (Nervonyl) und trans-15-Tetracosenyl.

Der Ausdruck "Cycloalkyl" oder "Cyc" bezieht sich für die Zwecke dieser Erfindung auf gesättigte und teilweise ungesättigte nicht aromatische cyclische Kohlenwasserstoffgruppen/-reste, mit 1 bis 3 Ringen, die 3 bis 20, vorzugsweise 3 bis 12, insbesondere bevorzugt 3 bis 8 Kohlenstoffatome, aufweisen. Der Cycloalkylrest kann auch Teil eines bi- oder polycyclischen Systems sein, in dem beispielsweise der Cycloalkylrest über (ein) beliebige(s) mögliche(s) und gewünschte(s) Ringglied(er) mit einem Aryl-, Heteroaryl- oder Heterocyclylrest wie hier definiert kondensiert ist. Die Bindung zu den Verbindungen der allgemeinen Formel kann über jedes beliebige mögliche Ringglied des Cycloalkylrestes erfolgen. Beispiele geeigneter Cycloalkylreste sind Cyclopropyl, Cyclobutyl, Cyclopentyl, Cyclohexyl, Cycloheptyl, Cyclooctyl, Cyclodecyl, Cyclohexenyl, Cyclopentenyl und Cyclooctadienyl. Besonders bevorzugt sind C₃-C₉-Cycloalkyl und C₄-C₈-Cycloalkyl. Bei einem C₄-C₈-Cycloalkylrest handelt es sich z.B. um ein Cyclobutyl, Cyclopentyl, Cyclohexyl, Cycloheptyl, Cyclooctyl.

Der Ausdruck "Heterocyclyl" bezieht sich für die Zwecke dieser Erfindung auf ein mono- oder polycyclisches System mit 3 bis 20, vorzugsweise 5 oder 6 bis 14 Ringatome enthaltend Kohlenstoffatome und 1, 2, 3, 4, oder 5 Heteroatome, insbesondere Stickstoff, Sauerstoff und/oder Schwefel, die gleich oder verschieden sind. Das cyclische System kann gesättigt oder ein- oder mehrfach ungesättigt sein, darf aber nicht aromatisch sein. Bei einem cyclischen System, das aus mindestens zwei Ringen besteht, können die Ringe anelliert oder spiro- oder anderweitig verbunden sein. Derartige "Heterocyclyl"-Reste können über ein beliebiges Ringglied verknüpft sein. Der Ausdruck "Heterocyclyl" umfasst auch Systeme, in denen der Heterocyclus Teil eines bi- oder polycyclischen gesättigten, teilweise ungesättigten und/oder aromatischen Systems ist, wie es der Fall ist, wenn der Heterocyclus über ein beliebiges gewünschtes und mögliches Ringglied des Heterocyclylrestes mit einer "Aryl"-, "Cycloalkyl"-, "Heteroaryl"- oder "Heterocyclyl"-Gruppe wie hier definiert kondensiert ist. Die Bindung zu den Verbindungen der allgemeinen Formel kann über jedes beliebige mögliche Ringglied des Heterocyclylrestes erfolgen. Beispiele geeigneter "Heterocyclyl"-Reste sind Pyrrolidinyl, Thiapyrrolidinyl, Piperidinyl, Piperazinyl, Oxapiperazinyl, Oxapiperidinyl, Oxadiazolyl, Tetrahydrofuryl, Imidazolidinyl, Thiazolidinyl, Tetrahydropyranyl, Morpholinyl, Tetrahydrothiophenyl, Dihydropyranyl, Indolinyl, Indolinylmethyl, Imidazolidinyl, 2-Aza-bicyclo[2.2.2]octanyl.

Der Ausdruck "Aryl" bezieht sich für die Zwecke dieser Erfindung auf ein mono- oder polycyclisches aromatisches Kohlenwasserstoffsystem mit 3 bis 14, vorzugsweise 5 bis 14, besonders bevorzugt 6 bis 10 Kohlenstoffatomen. Der Ausdruck "Aryl" umfasst auch Systeme, in denen der aromatische Cyclus Teil eines bi- oder polycyclischen gesättigten, teilweise ungesättigten und/oder aromatischen Systems ist, wie es der Fall ist, wenn der aromatische Cyclus über ein beliebiges gewünschtes und mögliches Ringglied des Arylrestes mit einer "Aryl"-, "Cycloalkyl"-, "Heteroaryl"- oder "Heterocyclyl"-Gruppe wie hier definiert kondensiert ist. Die Bindung zu den Verbindungen der allgemeinen Formel kann über jedes beliebige mögliche Ringglied des Arylrestes erfolgen. Beispiele geeigneter "Aryl"-Reste sind Phenyl, Biphenyl, Naphthyl, 1-Naphthyl, 2-Naphthyl und Anthracenyl, aber ebenso Indanyl, Indenyl oder 1,2,3,4-Tetrahydronaphthyl. Das insbesondere bevorzugte Aryl ist Phenyl.

Ar bedeutet bevorzugt Phenyl, o-, m- oder p-Tolyl, o-, m- oder p-Ethylphenyl, o-, m- oder p-Propylphenyl, o-, m- oder p-Isopropylphenyl, o-, m-oder p-tert.-Butylphenyl, o-, m- oder p-Hydroxyphenyl, o-, m- oder p-Methoxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonamido)-phenyl, o-, m- oder p-(Methylsulfonyl)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl, 2,5-Dimethyl-4-chlorphenyl, Naphthyl oder Biphenyl.

Ar bedeutet weiterhin vorzugsweise unsubstituiertes oder ein-, zwei-, drei-, vier- oder fünffach durch Hal, A und/oder (CRR)_{q}OR substituiertes Phenyl, Naphthyl oder Biphenyl substituiertes Phenyl, Indanyl, Naphthyl oder Biphenyl.

Der Ausdruck "Heteroaryl" bezieht sich für die Zwecke dieser Erfindung auf einen 3- bis 15-, vorzugsweise 5- bis 14-, besonders bevorzugt 5-, 6-oder 7-gliedrigen mono- oder polycyclischen aromatischen Kohlenwasserstoffrest, der mindestens 1, gegebenenfalls auch 2, 3, 4 oder 5 Heteroatome enthält, vorzugsweise Stickstoff, Sauerstoff und/oder Schwefel, wobei die Heteroatome gleich oder verschieden sind. Die Anzahl der Stickstoffatome beträgt vorzugsweise 0, 1, 2, oder 3 und die der Sauerstoff- und Schwefelatome unabhängig 0 oder 1. Der Ausdruck "Heteroaryl" umfasst auch Systeme, in denen der aromatische Cyclus Teil eines bi- oder polycyclischen gesättigten, teilweise ungesättigten und/oder aromatischen Systems ist, wie es der Fall ist, wenn der aromatische Cyclus über ein beliebiges gewünschtes und mögliches Ringglied des Heteroarylrestes mit einer "Aryl"-, cycloalkyl"-, "Heteroaryl"- oder "Heterocyclyl"-Gruppe wie hier definiert kondensiert ist. Die Bindung zu den Verbindungen der allgemeinen Formel kann über jedes beliebige mögliche Ringglied des Heteroarylrestes erfolgen. Beispiele geeigneter "Heteroaryl"-Reste sind Acridinyl, Benzdioxinyl, Benzimidazolyl, Benzisoxazolyl, Benzodioxolyl, Benzofuranyl, Benzothiadiazolyl, Benzothiazolyl, Benzothienyl, Benzoxazolyl, Carbazolyl, Cinnolinyl, Dibenzofuranyl, Dihydrobenzothienyl, Furanyl, Furazanyl, Furyl, Imidazolyl, Indazolyl, Indolinyl, Indolizinyl, Indolyl, Isobenzylfuranyl, Isoindolyl, Isochinolinyl, Isochinolyl, Isothiazolyl, Isoxazolyl, Naphthyridinyl, Oxadiazolyl, Oxazolyl, Phenazinyl, Phenothiazinyl, Phenoxazinyl, Phthalazinyl, Pteridinyl, Purinyl, Pyrazinyl, Pyrazolyl, Pyridazinyl, Pyridinyl, Pyridyl, Pyrimidinyl, Pyrimidyl, Pyrrolyl, Chinazolinyl, Chinolinyl, Chinolyl, Chinoxalinyl, Tetrazolyl, Thiadiazolyl, Thiazolyl, Thienyl, Thiophenyl, Triazinyl, Triazolyl.

Het bedeutet, ungeachtet weiterer Substitutionen, z.B. 2- oder 3-Furyl, 2- oder 3-Thienyl, 1-, 2- oder 3-Pyrrolyl, 1-, 2, 4- oder 5-lmidazolyl, 1-, 3-, 4-oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3-oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, Indazolyl, 1-, 2-, 4-oder 5-Benzimidazolyl, 1-, 3-, 4-, 5-, 6- oder 7-Benzopyrazolyl, 2-, 4-, 5-, 6-oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl oder Dibenzofuranyl.

Die heterocyclischen Reste können auch teilweise oder vollständig hydriert sein. Ungeachtet weiterer Substitutionen kann Het also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, - 4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3-oder -4-pyrazolyl, 1,4-Dihydro-1-, -2-, -3- oder 4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl, 2,3-Dihydro-2-oxo-furanyl, 3,4-Dihydro-2-oxo-1H-chinazolinyl, 2,3-Dihydrobenzoxazolyl, 2-Oxo-2,3-dihydro-benzoxazolyl, 2,3-Dihydro-benzimidazolyl, 1,3-Dihydroindol, 2-Oxo-1,3-dihydro-indol oder 2-Oxo-2,3-dihydrobenzimidazolyl.

Het bedeutet weiterhin vorzugsweise einen einkernigen gesättigten, ungesättigten oder aromatischen Heterocyclus mit 1 bis 4 N-, O- und/oder S-Atomen, der unsubstituiert oder ein-, zwei- oder dreifach durch A, Ar, (CRR)_{q}Het und/oder (CRR)_{q}OR substituiert sein kann.

Het bedeutet ganz besonders bevorzugt unsubstituiertes oder ein- oder zweifach durch A, Ar, (CRR)qHet und/oder (CRR)_{q}OR substituiertes Piperidinyl, Piperazinyl, Pyrrolidinyl, Morpholinyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl oder Pyrazinyl.

Für die Zwecke der vorliegenden Erfindung geben die Ausdrücke "Alkyl-cycloalkyl", "Cycloalkylalkyl", "Alkyl-heterocyclyl", "Heterocyclylalkyl", "Alkyl-aryl", "Arylalkyl", "Alkyl-heteroaryl" und "Heteroarylalkyl" an, dass Alkyl, Cycloalkyl, Heterocyclyl, Aryl und Heteroaryl jeweils wie hier definiert sind und der Cycloalkyl-, Heterocyclyl-, Aryl- und Heteroarylrest über einen Alkylrest, vorzugsweise C₁-C₈-Alkylrest, besonders bevorzugt C₁-C₄-Alkylrest an die Verbindungen der allgemeinen Formel gebunden ist.

Der Ausdruck "Alkyloxy" oder "Alkoxy" bezieht sich für die Zwecke dieser Erfindung auf einen Alkylrest gemäß der obigen Definition, der an ein Sauerstoffatom gebunden ist. Die Bindung an die Verbindungen der allgemeinen Formel erfolgt über das Sauerstoffatom. Beispiele sind Methoxy, Ethoxy und n-Propyloxy, Propoxy, Isopropoxy. Bevorzugt ist "C₁-C₄-Alkyloxy" mit der angegebenen Anzahl Kohlenstoffatome.

Der Ausdruck "Cycloalkyloxy" oder "Cycloalkoxy" bezieht sich für die Zwecke dieser Erfindung auf einen Cycloalkylrest gemäß der obigen Definition, der an ein Sauerstoffatom gebunden ist. Die Bindung zu den Verbindungen der allgemeinen Formel erfolgt über das Sauerstoffatom. Beispiele sind Cyclopropyloxy, Cyclobutyloxy, Cyclopentyloxy, Cyclohexyloxy, Cycloheptyloxy, Cyclooctyloxy. Bevorzugt ist "C₃-C₉Cycloalkyloxy" mit der angegebenen Anzahl Kohlenstoffatome.

Der Ausdruck "Heterocyclyloxy" bezieht sich für die Zwecke dieser Erfindung auf einen Heterocyclylrest gemäß der obigen Definition, der an ein Sauerstoffatom gebunden ist. Die Bindung zu den Verbindungen der allgemeinen Formel erfolgt über das Sauerstoffatom. Beispiele sind Pyrrolidinyloxy, Thiapyrrolidinyloxy, Piperidinyloxy, Piperazinyloxy.

Der Ausdruck "Aryloxy" bezieht sich für die Zwecke dieser Erfindung auf einen Arylrest gemäß der obigen Definition, der an ein Sauerstoffatom gebunden ist. Die Bindung zu den Verbindungen der allgemeinen Formel erfolgt über das Sauerstoffatom. Beispiele sind Phenyloxy, 2-Naphthyloxy, 1-Naphthyloxy, Biphenyloxy, Indanyloxy. Bevorzugt ist Phenyloxy.

Der Ausdruck "Heteroaryloxy" bezieht sich für die Zwecke dieser Erfindung auf einen Heteroarylrest gemäß der obigen Definition, der an ein Sauerstoffatom gebunden ist. Die Bindung zu den Verbindungen der allgemeinen Formel erfolgt über das Sauerstoffatom. Beispiele sind Pyrrolyloxy, Thienyloxy, Furyloxy, Imidazolyloxy, Thiazolyloxy.

Der Ausdruck "Carbonyl" oder "Carbonylteil" bezieht sich für die Zwecke dieser Erfindung auf eine -C(O)-Gruppe.

Der Ausdruck "Alkylcarbonyl" bezieht sich für die Zwecke dieser Erfindung auf eine "Alkyl-C(O)-"Gruppe, worin Alkyl wie hier definiert ist.

Der Ausdruck "Alkoxycarbonyl" oder "Alkyloxycarbonyl" bezieht sich für die Zwecke dieser Erfindung auf eine "Alkyl-O-C(O)-"Gruppe, worin Alkyl wie hier definiert ist.

Der Ausdruck "Alkoxyalkyl" bezieht sich für die Zwecke dieser Erfindung auf eine "Alkyl-O-alkyl-"Gruppe, worin Alkyl wie hier definiert ist.

Der Ausdruck "Haloalkyl" bezieht sich für die Zwecke dieser Erfindung auf eine Alkylgruppe wie hier definiert, welche mindestens ein mit mindestens einem Halogen wie hier definiert substituiertes Kohlenstoffatom enthält.

Der Ausdruck "Halogen", "Halogenatom", "Halogensubstituent" oder "Hal" bezieht sich für die Zwecke dieser Erfindung auf eine oder gegebenenfalls eine Mehrzahl von Fluor- (F, Fluoro), Brom- (Br, Bromo), Chlor- (Cl, Chloro) oder Iodatomen (I, lodo). Die Bezeichnungen "Dihalogen", "Trihalogen" und "Perhalogen" beziehen sich auf zwei, drei bzw. vier Substituenten, wobei jeder Substituent unabhängig aus der Gruppe bestehend aus Fluor, Chlor, Brom und lod ausgewählt sein kann. "Halogen" bedeutet vorzugsweise ein Fluor-, Chlor- oder Bromatom. Fluor ist insbesondere bevorzugt, wenn die Halogene an einer Alkyl- (Haloalkyl) oder Alkoxygruppe (z.B. CF₃ und CF₃O) substituiert sind.

Der Ausdruck "Hydroxyl" oder "Hydroxy" bezeichnet eine OH-Gruppe.

Der Ausdruck "Zusammensetzung", z.B. in pharmazeutische Zusammensetzung, soll für die Zwecke dieser Erfindung ein Produkt einschließen, das den oder die Wirkstoff(e) und den oder die inerten Inhaltsstoff(e), die den Träger ausmachen, enthält, sowie ein beliebiges Produkt, das direkt oder indirekt aus der Kombination, Komplexierung oder Aggregation von beliebigen zwei oder mehr der Inhaltsstoffe, oder aus der Dissoziation eines oder mehrerer der Inhaltsstoffe, oder aus anderen Arten von Reaktionen oder Interaktionen eines oder mehrerer der Inhaltsstoffe resultiert. Dementsprechend schließen die pharmazeutischen Zusammensetzungen der vorliegenden Erfindung beliebige Zusammensetzungen ein, die durch Mischen einer Verbindung der vorliegenden Erfindung und eines pharmazeutisch unbedenklichen Trägers hergestellt werden.

Die Ausdrücke "Verabreichung von" und "Verabreichen einer" Verbindung sind so zu verstehen, dass eine Verbindung der Erfindung oder ein Prodrug einer Verbindung der Erfindung den individuellen Bedürfnissen zur Verfügung gestellt wird.

So, wie er hier verwendet wird, bedeutet der Ausdruck "wirksame Menge" eine beliebige Menge eines Arzneimittels oder eines pharmazeutischen Mittels, die diejenige biologische oder medizinische Antwort in einem Gewebe, System, Tier oder Menschen hervorruft, die z.B. von einem Forscher oder Mediziner erstrebt wird. Darüber hinaus bedeutet der Ausdruck "therapeutisch wirksame Menge" eine beliebige Menge, die, verglichen zu einem entsprechenden Subjekt, das diese Menge nicht erhalten hat, zu verbesserter Behandlung, Heilung, Vorbeugung oder Verbesserung einer Erkrankung, Störung oder Nebenwirkung, oder zu einer Verminderung des Fortschreitens einer Erkrankung oder Störung führt. Der Ausdruck umfasst in seinem Umfang auch Mengen, die Mengen, die wirkungsvoll sind, die normale physiologische Funktion zu erhöhen.

Verbindungen der Formel (I) bedeuten auch deren pharmazeutisch verwendbare Derivate, optisch aktiven Formen (Stereoisomere), Tautomere, Polymorphe, Enantiomere, Racemate, Diastereomere sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate. Unter pharmazeutisch verwendbaren Derivaten versteht man z.B. die Salze der erfindungsgemäßen Verbindungen als auch sogenannte Prodrug-Verbindungen.

Gegenstand der Erfindung ist auch die Verwendung von Mischungen der Verbindungen der Formel (I), z.B. Gemische zweier Diastereomerer z.B. im Verhältnis 1:1, 1:2, 1:3, 1:4, 1:5, 1:10, 1:100 oder 1:1000. Besonders bevorzugt handelt es sich dabei um Mischungen stereoisomerer Verbindungen.

Alle Stereoisomere der Verbindungen der Erfindung werden in Betracht gezogen, entweder als Mischung oder in reiner oder im Wesentlichen reiner Form. Die Verbindungen der Erfindung können an jedem der Kohlenstoffatome asymmetrische Zentren aufweisen. Infolgedessen können sie in Form ihrer Racemate, in Form der reinen Enantiomere und/oder Diastereomere oder in Form von Mischungen dieser Enantiomere und/oder Diastereomere vorliegen. Die Mischungen können ein beliebiges gewünschtes Mischungsverhältnis der Stereoisomere aufweisen.

So können zum Beispiel die Verbindungen der Erfindung, die ein oder mehrere Chiralitätszentren aufweisen und die als Racemate oder als Diastereomerengemische vorliegen, nach an sich bekannten Methoden in ihre optisch reinen Isomere, d.h. Enantiomere oder Diastereomere, fraktioniert werden. Die Trennung der Verbindungen der Erfindung kann durch Säulentrennung an chiralen oder nicht chiral Phasen oder durch Umkristallisieren aus einem gegebenenfalls optisch aktiven Lösungsmittel oder unter Verwendung einer optisch aktiven Säure oder Base oder durch Derivatisierung mit einem optisch aktiven Reagenz wie zum Beispiel einem optisch aktiven Alkohol und anschließende Eliminierung des Restes erfolgen.

Die Verbindungen der Erfindung können in Form ihrer Doppelbindungsisomere als "reine" E- oder Z-Isomere oder in Form von Mischungen dieser Doppelbindungsisomere vorliegen.

Gegebenenfalls können die Verbindungen der Erfindung in Form der Tautomere, wie Keto-Enol-Tautomere, vorliegen.

Auch ist es möglich, dass die Verbindungen der Erfindung in Form beliebiger gewünschter Prodrugs wie z.B. Ester, Carbonate, Carbamate, Harnstoffe, Amide oder Phosphate, vorliegen, wobei in diesen Fällen die tatsächlich biologisch aktive Form erst durch den Stoffwechsel freigesetzt wird. Jede Verbindung, die in vivo so umgewandelt werden kann, dass sie das bioaktive Mittel (d.h. Verbindungen der Erfindung) bereitstellt, ist ein Prodrug innerhalb des Umfangs und Gedankens der Erfindung.

Verschiedene Formen von Prodrugs sind in der Technik gut bekannt und beispielsweise beschrieben in:
(i) Wermuth CG et al., Kapitel 31: 671-696, The Practice of Medicinal Chemistry, Academic Press 1996;
(ii) Bundgaard H, Design of Prodrugs, Elsevier 1985; und
(iii) Bundgaard H, Kapitel 5: 131-191, A Textbook of Drug Design and Development, Harwood Academic Publishers 1991.

Diese Literaturstellen sind hierin durch Bezugnahme aufgenommen.

Es ist weiterhin bekannt, dass chemische Substanzen im Körper in Metaboliten umgewandelt werden, die gegebenenfalls ebenfalls die gewünschte biologische Wirkung hervorrufen können - unter einigen Umständen sogar in ausgeprägterer Form.

Jede biologisch aktive Verbindung, die in vivo durch Stoffwechsel aus einer beliebigen der Verbindungen der Erfindung umgewandelt wurde, ist ein Metabolit innerhalb des Umfangs und Gedankens der Erfindung.

Die Verbindungen der Erfindung können, wenn sie eine ausreichend basische Gruppe wie z.B. ein sekundäres oder tertiäres Amin aufweisen, mit anorganischen und organischen Säuren in Salze umgewandelt werden. Die pharmazeutisch unbedenklichen Salze der Verbindungen der Erfindung bildet man vorzugsweise mit Salzsäure, Bromwasserstoffsäure, Iodsäure, Schwefelsäure, Phosphorsäure, Methansulfonsäure, p-Toluolsulfonsäure, Kohlensäure, Ameisensäure, Essigsäure, Sulfoessigsäure, Trifluoressigsäure, Oxalsäure, Malonsäure, Maleinsäure, Bernsteinsäure, Weinsäure, Traubensäure, Äpfelsäure, Embonsäure, Mandelsäure, Fumarsäure, Milchsäure, Zitronensäure, Taurocholsäure, Glutarsäure, Stearinsäure, Glutaminsäure oder Asparaginsäure. Die gebildeten Salze sind u.a. Hydrochloride, Chloride, Hydrobromide, Bromide, Iodide, Sulfate, Phosphate, Methansulfonate, Tosylate, Carbonate, Bicarbonate, Formiate, Acetate, Sulfoacetate, Triflate, Oxalate, Malonate, Maleate, Succinate, Tartrate, Malate, Embonate, Mandelate, Fumarate, Lactate, Citrate, Glutarate, Stearate, Aspartate und Glutamate. Die Stöchiometrie der aus den Verbindungen der Erfindung gebildeten Salze kann zudem ein integrales oder nicht integrales Vielfaches von eins sein.

Die Verbindungen der Erfindung können, wenn sie eine ausreichend saure Gruppe wie z.B. die Carboxy-, Sulfonsäure-, Phosphorsäure- oder eine phenolische Gruppe enthalten, mit anorganischen und organischen Basen in ihre physiologisch verwendbaren Salze umgewandelt werden. Beispiele geeigneter anorganischer Basen sind Ammoniak, Natriumhydroxid, Kaliumhydroxid, Calciumhydroxid, und Beispiele geeigneter organischer Basen sind Ethanolamin, Diethanolamin, Triethanolamin, Ethylendiamin, t-Butylamin, t-Octylamin, Dehydroabietylamin, Cyclohexylamin, Dibenzylethylen-diamin und Lysin. Die Stöchiometrie der aus den Verbindungen der Erfindung gebildeten Salze kann zudem ein integrales oder nicht integrales Vielfaches von eins sein.

Auch ist es möglich, dass die Verbindungen der Erfindung in Form ihrer Solvate und insbesondere Hydrate vorliegen, die beispielsweise durch Kristallisieren aus einem Lösungsmittel oder aus wässriger Lösung erhalten werden können. Auch können sich ein, zwei, drei oder eine beliebige Anzahl von Solvat- oder Wassermolekülen an die Verbindungen der Erfindung anlagern, so dass Solvate und Hydrate entstehen.

Mit dem Ausdruck "Solvat" wird ein Hydrat, Alkoholat oder anderes Solvat der Kristallisierung bezeichnet.

Es ist bekannt, dass chemische Substanzen Feststoffe bilden, die in unterschiedlichen Ordnungszustände vorliegen, die als polymorphe Formen oder Modifikationen bezeichnet werden. Die verschiedenen Modifikationen einer polymorphen Substanz können sich in ihren physikalischen Eigenschaften stark unterscheiden. Die Verbindungen der Erfindung können in verschiedenen polymorphen Formen vorliegen und bestimmte Modifikationen können zudem metastabil sein. Alle diese polymorphen Formen der Verbindungen sind als Teil der Erfindung zu betrachten.

Die Verbindungen der Erfindung zeichnen sich überraschend durch eine starke und/oder selektive Inhibition/Hemmung von Autotaxin aus.

Aufgrund ihrer überraschend starken und/oder selektiven Enzymhemmung können die Verbindungen der Erfindung im Vergleich zu anderen weniger wirkungsstarken oder selektiven Inhibitoren des Standes der Technik vorteilhaft in geringeren Dosen verabreicht werden und doch gleichwertige oder sogar bessere gewünschte biologische Wirkungen erzielen. Zusätzlich kann eine solche Herabsetzung der Dosis vorteilhaft zu weniger oder sogar gar keinen unerwünschten medizinischen Wirkungen führen. Weiterhin kann sich die hohe Selektivität der Inhibition der Verbindungen der Erfindung für sich genommen in einer Abnahme unerwünschter Nebenwirkungen äußern, unabhängig von der angewendeten Dosis.

Als Autotaxin-Inhibitoren besitzen die Verbindungen der Erfindung im Allgemeinen eine Inhibitionshemmkonstante IC₅₀ von weniger als etwa 30 µM und vorzugsweise weniger als etwa 10 µM.

Die Ausdrücke "Inhibieren/Hemmen, Inhibition und/oder Verzögerung" sollen sich für die Zwecke der vorliegenden Erfindung auf Folgendes beziehen: "teilweise oder vollständige(s) Inhibieren/Hemmen, Inhibition/Hemmung und/oder Verzögerung". Hierbei liegt es innerhalb des Fachwissens des Durchschnittsfachmanns, ein derartiges Hemmen, eine derartige Hemmung und/oder eine derartige Verzögerung mit den gebräuchlichen Mess- und Bestimmungsmethoden zu messen und zu bestimmen. Teilweise(s) Inhibieren, Inhibition und/oder Verzögerung kann somit beispielsweise im Verhältnis zu völliger oder völligem Inhibieren, Inhibition und/oder Verzögerung gemessen und bestimmt werden.

Die Aufgabe der vorliegenden Erfindung wurde in einem weiteren Aspekt überraschend gelöst durch Bereitstellen eines Verfahrens zur Herstellung einer Verbindung der Erfindung sowie ihrer pharmazeutisch verwendbaren Salze, Solvate und Stereoisomere, dadurch gekennzeichnet, dass man eine Verbindung der Formel (II) 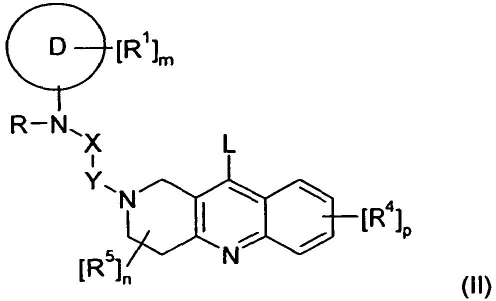 worin D, X, Y, R, R¹, R⁴, R⁵, m, n und p die hier definierten angegebenen Bedeutungen haben und L ein Halogen, Tosylat, Mesylat oder Triflat ist,
mit einer Verbindung der Formel (III)

H-NR²R³ (III)

worin R², R³ die hier definierten angegebenen Bedeutungen haben, umsetzt,
und/oder eine Base oder Säure der resultierenden Verbindungen der Formel (I) wie hier definiert in eines ihrer Salze umwandelt,
oder
dadurch gekennzeichnet, dass man eine Verbindung der Formel (IV) 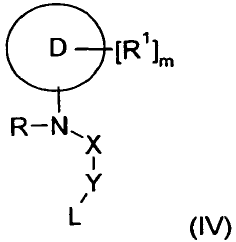 worin D, X, Y, R, R¹ und m die hier definierten angegebenen Bedeutungen haben und L ein Halogen, Tosylat, Mesylat, Triflat oder eine freie oder reaktionsfähig abgewandelte OH-Gruppe, wie z.B. einen aktivierten Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy), darstellt,
mit einer Verbindung der Formel (V) 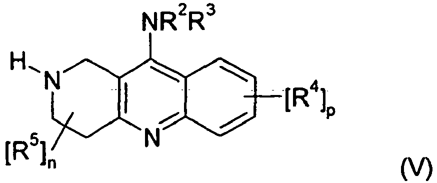 worin R², R³, R⁴, R⁵, n und p die hier definierten angegebenen Bedeutungen haben, umsetzt,
und/oder eine Base oder Säure der resultierenden Verbindungen der Formel (I) wie hier definiert in eines ihrer Salze umwandelt,
oder
dadurch gekennzeichnet, dass man eine Verbindung der Formel (VI) 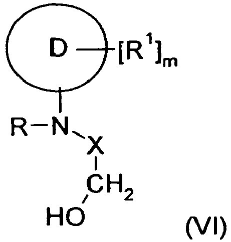 worin D, X, R, R¹ und m die hier definierten angegebenen Bedeutungen haben,
zunächst mit einem Carbonylierungsmittel, bspw.1,1'-Carbonyldiimidazol, Phosgen, Diphosgen, Triphosgen, Harnstoff und Dialkylcarbonat, bevorzugt 1,1'-Carbonyldiimidazol, umsetzt,
und dann mit einer Verbindung der Formel (V) 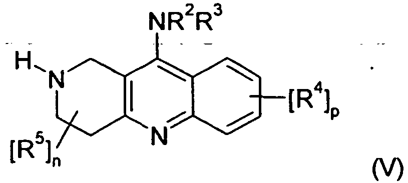 worin R², R³, R⁴, R⁵, n und p die hier definierten angegebenen Bedeutungen haben, reagiert,
und/oder eine Base oder Säure der resultierenden Verbindungen der Formel (I) wie hier definiert in eines ihrer Salze umwandelt.

Eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy): derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben. Aktivierte Ester werden zweckmäßig in situ gebildet, z. B. durch Zusatz von HOBt oder N-Hydroxysuccinimid.

All Rohprodukte wurden einer Standardchromatographie unter Verwendung von Lösungsmittelgemischen mit Methanol, Ethanol, Isopropanol, n-Hexan, Cyclohexan bzw. Petrolether unterworfen.

Eine weitergehende ausführliche Beschreibung der Herstellungsverfahren findet sich auch in den Beispielen und der folgenden allgemeinen Beschreibung der bevorzugten Bedingungen.

Ein physiologisch unbedenkliches Salz einer Verbindung der Erfindung ist auch durch Isolieren und/oder Behandeln der nach der beschriebenen Umsetzung erhaltenen Verbindung der Erfindung mit einer Säure oder Base erhältlich.

Die Verbindungen der Erfindung und auch die Ausgangsstoffe für ihre Herstellung werden nach Verfahren, wie sie in den Beispielen beschrieben sind, oder nach an sich bekannten Verfahren, wie sie in der Literatur (z.B. in Standardwerken wie Houben-Weyl, Methoden der Organischen Chemie, Georg Thieme Verlag, Stuttgart; Organic Reaktions, John Wiley & Sons, Inc., New York), beschrieben sind, hergestellt, und zwar unter Reaktionsbedingungen, die für die genannten Umsetzungen bekannt und geeignet sind. Dabei kann man auch von an sich bekannten, hier aber nicht näher erwähnten Varianten Gebrauch machen.

Die Ausgangsstoffe für das beanspruchte Verfahren können, falls erwünscht, auch in situ gebildet werden, wobei man sie aus dem Reaktionsgemisch nicht isoliert, sondern stattdessen sofort weiter zu den Verbindungen der Erfindung umsetzt. Andererseits ist es möglich, die Umsetzung schrittweise durchzuführen.

Vorzugsweise wird die Umsetzung der Verbindungen in Gegenwart eines geeigneten Lösungsmittels durchgeführt, das vorzugsweise unter den jeweiligen Reaktionsbedingungen inert ist. Beispiele geeigneter Lösungsmittel sind Kohlenwasserstoffe, wie Hexan, Petrolether, Benzol, Toluol oder Xylol; chlorierte Kohlenwasserstoffe, wie Trichlorethylen, 1,2-Dichlorethan, Tetrachlormethan, Chloroform oder Dichlormethan; Alkohole, wie Methanol, Ethanol, Isopropanol, n-Propanol, n-Butanol oder tert-Butanol; Ether, wie Diethylether, Diisopropylether, Tetrahydrofuran (THF) oder Dioxan; Glykolether, wie Ethylenglykolmonomethyl- oder -monoethyl-ether oder Ethylenglykol-dimethylether (Diglyme); Ketone, wie Aceton oder Butanon; Amide, wie Acetamid, Dimethylacetamid, Dimethylformamid (DMF) oder N-Methylpyrrolidinon (NMP); Nitrile, wie Acetonitril; Sulfoxide, wie Dimethylsulfoxid (DMSO); Nitroverbindungen, wie Nitromethan oder Nitrobenzol; Ester, wie Ethylacetat oder Mischungen der genannten Lösungsmittel oder Mischungen mit Wasser. Polare Lösungsmittel sind im Allgemeinen bevorzugt. Beispiele geeigneter polarer Lösungsmittel sind chlorierte Kohlenwasserstoffe, Alkohole, Glykolether, Nitrile, Amide und Sulfoxide oder deren Mischungen. Besonders bevorzugt sind Amide, insbesondere Dimethylformamid (DMF).

Wie oben angegeben, liegt die Reaktionstemperatur je nach Reaktionsschritt und den verwendeten Bedingungen zwischen etwa - 100° C und 300° C.

Die Reaktionszeiten liegen je nach Reaktivität der jeweiligen Verbindungen und den jeweiligen Reaktionsbedingungen im Allgemeinen im Bereich zwischen einigen Minuten und mehreren Tagen. Geeignete Reaktionszeiten lassen sich durch fachbekannte Verfahren, beispielsweise Reaktionsüberwachung, leicht bestimmen. Ausgehend von den oben angegebenen Reaktionstemperaturen liegen geeignete Reaktionszeiten im Allgemeinen im Bereich zwischen 10 min und 48 h.

Die Ausgangsverbindungen der Formeln (II), (III), (IV), (V) und (VI) sind in der Regel bekannt. Sind sie neu, so können sie aber nach an sich bekannten Methoden hergestellt werden. Die Edukte sind im Allgemeinen auch kommerziell erhältlich.

Durch Verwendung einer Säure, beispielsweise durch Umsetzen äquivalenter Mengen der Base und der Säure in einem vorzugsweise inerten Lösungsmittel, wie Ethanol, gefolgt such Verdampfen, kann man eine Base einer Verbindung der Erfindung in das dazugehörige Säureadditionssalz überführen. Geeignete Säuren für diese Reaktion sind insbesondere diejenigen, die physiologisch unbedenklichen Salze liefern. Dementsprechend kann man anorganische Säuren verwenden, zum Beispiel Schwefelsäure, schweflige Säure, Dithionsäure, Salpetersäure, Halogenwasserstoffsäuren, wie Salzsäure oder Bromwasserstoffsäure, Phosphorsäuren, wie z.B. Orthophosphorsäure, Sulfaminsäure, weiterhin organische Säuren, insbesondere aliphatische, alicyclische, araliphatische, aromatische oder heterocyclische monobasische oder polybasische Carbon-, Sulfon- oder Schwefelsäuren, z.B. Ameisensäure, Essigsäure, Propionsäure, Hexansäure, Octansäure, Decansäure, Hexadecansäure, Octadecansäure, Pivalinsäure, Diethylessigsäure, Malonsäure, Bernsteinsäure, Pimelinsäure, Fumarsäure, Maleinsäure, Milchsäure, Weinsäure, Äpfelsäure, Zitronensäure, Gluconsäure, Ascorbinsäure, Nikotinsäure, Isonikotinsäure, Methan- oder Ethansulfonsäure, Ethandisulfonsäure, 2-Hydroxyethansulfonsäure, Benzolsulfonsäure, Trimethoxybenzoesäure, Adamantancarbonsäure, p-Toluolsulfonsäure, Glykolsäure, Embonsäure, Chlorphenoxyessigsäure, Asparaginsäure, Glutaminsäure, Prolin, Glyoxylsäure, Palmitinsäure, Parachlorphenoxyisobuttersäure, Cyclohexancarbonsäure, Glukose-1-phosphat, Naphthalinmono- und -disulfonsäuren oder Laurylschwefelsäure.

Salze mit physiologisch nicht akzeptablen Säuren, z.B. Pikrate, können zur Isolierung und/oder Reinigung der Verbindungen der Erfindung verwendet werden.

Andererseits können Verbindungen der Erfindung unter Verwendung von Basen (z.B. Natriumhydroxid, Kaliumhydroxid, Natriumcarbonat oder Kaliumcarbonat) in die entsprechenden Metallsalze, insbesondere Alkalimetall- oder Erdalkalimetallsalze, oder in die entsprechenden Ammoniumsalze überführt werden. Geeignete Salze sind weiterhin substituierte Ammoniumsalze, z.B. die Dimethyl-, Diethyl- und Diisopropylammoniumsalze, Monoethanol-, Diethanol- und Diisopropanolammoniumsalze, Cyclohexyl- und Dicyclohexylammoniumsalze, Dibenzylethylendiammoniumsalze, daneben beispielsweise Salze mit Arginin oder Lysin.

Gegebenenfalls können die freien Basen der Verbindungen der Erfindung durch Behandeln mit starken Basen, wie Natriumhydroxid, Kaliumhydroxid, Natriumcarbonat oder Kaliumcarbonat aus ihren Salzen freigesetzt werden, solange keine weiteren sauren Gruppen im Molekül vorhanden sind. Bei denjenigen Verbindungen der Erfindung, die freie Säuregruppen aufweisen, kann die Salzbildung auch durch Behandeln mit Basen erfolgen. Geeignete Basen sind Alkalimetallhydroxide, Erdalkalimetallhydroxide oder organische Basen in Form von primären, sekundären oder tertiären Aminen.

Jedem hier beschriebenen Reaktionsschritt können gegebenenfalls ein oder mehrere Aufarbeitungs- und/oder Isolierungsmethoden folgen. Geeignete derartige Methoden sind fachbekannt, beispielsweise aus Standardwerken, wie Houben-Weyl, Methoden der organischen Chemie, Georg-Thieme-Verlag, Stuttgart). Beispiele für derartige Methoden sind z.B. Verdampfen eines Lösungsmittels, Destillieren, Kristallisierung, fraktionierte Kristallisierung, Extraktionsmethoden, Waschmethoden, Aufschlussmethoden, Filtrationsmethoden, Chromatographie, Chromatographie durch HPLC und Trocknungsmethoden, insbesondere Trocknungsmethoden im Vakuum und/oder bei erhöhten Temperaturen, ohne hierauf beschränkt zu sein.

Die Aufgabe der vorliegenden Erfindung wurde in einem weiteren Aspekt überraschend gelöst durch Bereitstellen eines Arzneimittels enthaltend mindestens eine Verbindung der Erfindung und/oder ihrer pharmazeutisch verwendbaren Salze, Solvate und Stereoisomere, einschließlich deren Mischungen in allen Verhältnissen, sowie gegebenenfalls Träger- und/oder Hilfsstoffe.

Die Aufgabe der vorliegenden Erfindung wurde in einem weiteren Aspekt überraschend gelöst durch Bereitstellen eines Arzneimittels enthaltend mindestens eine Verbindung der Erfindung und/oder ihrer pharmazeutisch verwendbaren Salze, Solvate und Stereoisomere, einschließlich deren Mischungen in allen Verhältnissen, zur Verwendung in der Behandlung und/oder Prophylaxe von physiologischen und/oder pathophysiologischen Zuständen, bei denen die Hemmung, Regulierung und/oder Modulation der Phosphodiesterase bzw. Lysophospholipase Autotaxin eine Rolle spielt. Eine entsprechende Verwendung zur Herstellung eines Arzneimittels für die Behandlung und/oder Prophylaxe der vorgenannten Zustände wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung soll hierbei mit umfasst sein.

Die Aufgabe der vorliegenden Erfindung wurde in einem weiteren Aspekt überraschend gelöst durch Bereitstellen eines Arzneimittels enthaltend mindestens eine Verbindung der Erfindung und/oder ihrer pharmazeutisch verwendbaren Salze, Solvate und Stereoisomere, einschließlich deren Mischungen in allen Verhältnissen, zur Verwendung in der Behandlung und/oder Prophylaxe von Krebs, Tumoren, malignen Tumoren, benignen Tumoren, festen Tumoren, Sarkoma, Karzinoma, hyperproliferativen Krankheiten, Karzinoiden, Ewing Sarkoma, Kaposi Sarkoma, Gehirntumoren, Tumoren ausgehend vom Gehirn und/oder Nervensystem und/oder Hirnhäuten, Glioma, Glioblastoma, Neuroblastoma, Magenkrebs, Nierenkrebs, Nierenzellkarzinoma, Prostatakrebs, Prostatakarzinoma, Binde-gewebstumoren, Weichteilsarkoma, Bauchspeicheldrüsentumoren, Lebertumoren, Kopftumoren, Halstumoren, Kehlkopfkrebs, Speiseröhrenkrebs, Schilddrüsenkrebs, Osteosarkoma, Retinoblastoma, Thymusdrüsenkrebs, Hodenkrebs, Lungenkrebs, Lungenadenokarzinoma, kleinzellige Lungenkarzinoma, Bronchialkarzinoma, Brustkrebs, Mammakarzinoma, Darmkrebs, Kolorektaltumoren, Kolonkarzinoma, Rektumkarzinoma, gynäkologischen Tumoren, Eierstocktumoren, Gebärmutterkrebs, Gebärmutterhalskrebs, Gebärmutterhalskarzinoma, Korpuskarzinoma, Endometriumkarzinoma, Harnblasenkrebs, Urogenitaltraktkrebs, Blasenkrebs, Hautkrebs, Epitheltumoren, Plattenepithelkarzinoma, Basalioma, Spinalioma, Melanoma, intraokulären Melanoma, Leukämien, Monozytenleukämien, chronischen Leukämien, chronisch myelotischen Leukämien, chronisch lymphatischen Leukämien, akuten Leukämien, akuten myelotischen Leukämien, akuten lymphatischen Leukämien, Lymphomen, Angiogenese, Arteriosklerose, Atherosklerose, Augenerkrankungen, Uveititis, choroidale Neovascularisation, diabetische Retinopathie, Autoimmunerkrankungen, Entzündungskrankheiten, Asthma, chronisch obstruktive Lungenerkrankung (COPD), chronisch-entzündlichen Darmerkrankungen (Inflammatory Bowel Disease, IBD), Arthritis, Osteoporose, Osteoarthritis, Gicht, Gichtarthritis, Rheumatoide Spondylitis, allergische Rhinitis, Psoriasis, neurodegenerativen Erkrankungen, Restenose, Wundheilung, Transplantatabstoßung, Autoimmunenteropathie, Autoimmunhepatitis, Autoimmun-Polyendokrinopathie-Candidiasis-Ektodermaldystrophie-Syndrom Typ I (APECED), Bullöses Pemphigoid, Chronischer Gastritis, Churg-Strauss-Syndrom, Colitis ulcerosa, Dermatomyositis, Diabetes mellitus Typ 1, Dermatitis herpetiformis Duhring, Epidermolysis bullosa acquisita, Glomerulonephritis, Goodpasture-Syndrom, Guillain-Barre-Syndrom, Hashimoto-Thyreoiditis, Lichen sclerosus, Linearer IgA-Dermatose, Lupus erythematodes, Mikroskopischer Polyangiitis, Morbus Adamantiades-Behçet, Morbus Basedow, Morbus Bechterew, Morbus Crohn, Multipler Sklerose, Myasthenia gravis, PANDAS (Pediatric Autoimmune Neuropsychiatric Disorders Associated with Streptococcal Infections), entzündlicher Becken-Krankheit (pelvic inflammatory disease, PID), Pemphigus foliaceus, Pemphigus seborrhoicus, Pemphigus vulgaris, Polychondritis, Polymyositis, Rheumatischem Fieber, Rheumatoider Arthritis, SAPHO-Syndrom, Sarkoidose (Morbus Boeck), Sjögren-Syndrom, Sklerodermie, Stiff-Man-Syndrom, Sympathischer Ophthalmie, Systemischen Lupus erythematodes, Vasculitis allergica, Vitiligo, Wegenerschen Granulomatose und/oder Zöliakie. Eine entsprechende Verwendung zur Herstellung eines Arzneimittels für die Behandlung und/oder Prophylaxe der vorgenannten Leiden wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung soll inbegriffen sein.

Verbindungen der Erfindung können zusammen mit einer oder mehreren weiteren Wirkstoffen (Inhaltsstoffen, Arzneimitteln) in der Behandlung, Prävention, Unterdrückung oder Besserung von Krankheiten oder Leiden, für die Verbindungen der Erfindung oder die weiteren Substanzen Nutzen haben, verwendet werden. Typischerweise ist die Kombination der Arzneimittel sicherer oder wirksamer als das jeweilige Arzneimittel alleine, oder die Kombination ist sicherer oder wirksamer als auf der Grundlage der additive Eigenschaften der einzelnen Arzneimittel zu erwarten wäre. Ein derartiges weiteres oder derartige weitere Arzneimittel können über einen Weg und in einer Menge, wie sie üblicherweise verwendet werden, gleichzeitig oder nacheinander mit einer Verbindung der Erfindung verabreicht werden. Wird eine Verbindung der Erfindung gleichzeitig mit einem oder mehreren weiteren Arzneimitteln verwendet, so genießt ein Kombinationsprodukt, das (ein) derartige(s) weitere(s) Arzneimittel und die Verbindung der Erfindung enthält, den Vorzug. Kombinationstherapie schließt jedoch auch Therapien ein, bei denen die Verbindung der Erfindung und ein oder mehrere weitere Arzneimittel nach unterschiedlichen, einander überlappenden Zeitplänen verabreicht werden. Es wird in Betracht gezogen, dass bei Verwendung in Kombination mit anderen Wirkstoffen die Verbindung der vorliegenden Erfindung oder der weitere Wirkstoff oder beide effektiv in niedrigeren Dosen verwendet werden können als wenn jedes allein verwendet wird. Daher umfassen die pharmazeutischen Zusammensetzungen der vorliegenden Erfindung solche, die neben einer Verbindung der Erfindung einen oder mehrere weitere Wirkstoffe enthalten.

Zu den Beispielen anderer Wirkstoffen (Inhaltsstoffe, Medikamente), die zusammen mit einer Verbindung der Erfindung verabreicht werden können und entweder getrennt oder in der gleichen pharmazeutischen Zusammensetzung verabreicht werden können, zählen die in Tabelle 1 aufgeführten Verbindungsklassen und spezifischen Verbindungen, ohne hierauf beschränkt zu sein:

| **Tabelle 1** | | |
|---|---|---|
| Alkylierungsmittel | Cyclophosphamid Busulfan | Lomustin Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (AeternaZentaris) |
| | Carboxyphthalatoplatinum | |
| | Tetraplatin | Satraplatin (Johnson Matthey) |
| | Ormiplatin | |
| | Iproplatin | BBR-3464 (Hoffrnann-La Roche) |
| | | SM-11355 (Sumitomo) |
| | | AP-5280 (Access) |
| | | |
| Antimetabolite | Azacytidin | Tomudex |
| | Gemcitabin | Trimetrexat |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche) |
| | Idatrexat | |
| | | Ethinylcytidin (Taiho ) |
| | | |
| Topoisomerase-Hemmer | Amsacrin | Rubitecan (SuperGen) |
| | Epirubicin | Exatecanmesylat (Daiichi) |
| | Etoposid | Quinamed (ChemGenex) |
| | Teniposid oder | Gimatecan (Sigma-Tau) |
| | Mitoxantron | Diflomotecan (Beaufour-Ipsen) |
| | Irinotecan (CPT-11) | |
| | 7-Ethyl-10-hydroxycamptothecin | TAS-103 (Taiho) |
| | | Elsamitrucin (Spectrum) |
| | Topotecan | J-107088 (Merck & Co) |
| | Dexrazoxanet (TopoTarget) | BNP-1350 (BioNumerik) |
| | | CKD-602 (Chong Kun Dang) |
| | Pixantron (Novuspharrna) | |
| | Rebeccamycin-Analogon (Exelixis) | KW-2170 (Kyowa Hakko) |
| | BBR-3576 (Novuspharrna) | |
| | | |
| Antitumor-Antibiotika | Dactinomycin (Actinomycin D) | Amonafid |
| | | Azonafid |
| | Doxorubicin (Adriamycin) | Anthrapyrazol |
| | Deoxyrubicin | Oxantrazol |
| | Valrubicin | Losoxantron |
| | Daunorubicin (Daunomycin) | Bleomycinsulfat (Blenoxan) |
| | Epirubicin | Bleomycinsäure |
| | Therarubicin | Bleomycin A |
| | Idarubicin | Bleomycin B |
| | Rubidazon | Mitomycin C |
| | Plicamycinp | MEN-10755 (Menarini) |
| | Porfiromycin | GPX-100 (Gem Pharmaceuticals) |
| | Cyanomorpholinodoxorubicin | |
| | Mitoxantron (Novantron) | |
| | | |
| Antimitotische Mittel | Paclitaxel | SB 408075 (GlaxoSmithKline) |
| | Docetaxel | |
| | Colchicin | E7010 (Abbott) |
| | Vinblastin | PG-TXL (Cell |
| | Vincristin | Therapeutics) |
| | Vinorelbin | IDN 5109 (Bayer) |
| | Vindesin | A 105972 (Abbott) |
| | Dolastatin 10 (NCI) | A 204197 (Abbott) |
| | Rhizoxin (Fujisawa) | LU 223651 (BASF) |
| | Mivobulin (Warner-Lambert) | D 24851 (ASTA Medica) |
| | | ER-86526 (Eisai) |
| | Cemadotin (BASF) | Combretastatin A4 (BMS) |
| | RPR 109881A (Aventis) | Isohomohalichondrin-B (PharmaMar) |
| | TXD 258 (Aventis) | |
| | Epothilon B (Novartis) | ZD 6126 (AstraZeneca) |
| | T 900607 (Tularik) | PEG-Paclitaxel (Enzon) |
| | T 138067 (Tularik) | AZ10992 (Asahi) |
| | Cryptophycin 52 (Eli Lilly) | IDN-5109 (Indena) |
| | Vinflunin (Fabre) | AVLB (Prescient NeuroPharma) |
| | Auristatin PE (Teikoku Hormone) | |
| | | Azaepothilon B (BMS) |
| | BMS 247550 (BMS) | BNP- 7787 (BioNumerik) |
| | BMS 184476 (BMS) | CA-4-Prodrugs (OXiGENE) |
| | BMS 188797 (BMS) | Dolastatin-10 (NrH) |
| | Taxoprexin (Protarqa) | CA-4 (OXiGENE) |
| | | |
| Aromatase-Hemmer | Aminoglutethimid Letrozol | Exemestan Atamestan (BioMedicines) |
| | Anastrazol Formestan | YM-511 (Yamanouchi) |
| | | |
| Thymidylatsyntha se-Hemmer | Pemetrexed (Eli Lilly) | Nolatrexed (Eximias) |
| | ZD-9331 (BTG) | CoFactor™ (BioKeys) |
| | | |
| DNA-Antagonisten | Trabectedin (PharmaMar) | Mafosfamid (Baxter International) |
| | Glufosfamid (Baxter International) | |
| | | Apaziquon (Spectrum Pharmaceuticals) |
| | Albumin + 32P (Isotope Solutions) | |
| | | O6-Benzylguanin (Paligent) |
| | Thymectacin (NewBiotics) | |
| | Edotreotid (Novartis) | |
| | | |
| Farnesyltransfera se-Hemmer | Arglabin (NuOncology Labs) | Tipifarnib (Johnson & Johnson) |
| | lonafarnib (Schering-Plough) | Perillylalkohol (DOR BioPharma) |
| | BAY-43-9006 (Bayer) | |
| | | |
| Pumpen-Hemmer | CBT-1 (CBA Pharma) | Zosuquidar-Trihydrochlorid (Eli Lilly) |
| | Tariquidar (Xenova) | |
| | MS-209 (Schering AG) | Biricodar-Dicitrat (Vertex) |
| | | |
| Histonacetyltransferase-Hemmer | Tacedinalin (Pfizer) | Pivaloyloxymethylbutyrat (Titan) |
| | SAHA (Aton Pharma) | |
| | MS-275 (Schering AG) | Depsipeptid (Fujisawa) |
| | | |
| Metalloproteinase-Hemmer Ribonucleosidreduktase-Hemmer | Neovastat (Aeterna Laboratories) | CMT -3 (CollaGenex) |
| | | BMS-275291 (Celltech) |
| | Marimastat (British Biotech) | Tezacitabin (Aventis) |
| | | Didox (Molecules for Health) |
| | Galliummaltolat (Titan) | |
| | Triapin (Vion) | |
| | | |
| TNF-alpha-Agonisten/ Antagonisten | Virulizin (Lorus Therapeutics) | Revimid (Celgene) |
| | CDC-394 (Celgene) | |
| | | |
| Endothelin-A-Rezeptor-Antagonisten | Atrasentan (Abbot) | YM-598 (Yamanouchi) |
| | ZD-4054 (AstraZeneca) | |
| | | |
| Retinsäurerezepto r-Agonisten | Fenretinid (Johnson & Johnson) | Alitretinoin (Ligand) |
| | LGD-1550 (Ligand) | |
| | | |
| Immunmodulatoren | Interferon | Dexosom-Therapie (Anosys) |
| | Oncophage (Antigenics) | |
| | GMK (Progenics) | Pentrix (Australian Cancer Technology) |
| | Adenokarzinom-Impfstoff (Biomira) | |
| | | JSF-154 (Tragen) |
| | CTP-37 (AVI BioPharma) | Krebsimpfstoff (Intercell) |
| | JRX-2 (Immuno-Rx) | Norelin (Biostar) |
| | PEP-005 (Peplin Biotech) | BLP-25 (Biomira) |
| | Synchrovax-Impfstoff (CTL Immuno) | MGV (Progenics) |
| | | β-Alethin (Dovetail) |
| | Melanom-Impfstoff (CTL Immuno) | CLL-Thera (Vasogen) |
| | p21-RAS-Impfstoff (GemVax) | |
| | | |
| Hormonelle und antihormonelle Mittel | Östrogene | Prednison |
| | konjugierte Östrogene | Methylprednisolon |
| | Ethinylöstradiol | Prednisolon |
| | Chlortrianisen | Aminoglutethimid |
| | Dienestrol | Leuprolid |
| | Hydroxyprogesteroncaproat | Goserelin |
| | | Leuporelin |
| | Medroxyprogesteron | Centrorelix |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol (EntreMed) |
| | Toremofin | |
| | Dexamethason | Arzoxifen (Eli Lilly) |
| | | |
| Photodynamische Mittel | Talaporfin (Light Sciences) | Pd-Bacteriopheophorbid (Yeda) |
| | Theralux (Theratechnologies) | |
| | | Lutetium-Texaphyrin (Pharmacyclics) |
| | Motexafin-Gadolinium (Pharmacyclics) | |
| | | Hypericin |
| | | |
| Tyrosinkinase-Hemmer | Imatinib (Novartis) | Kahalid F (PharmaMar) |
| | Leflunomid (Sugen/Pharmacia) | CEP-701 (Cephalon) |
| | | CEP-751 (Cephalon) |
| | ZD1839 (AstraZeneca) | MLN518 (Millenium) |
| | Erlotinib (Oncogene Science) | PKC412 (Novartis) |
| | | Phenoxodiol O |
| | Canertjnib (Pfizer) | Trastuzumab (Genentech) |
| | Squalamin (Genaera) | C225 (ImClone) |
| | SU5416 (Pharmacia) | rhu-Mab (Genentech) |
| | SU6668 (Pharmacia) | MDX-H210 (Medarex) |
| | ZD4190 (AstraZeneca) | 2C4 (Genentech) |
| | ZD6474 (AstraZeneca) | MDX-447 (Medarex) |
| | Vatalanib (Novartis) | ABX-EGF (Abgenix) |
| | PKI166 (Novartis) | IMC-1C11 (ImClone) |
| | GW2016 (GlaxoSmithKline) | |
| | EKB-509 (Wyeth) | |
| | EKB-569 (Wyeth) | |
| Verschiedene Mittel | SR-27897 (CCK-A-Hemmer, Sanofi-Synthelabo) | BCX-1777 (PNP-Hemmer, BioCryst) |
| | | Ranpirnase (Ribonuclease-Stimulans, Alfacell) |
| | Tocladesin (cyclisches-AMP-Agonist, Ribapharm) | |
| | Alvocidib (CDK-Hemmer, Aventis) | Galarubicin (RNA-Synthese-Hemmer, Dong-A) |
| | CV-247 (COX-2-Hemmer, Ivy Medical) | |
| | | Tirapazamin (Reduktionsmittel, SRI International) |
| | P54 (COX-2-Hemmer, Phytopharm) | |
| | CapCell™ (CYP450-Stimulans, Bavarian Nordic) | N-Acetylcystein (Reduktionsmittel, Zambon) |
| | GCS-IOO (gal3-Antagonist, GlycoGenesys) | R-Flurbiprofen (NFkappaB-Hemmer, Encore) |
| | | 3CPA (NF-kappaB-Hemmer, Active Biotech) |
| | G17DT-Immunogen (Gastrin-Hemmer, Aphton) | |
| | | Seocalcitol (Vitamin-D-Rezeptor-Agonist, Leo) |
| | Efaproxiral (Oxygenator, Allos Therapeutics) | |
| | | 131-I-TM-601 (DNA-Antagonist, TransMolecular) |
| | PI-88 (Heparanase-Hemmer, Progen) | |
| | Tesmilifen (Histamin-Antagonist, YM BioSciences) | Eflornithin (ODC-Hemmer, ILEX Oncology) |
| | | Minodronsäure (Osteoclasten-Hemmer, Yamanouchi) |
| | Histamin (Histamin-H2-Rezeptor-Agonist, Maxim) | |
| | Tiazofurin (IMPDH-Hemmer, Ribapharm) | Indisulam (p53-Stimulans, Eisai) |
| | Cilengitid (Integrin-Antagonist, Merck KGaA) | Aplidin (PPT-Hemmer, PharmaMar) |
| | SR-31747 (IL-1-Antagonist, Sanofi-Synthelabo) | Rituximab (CD20-Antikörper, Genentech) |
| | | Gemtuzumab (CD33-Antikörper, Wyeth Ayerst) |
| | CCI-779 (mTOR-Kinase-Hemmer, Wyeth) | |
| | Exisulind (PDE-V-Hemmer, Cell Pathways) | PG2 (Hämatopoese-Verstärker, Pharmagenesis) |
| | CP-461 (PDE-V-Hemmer, Cell Pathways) | Immunol™ (Triclosan-Oralspülung, Endo) |
| | AG-2037 (GART-Hemmer, Pfizer) | Triacetyluridin (Uridin-Prodrug, Wellstat) |
| | WX-UK1 (Plasminogenaktivator-Hemmer, Wilex) | SN-4071 (Sarkom-Mittel, Signature BioScience) |
| | | TransMID-107™ (Immunotoxin, KS Biomedix) |
| | PBI-1402 (PMN-Stimulans, ProMetic LifeSciences) | |
| | Bortezomib (Proteasom-Hemmer, Millennium) | PCK-3145 (Apoptose-Förderer, Procyon) |
| | SRL-172 (T-Zell-Stimulans, SR Pharma) | Doranidazol (Apoptose-Förderer, Pola) |
| | TLK-286 (Glutathion-S-Transferase-Hemmer, Telik) | CHS-828 (cytotoxisches Mittel, Leo) |
| | | trans-Retinsäure (Differentiator, NIH) |
| | PT-100 (Wachstumsfaktor-Agonist, Point Therapeutics) | |
| | | MX6 (Apoptose-Förderer, MAXIA) |
| | Midostaurin (PKC-Hemmer, Novartis) | Apomin (Apoptose-Förderer, ILEX Oncology) |
| | Bryostatin-1 (PKC-Stimulans, GPC Biotech) | Urocidin (Apoptose-Förderer, Bioniche) |
| | CDA-II (Apoptose-Förderer, Everlife) | Ro-31-7453 (Apoptose-Förderer, La Roche) |
| | SDX-101 (Apoptose-Förderer, Salmedix) | Brostallicin (Apoptose-Förderer, Pharmacia) |
| | Ceflatonin (Apoptose-Förderer, ChemGenex) | |

In einer bevorzugten Ausführungsform wird eine Verbindung der Erfindung gemeinsam mit einem oder mehreren bekannten Antitumormitteln, wie den folgenden, verabreicht: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer, Angiogenesehemmer.

Die Verbindungen der Erfindung eignen sich insbesondere zur Verabreichung in Kombination mit Radiotherapie. Die synergistischen Wirkungen der Hemmung des VEGF in Kombination mit Radiotherapie sind dem Fachmann bekannt (WO 00/61186).

Der Ausdruck "Östrogenrezeptormodulatoren" im Verlauf der vorliegenden Erfindung bezieht sich auf Verbindungen, die die Bindung von Östrogen an den Östrogenrezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]phenyl]-2H-1-benzopyran-3-yl]phenyl-2,2-dimethylpropanoat, 4,4'-Dihydroxybenzo-phenon-2,4-dinitrophenylhydrazon und SH646, was jedoch keine Einschränkung darstellen soll.

Der Ausdruck "Androgenrezeptormodulatoren" im Verlauf der vorliegenden Erfindung bezieht sich auf Verbindungen, die die Bindung von Androgenen an den Androgenrezeptor stören oder diese hemmen, und zwar unabhängig davon, wie dies geschieht. Zu den Androgenrezeptormodulatoren zählen zum Beispiel Finasterid und andere 5alpha-Reduktase-Hemmer, Nilutamid, Flutamid, Bicalutamid, Liarozol und Abirateron-acetat, was jedoch keine Einschränkung darstellen soll.

Der Ausdruck "Retinoidrezeptormodulatoren" im Verlauf der vorliegenden Erfindung bezieht sich auf Verbindungen, die die Bindung von Retinoiden an den Retinoidrezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, alpha-Difluormethylornithin, ILX23-7553, trans-N-(4'-Hydroxyphenyl)retinamid und N-4-Carboxyphenylretinamid, was jedoch keine Einschränkung darstellen soll.
Der Ausdruck "Zytotoxika" im Verlauf der vorliegenden Erfindung bezieht sich auf Verbindungen, die in erster Linie durch direkte Einwirkung auf die Zellfunktion(en) zum Zelltod führen oder die die Zellmyose hemmen oder diese stören, wie Alkylierungsmittel, Tumornekrosefaktoren, interkaliernde Mittel, Mikrotubulin-Hemmer und Topoisomerase-Hemmer. Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis-[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyl-daunorubicin (WO 00/50032), was jedoch keine Einschränkung darstellen soll.

Zu den Mikrotubulin-Hemmern zählen zum Beispiel Paclitaxel, Vindesin-sulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-Dimethyl-L-valyl-L-valyl-N-methyl-L-valyl-L-prolyl-L-prolin-t-butylamid, TDX258 und BMS188797, was jedoch keine Einschränkung darstellen soll.

Zu den Topoisomerase-Hemmern zählen zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNP1100, BN80915, BN80942, Etoposidphosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5α,5aβ,8aα,9β)-9-[2-[N-[2-(Dimethylamino)ethyl]-N-methylamino]ethyl]-5-[4-hydroxy-3,5-dimethoxy-phenyl]-5,5a,6,8,8a,9-hexahydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)-amino]benzo[g]isochinolin-5,10-dion, 5-(3-Aminopropylamino)-7,10-dihydroxy-2-(2-hydroxyethylaminomethyl)-6H-pyrazolo[4,5,1-de]acridin-6-on, N-[1-[2-(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna, was jedoch keine Einschränkung darstellen soll.

Zu den "antiproliferativen Mitteln" zählen zum Beispiel Antisense-RNA-und -DNA-Oligonukleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-hepto-pyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Fluoruracil, Alanosin, 11-Acetyl-8-(carbamoyloxymethyl)-4-formyl-6-methoxy-14-oxa-1,11-diazatetracyclo-(7.4.1.0.0)-tetradeca-2,4,6-trien-9-ylessigsäureester, Swainsonin, Lometrexol, Dexrazoxan, Methioninase. 2'-Cyan-2'-desoxy-N4-palmitoyl-1-B-D-arabinofuranosylcytosin und 3-Aminopyridin-2-carboxaldehyd-thiosemi-carbazon, was jedoch keine Einschränkung darstellen soll.

Die "antiproliferativen Mittel" beinhalten auch monoklonale Antikörper gegen Wachstumsfaktoren, die nicht unter den "Angiogenese-Hemmern" aufgeführt wurden, wie Trastuzumab, sowie Tumorsuppressorgene, wie p53.

In einem weiteren Aspekt der Erfindung wird ein Arzneimittel gemäß den obigen Aspekten und bevorzugten Ausführungsformen bereitgestellt, wobei ein derartiges Arzneimittel mindestens eine zusätzliche pharmakologisch aktive Substanz (Medikament, Inhaltsstoff) umfasst.

In einer bevorzugten Ausführungsform handelt es sich bei der mindestens einen zusätzlichen pharmakologisch aktiven Substanz um eine Substanz wie hier beschrieben.

In einem weiteren Aspekt der Erfindung wird ein Arzneimittel gemäß den obigen Aspekten und Ausführungsformen bereitgestellt, wobei das Arzneimittel vor und/oder während und/oder nach der Behandlung mit mindestens einer zusätzlichen pharmakologisch aktiven Substanz verabreicht wird.

In einer bevorzugten Ausführungsform handelt es sich bei der mindestens einen zusätzlichen pharmakologisch aktiven Substanz um eine Substanz wie hier beschrieben.

In einem weiteren Aspekt der Erfindung wird eine pharmazeutische Zusammensetzung umfassend eine therapeutisch wirksame Menge mindestens einer Verbindung der Erfindung bereitgestellt.

In einer bevorzugten Ausführungsform umfasst die pharmazeutische Zusammensetzung mindestens eine zusätzliche Verbindung ausgewählt aus der Gruppe bestehend aus physiologisch verträglichen Träger- und Hilfsstoffen und/oder zusätzlicher pharmakologisch aktiver Substanz außer den Verbindungen der Erfindung.

In einem weiteren Aspekt der Erfindung wird eine pharmazeutische Zusammensetzung offenbart, die mindestens eine Verbindung der Erfindung, mindestens eine pharmakologisch aktive Substanz außer den Verbindungen der Erfindung wie hier beschrieben und einen physiologisch verträglichen Träger- und/oder Hilfsstoff umfasst.

Eine weitere Ausführungsform der vorliegenden Erfindung ist ein Verfahren zur Herstellung der pharmazeutischen Zusammensetzungen, dadurch gekennzeichnet, dass man eine oder mehrere erfindungsgemäße Verbindungen und eine oder mehrere Verbindungen ausgewählt aus der Gruppe bestehend aus festen, flüssigen oder halbflüssigen Streckmitteln, Hilfsstoffen, Zusatzstoffen, Verdünnern, Trägerstoffen und pharmakologisch aktiver Substanzen außer den erfindungsgemäßen Verbindungen in eine geeignete Darreichungsform umwandelt.

In einem weiteren Aspekt der Erfindung wird ein Kit bereitgestellt umfassend eine therapeutisch wirksame Menge mindestens einer Verbindung der Erfindung und/oder mindestens eine pharmazeutische Zusammensetzung wie hier beschrieben und eine therapeutisch wirksame Menge mindestens einer weiteren pharmakologisch aktiven Substanz außer den Verbindungen der Erfindung.

Die pharmazeutischen Zusammensetzungen der vorliegenden Erfindung können auf jede Weise verabreicht werden, durch die der angestrebte Zweck erreicht wird. Beispielsweise kann die Verabreichung auf oralem, parenteralem, topischem, enteralem, intravenösem, intramuskulärem, inhalierendem, nasalem, intraartikulärem, intraspinalem, transtrachealem, transokularem, subkutanem, intraperitonealem, transdermalem oder bukkalem Wege erfolgen. Alternativ oder gleichzeitig kann die Verabreichung auf oralem Wege erfolgen. Die verabreichte Dosis hängt vom Alter, Gesundheitszustand und Gewicht des Empfängers, der Art einer eventuellen gleichzeitigen Behandlung, der Behandlungshäufigkeit und der Art der gewünschten Wirkung ab. Eine parenterale Verabreichung ist bevorzugt. Eine orale Verabreichung ist besonders bevorzugt.

Zu den geeigneten Darreichungsformen zählen, ohne hierauf beschränkt zu sein, Kapseln, Tabletten, Pellets, Dragees, halbfeste Stoffe, Pulver/Puder, Granulate, Zäpfchen, Salben, Cremes, Lotionen, Inhalate, Injektionen, Kataplasmen, Gele, Band, Augentropfen, Lösung, Sirupe, Aerosole, Suspension, Emulsion, die nach im Stand der Technik bekannten Verfahren hergestellt werden können, beispielsweise wie unten beschrieben:
Tabletten: Mischen des Wirkstoffs/der Wirkstoffe und Hilfsstoffe, Verpressen der Mischung zu Tabletten (direkte Verpressung), gegebenenfalls Granulieren eines Teils der Mischung vor dem Verpressen.
Kapseln: Mischen des Wirkstoffs/der Wirkstoffe und Hilfsstoffe zu einem rieselfähigen Pulver, gegebenenfalls Granulieren des Pulvers, Einfüllen von Pulvern/Granulaten in geöffnete Kapseln, Verschließen der Kapseln.
Halbfeste Stoffe (Salben, Gele, Cremes): Lösen/Dispergieren des Wirkstoffs/der Wirkstoffe in einem wässrigen oder Fett-Trägerstoff; anschließendes Mischen der Wasser-/Fettphase mit entsprechender Fett-/Wasserphase, Homogenisieren (nur Cremes).
Zäpfchen (rektal und vaginal): Lösen/Dispergieren des Wirkstoffs/der Wirkstoffe im durch Wärme verflüssigten Trägermaterial (rektal: Trägermaterial normalerweise ein Wachs; vaginal: Trägerstoff normalerweise eine erhitzte Lösung eines Geliermittels), Gießen der Mischung in Zäpfchenformen, Verfestigen und Entnehmen der Zäpfchen aus den Formen.
Aerosole: Lösen/Dispergieren des Wirkmittels/der Wirkmittel in einem Treibgas, Abfüllen der Mischung in einen Zerstäuber.

Im Allgemeinen umfassen nicht chemische Wege für die Herstellung pharmazeutischer Zusammensetzungen und/oder pharmazeutischer Zubereitungen Verarbeitungsschritte auf geeigneten, im Stand der Technik bekannten mechanischen Mitteln, die eine oder mehrere Verbindungen der Erfindung in eine Darreichungsform überführen, die für die Verabreichung an einen Patienten geeignet ist, der einer solchen Behandlung bedarf. Üblicherweise umfasst die Überführung einer oder mehrerer Verbindungen der Erfindung in eine derartige Darreichungsform die Zugabe einer oder mehrerer Verbindungen, ausgewählt aus der Gruppe bestehend aus Trägerstoffen, Streckmitteln, Hilfsstoffen und pharmazeutischen Wirkstoffen außer den Verbindungen der Erfindung. Geeignete Verfahrensschritte sind, ohne hierauf beschränkt zu sein, Vermengen, Mahlen, Mischen, Granulieren, Lösen, Dispergieren, Homogenisieren, Gießen und/oder Verpressen der jeweiligen aktiven und nicht aktiven Inhaltsstoffe. Mechanische Mittel für die Durchführung der genannten Verarbeitungsschritte sind im Stand der Technik bekannt, z.B. aus Ullmann's Encyclopedia of Industrial Chemistry, 5. Auflage. Hierbei sind Wirkstoffe vorzugsweise mindestens eine Verbindung der Erfindung und eine oder mehrere zusätzliche Verbindungen außer den Verbindungen der Erfindung, die wertvolle pharmazeutische Eigenschaften aufweisen, vorzugsweise solche pharmazeutischen Wirkmittel außer den Verbindungen der Erfindung, die hier offenbart sind.

Besonders geeignet für die orale Anwendung sind Tabletten, Pillen, Dragees, Kapseln, Pulver, Granulate, Sirupe, Säfte oder Tropfen, für die rektale Anwendung Zäpfchen, für die parenterale Anwendung Lösungen, vorzugsweise ölige oder wässrige Lösungen, weiterhin Suspensionen, Emulsionen oder Implantate, und für die topische Verwendung Salben, Cremes oder Puder. Die Verbindungen der Erfindung können auch lyophilisiert und die erhaltenen Lyophilisate beispielsweise für die Herstellung von Injektionszubereitungen verwendet werden. Die angegebenen Zubereitungen können sterilisiert werden und/oder Hilfsmittel enthalten, wie Gleitmittel, Konservierungsstoffe, Stabilisatoren und/oder Netzmittel, Emulgatoren, Salze zur Änderung des osmotischen Drucks, Puffersubstanzen, Farbstoffe, Geschmacksstoffe und/oder eine Vielzahl weiterer Wirkstoffe, z.B. ein oder mehrere Vitamine.

Geeignete Streckmittel sind organische oder anorganische Substanzen, die sich für die enterale (z.B. orale), parenteral oder topische Verabreichung eignen und mit den Verbindungen der Erfindung nicht reagieren, beispielsweise Wasser, Pflanzenöle, Benzylalkohole, Alkylenglykole, Polyethylenglykole, Glycerintriacetat, Gelatine, Kohlenhydrate, wie Laktose, Saccharose, Mannit, Sorbit oder Stärke (Maisstärke, Weizenstärke, Reisstärke, Kartoffelstärke), Cellulosezubereitungen und/oder Calciumphosphate, z.B. Tricalciumphosphat oder Calciumhydrogenphosphat, Magnesiumstearat, Talkum, Tragant, Methylcellulose, Hydroxypropylmethylcellulose, Natriumcarboxymethylcellulose, Polyvinylpyrrolidon und/oder Vaseline.

Gegebenenfalls können Sprengmittel zugegeben werden, wie die oben genannten Stärken und auch Carboxymethylstärke, vernetztes Polyvinylpyrrolidon, Agar oder Algininsäure oder ein Salz davon, wie Natriumalginat. Hilfsstoffe umfassen, ohne Beschränkung, Fließ- und Gleitmittel, z.B. Kieselsäure, Talkum, Stearinsäure oder deren Salze, wie Magnesiumstearat oder Calciumstearat, und/oder Polyethylenglykol. Drageekerne werden mit geeigneten Überzügen versehen, die gegebenenfalls magensaftresistent sind. Zu diesem Zweck können konzentrierte Saccharidlöungen verwendet werden, die gegebenenfalls Gummi arabicum, Talkum, Polyvinylpyrrolidon, Polyethylenglykol und/oder Titandioxid, Lacklösungen und geeignete organische Lösungsmittel oder Lösungsmittelgemische enthalten können. Zur Herstellung von magensaftresistenten Überzügen oder zur Bereitstellung einer Darreichungsform mit dem Vorteil verlängerter Wirkung kann die Tablette, Dragee oder Pille eine innere und eine äußere Dosiskomponente enthalten, wobei letztere die Form einer Umhüllung um erstere hat. Die beiden Komponenten können durch eine magensaftresistente Schicht getrennt sein, die einem Zerfall im Magen entgegensteht und es erlaubt, dass die innere Komponente intakt in den Zwölffingerdarm gelangt oder in ihrer Freigabe verzögert wird. Für derartige magensaftresistente Schichten oder Überzüge kann eine Vielfalt von Materialien verwendet werden, wobei derartige Materialien eine Anzahl von polymeren Säuren und Mischungen von polymeren Säuren mit solchen Materialien wie Schellack, Acetylalkohol, Lösungen von geeigneten Cellulosezubereitungen wie Acetyl-cellulosephthalat, Celluloseacetat oder Hydroxypropylmethylcellulosephthalat umfassen. Die Tabletten oder Drageeüberzüge können mit Farbstoffen oder Pigmenten versetzt werden, beispielsweise zur Identifizierung oder um Kombinationen von Dosen aktiver Verbindungen zu kennzeichnen.

Geeignete Trägersubstanzen sind organische oder anorganische Substanzen, die sich für die enterale (z.B. orale) oder parenterale Verabreichung oder topische Anwendung eignen und mit den neuen Verbindungen nicht reagieren, zum Beispiel Wasser, Pflanzenöle, Benzylalkohole, Polyethylenglykole, Gelatine, Kohlenhydrate wie Laktose oder Stärke, Magnesiumstearat, Talkum und Petrolatum. Insbesondere verwendet man für die enterale Verabreichung Tabletten, Dragees, Kapseln, Sirupe, Suspensionen, Tropfen oder Zäpfchen, für die parenterale Verabreichung Lösungen, vorzugsweise ölige oder wässrige Lösungen, weiterhin Suspensionen, Emulsionen oder Implantate, und für die topische Anwendung Salben, Cremes oder Puder. Die Verbindungen der Erfindung können auch lyophilisiert und die erhaltenen Lyophilisate beispielsweise für die Herstellung von Injektionszubereitungen verwendet werden.

Die angegebenen Zubereitungen können sterilisiert werden und/oder Streckmittel enthalten, wie Gleitmittel, Konservierungsstoffe, Stabilisatoren und/oder Netzmittel, Emulgatoren, Salze zur Änderung des osmotischen Drucks, Puffersubstanzen, Farbstoffe, Geschmacksstoffe und/oder Aromatisierungsmittel. Sie können gegebenenfalls auch eine oder mehrere weitere aktive Verbindungen enthalten, z.B. ein oder mehrere Vitamine.

Weitere pharmazeutische Zubereitungen, die oral verwendet werden können, sind u.a. Steckkapseln aus Gelatine, sowie weiche, versiegelte Kapseln aus Gelatine und einem Plastifiziermittel wie Glycerin oder Sorbit. Die Steckkapseln können die aktiven Verbindungen in Form von Granulat enthalten, das mit Füllstoffen wie Laktose, Bindemitteln wie Stärken und/oder Gleitmitteln wie Talkum oder Magnesiumstearat und gegebenenfalls Stabilisatoren vermischt sein kann. In Weichkapseln sind die aktiven Verbindungen vorzugsweise in geeigneten Flüssigkeiten, wie fetten Ölen oder flüssigem Paraffin gelöst oder suspendiert. Außerdem können Stabilisatoren zugegeben werden.

Zu den flüssigen Formen, in die die neuen Zusammensetzungen der vorliegenden Erfindung für die orale Verabreichung eingearbeitet werden können, zählen wässrige Lösungen, geeignet aromatisierte Sirupe, wässrige oder Ölsuspensionen und aromatisierte Emulsionen mit Speiseölen wie Baumwollsaatöl, Sesamöl, Kokosöl oder Erdnussöl, sowie Elixire und ähnliche pharmazeutische Vehikel. Zu den geeigneten Dispergier- oder Suspendiermitteln für wässrige Suspensionen zählen synthetische und natürliche Gummis wie Tragant, Gummi arabicum, Alginat, Dextran, Natriumcarboxymethylcellulose, Methylcellulose, Polyvinylpyrrolidon oder Gelatine.

Zu den geeigneten Formulierungen für die parenterale Verabreichung zählen wässrige Lösungen der aktiven Verbindungen in wasserlöslicher Form, beispielsweise wasserlösliche Salze und alkalische Lösungen. Zusätzlich können Suspensionen der aktiven Verbindungen als geeignete ölige Injektionssuspensionen verabreicht werden. Zu den geeigneten lipophilen Lösungsmitteln oder Vehikeln zählen fette Öle, z.B. Sesamöl, oder synthetische Fettsäureester, z.B. Ethyloleat oder Triglyceride oder Polyethylenglykol-400 (die Verbindungen sind in PEG-400 löslich).

Wässrige Injektionssuspensionen können Substanzen enthalten, die die Viskosität der Suspension erhöhen, darunter beispielsweise Natriumcarboxymethylcellulose, Sorbit und/oder Dextran, gegebenenfalls kann die Suspension auch Stabilisatoren enthalten.

Für die Verabreichung als Inhalationsspray ist die Verwendung von Sprays möglich, in denen der Wirkstoff in einem Treibgas oder Treibgasgemisch (z.B. CO₂ oder Fluorchlorkohlenwasserstoffe) entweder gelöst oder suspendiert ist. Der Wirkstoff wird hier vorzugsweise in mikronisierter Form verwendet, wobei ein oder mehrere zusätzliche physiologisch unbedenkliche Lösungsmittel vorhanden sein können, z.B. Ethanol. Inhalationslösungen können mit Hilfe herkömmlicher Inhalatoren verabreicht werden.

Zu den möglichen pharmazeutischen Zubereitungen, die rektal verwendet werden können, zählen beispielsweise Zäpfchen, die aus einer Kombination einer oder mehrerer der aktiven Verbindungen mit einer Zäpfchengrundlage bestehen. Geeignete Zäpfchengrundlagen sind zum Beispiel natürliche oder synthetische Triglyceride oder Paraffinkohlenwasserstoffe. Daneben ist es auch möglich, Gelatine-Rektalkapseln zu verwenden, die aus einer Kombination der aktiven Verbindungen mit einer Grundlage bestehen. Zu den möglichen Grundlagenmaterialien zählen z.B. flüssige Triglyceride, Polyethylenglykole oder Paraffinkohlenwasserstoffe.

Für die medizinische Verwendung liegen die Verbindungen der vorliegenden Erfindung in Form pharmazeutisch unbedenklicher Salze vor. Andere Salze können jedoch bei der Herstellung der Verbindungen der Erfindung oder ihrer pharmazeutisch unbedenklichen Salze von Nutzen sein. Zu den geeigneten pharmazeutisch unbedenklichen Salzen der Verbindungen dieser Erfindung zählen Säureadditionssalze, die beispielsweise durch Mischen einer Lösung der erfindungsgemäßen Verbindung mit einer Lösung einer pharmazeutisch unbedenklichen Säure wie Salzsäure, Schwefelsäure, Methansulfonsäure, Fumarsäure, Maleinsäure, Bernsteinsäure, Essigsäure, Benzoesäure, Oxalsäure, Zitronensäure, Weinsäure, Kohlensäure oder Phosphorsäure gebildet werden können. Wenn die Verbindungen der Erfindung einen sauren Teil tragen, können zu deren geeigneten pharmazeutisch unbedenklichen Salzen weiterhin Alkalimetallsalze, z.B. Natrium- oder Kaliumsalze; Erdalkalimetallsalze, z.B. Calcium- oder Magnesiumsalze; und mit geeigneten organischen Basen, z.B. quaternären Ammoniumsalzen, gebildete Salze zählen.

Die pharmazeutischen Zubereitungen können als Arzneimittel in der Human- und Veterinärmedizin eingesetzt werden. Wie hier verwendet, bedeutet der Ausdruck "wirksame Menge" diejenige Menge eines Arzneimittels oder pharmazeutischen Mittels, die diejenige biologische oder medizinische Antwort in einem Gewebe, System, Tier oder Menschen hervorruft, die z.B. von einem Forscher oder Mediziner erstrebt wird. Darüber hinaus bezeichnet der Ausdruck "therapeutisch wirksame Menge" eine beliebige Menge, die im Vergleich zu einem entsprechenden Subjekt, das eine solche Menge nicht erhalten hat, zur verbesserter Behandlung, Heilung, Vorbeugung oder Verbesserung einer Erkrankung, Störung oder Nebenwirkung, oder zu einer Verminderung des Fortschreitens einer Erkrankung oder Störung führt. Der Ausdruck umfasst in seinem Umfang auch Mengen, die eine Erhöhung normaler physiologischer Funktionen bewirken. Die therapeutisch wirksame Menge einer oder mehrerer Verbindungen der Erfindung ist dem Fachmann bekannt oder kann nach fachbekannten Standardmethoden leicht bestimmt werden.

Die Verbindungen der Erfindung und die zusätzlichen Aktivsubstanzen werden im Allgemeinen analog zu handelsüblichen Zubereitungen verabreicht. Gewöhnlich liegen geeignete Dosen, die therapeutisch wirksam sind, im Bereich zwischen 0,0005 mg und 1000 mg, vorzugsweise zwischen 0,005 mg und 500 mg und insbesondere zwischen 0,5 mg und 100 mg pro Dosierungseinheit. Die tägliche Dosis liegt vorzugsweise zwischen etwa 0,001 mg/kg und 10 mg/kg Körpergewicht.

Dem Fachmann wird deutlich sein, dass Dosierungsmengen in Abhängigkeit von der spezifischen Verbindung, der Schwere der Symptome und der Empfindlichkeit des Subjekts gegenüber Nebenwirkungen variieren können. Einige der spezifischen Verbindungen besitzen eine stärkere Wirkung als andere. Der Fachmann kann bevorzugte Dosierungen für eine gegebene Verbindung leicht auf verschiedene Arten bestimmen. Eine bevorzugte Art besteht darin, die physiologische Wirkungsstärke einer gegebenen Verbindung zu messen.

Für die Zwecke der vorliegenden Erfindung werden alle Säugetierspezies als umfasst betrachtet. In eine bevorzugten Ausführungsform sind derartige Säugetiere ausgewählt aus der Gruppe bestehend aus "Primaten, Menschen, Nagetieren, Pferden, Rindern, Hundeartigen, Katzen, domestizierten Tieren, Zuchtvieh, Vieh, Haustieren, Kuh, Schaf, Schwein, Ziege, Pferd, Pony, Esel, Maulesel, Maultier, Hase, Kaninchen, Katze, Hund, Meerschweinchen, Hamster, Ratte, Maus". Besonders bevorzugt handelt es sich bei diesen Säugetieren um Menschen. Tiermodelle sind für experimentelle Untersuchungen interessant und bieten ein Modell für die Behandlung menschlicher Krankheiten.

Die spezifische Dosis für den einzelnen Patienten hängt jedoch von der Vielzahl von Faktoren ab, beispielsweise von der Wirksamkeit der spezifischen eingesetzten Verbindungen, vom Alter, Körpergewicht, allgemeinen Gesundheitszustand, Geschlecht, von der Ernährung, vom Verabreichungszeitpunkt und -weg, von der Ausscheidungsgeschwindigkeit, der Verabreichungsart und der zu verabreichenden Darreichungsform, der pharmazeutische Kombination und Schwere der genauen Störung, der die Therapie gilt. Die jeweilige therapeutisch wirksame Dosis für den jeweiligen Patienten kann leicht durch Routineversuche bestimmt werden, zum Beispiel durch den Arzt, der zu dieser therapeutischen Behandlung rät bzw. sie begleitet.

Bei vielen Störungen lässt sich die Empfindlichkeit einer bestimmten Zelle für eine Behandlung mit den betreffenden Verbindungen durch Invitro-Tests bestimmen. Typisch wird eine Kultur der Zelle mit der betreffenden Verbindung in verschiedenen Konzentrationen kombiniert, und zwar für einen Zeitraum, der es den aktiven Mitteln ermöglicht, eine relevante Reaktion zeigen, üblicherweise zwischen einer Stunde und einer Woche. Für In-vitro-Tests können kultivierte Zellen aus einer Biopsieprobe verwendet werden.

Auch ohne weitere Ausführungen wird davon ausgegangen, dass ein Fachmann die obige Beschreibung im weitesten Umfang nutzen kann. Die bevorzugten Ausführungsformen sind deswegen lediglich als beschreibende Offenbarung aufzufassen, die keineswegs in irgendeiner Weise limitierend ist.

Vor- und nachstehend sind alle Temperaturen in °C angegeben. In den nachstehenden Beispielen bedeutet "übliche Aufarbeitung": Man entfernt, falls erforderlich, das Lösungsmittel, gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, wäscht die organische Phase mit gesättigter NaHCO₃-Lösung, gegebenenfalls mit Wasser und gesättigter NaCl-Lösung, trocknet die organische Phase über Natriumsulfat, filtriert, engt ein und reinigt durch Chromatographie an Kieselgel, durch präparative HPLC und/oder durch Kristallisation. Die gereinigten Verbindungen werden gegebenenfalls gefriergetrocknet.

### Liste der Abkürzungen und Akronyme:

AcOH Essigsäure, wf. wasserfrei, atm Atmosphäre(n), BOC tert-Butoxycarbonyl CDI 1,1'-Carbonyldiimidazol, konz. konzentriert, d Tag(e), Zers. Zersetzung, DMAC NN-Dimethylacetamid, DMPU 1,3-Dimethyl-3,4,5,6-tetrahydro-2(1H)-pyrimidinon, DMF NN-Dimethylformamid, DMSO Dimethylsulfoxid, DPPA Diphenylphosphorylazid, EDCI 1-(3-Dimethylaminopropyl)-3-ethylcarbodiimid, EtOAc Ethylacetat, EtOH Ethanol (100%), Et₂O Diethylether, Et₃N Triethylamin, h Stunde(n), MeOH Methanol, Pet.-Ether Petrolether (Siedebereich 30-60°C), Temp. Temperatur, THF Tetrahydrofuran, TFA TrifluorAcOH, Tf Trifluormethansulfonyl.

Der Inhalt aller zitierter Literaturstellen wird hierbei durch Bezugnahme in Gänze aufgenommen. Die Erfindung wird durch die folgenden Beispiele näher erläutert, ohne jedoch hierauf beschränkt zu sein.

### Beispiele

### I. Synthese ausgewählter Verbindungen der Erfindung

Die folgenden Verbindungen wurden synthetisiert und charakterisiert. Es gehört jedoch zu den Kenntnissen des Fachmanns, diese Verbindungen auf andere Weise herzustellen und zu charakterisieren.

### Beispiel 1

### Synthese von N-(5-Chlor-2-methoxy-phenyl)-2-[10-(2-oxo-2-pyrrolidin-1-yl-ethylamin)-3,4-dihydro-1H-benzo[b][1,6]naphthyridin-2-yl]-acetamid (3)

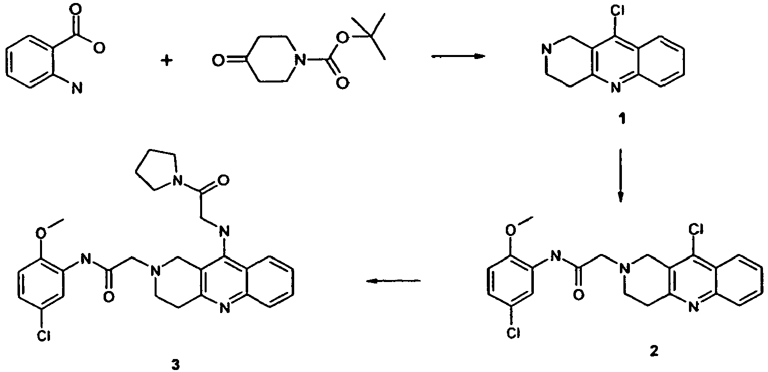

**a.** 2,00 g (14.6 mmol) Anthranilsäure und 2.90 g (14.6 mmol) 4-Oxo-piperidin-1-carbonsäure-tert-butylester werden in 50 ml Phosphorylchlorid gelöst und 2 Stunden bei Raumtemperatur gerührt. Dann wird die Lösung im Eisbad gekühlt und mit 1 N NaHCO₃-Lösung auf pH=8 eingestellt. Diese Lösung wurde dann 2x mit je 100 ml Dichlormethan extrahiert. Die organische Phase wurde über Magnesiumsulfat getrocknet, abfiltriert und i.Vak. zur Trockne eingedampft. Man erhält 2,9 g (76,4%) 1 als gelblichen kristallinen Rückstand.
**b.** 1,54 g (7,04 mmol) **1**, 2,00 g (7,2 mmol) 2-Brom-N-(5-chlor-2-methoxy-phenyl)-acetamid und 4,68 g (14.4 mmol) Cäsiumcarbonat werden in 50 ml DMF 24 Stunden bei Raumtemperatur und 2 Stunden bei 50° C gerührt. Dann gibt man 100 ml Wasser zu der Reaktionsmischung und filtriert ab. Man erhält 2,34 g (79,8%) **2** als amorphes Festprodukt.
**c.** 0.30 g (0.72 mmol) **2**, 0.18 g (1,1 mmol) 2-Amino-1pyrrolidin-1-yl-ethanone x HCl, 0,2 g (2,1 mmol) Phenol und 0,15 ml (1,1 mmol) Triethylamin werden 6 Stunden bei 120° C gerührt. Das Reaktionsgemisch wird dann in 5o ml Ethylacetat und 50 ml 1N NaOH-Lösung aufgenommen. Nach dem Ausschütteln wird die organische Phase abgetrennt und noch 2 x mit je 30 ml gesättigter NaCl-Lösung extrahiert. Dann wird sie i.Vak. zur Trockene eingedampft. Der Rückstand wird in wenig Methanol aufgenommen und mit 5 ml 6N HCl in Isopropanol versetzt. Nun wird wieder i. Vak. Zur Trockene eingedampft. Der Rückstand wird mit wenig Ethanol versetzt und der Niederschlag abfiltriert. Man erhält 98 mg (25%) 3 als amorphes Hydrochlorid.

### Beispiel 2

### Synthese von N-(5-Chlor-2-methoxy-phenyl)-2-{10-[4-(2-hydroxy-ethyl)-piperazin-1-yl]-3,4-dihydro-1H-benzo[b][1,6]naphthyridin-2-yl}-acetamid (7)

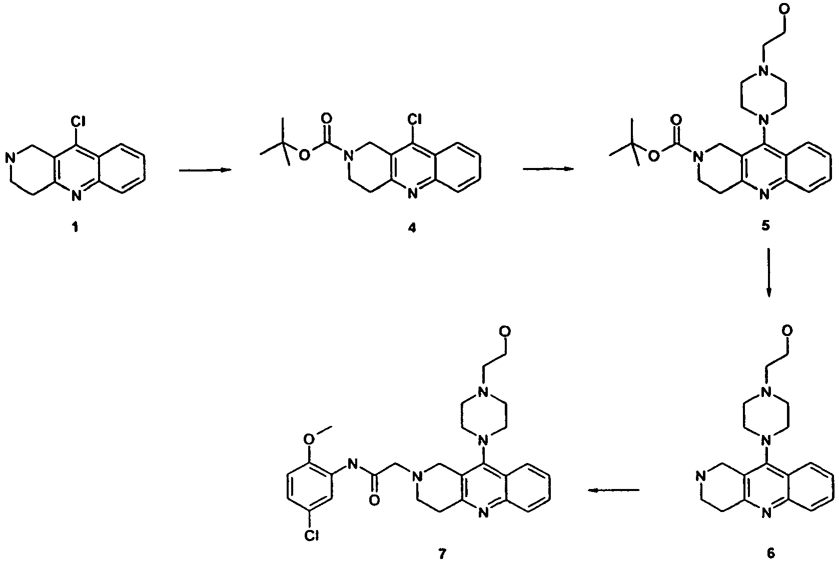

**a.** 2,90 g (13,3 mmol) **1** (Synthese wird in Beispiel 1 beschrieben) werden in 130 ml Dioxan gelöst. Man gibt 50 ml 1N Natriumhydrogencarbonat-Lösung und 3,4 ml (15,9 mmol) Di-tert-butyldicarbonat, gemischt mit 30 ml Dioxan, Die Reaktionsmischung wird mit Ethylacetat und Wasser versetzt, ausgeschüttelt und die Phasen getrennt. Die wässerige Phase wird nochmals mit Ethylacetat ausgeschüttelt, dann werden die vereinigten organischen Phasen mit gesättigter Kochsalzlösung gewaschen, über MgSO4 getrocknet, abfiltriert und i.Vak. zur Trockene eingedampft. Das Rohprodukt wird chromatographisch an Kieselgel60 mit Cyclohexan und Ethylacetat als Laufmittel gereinigt. Die entsprechenden Fraktionen werden i.Vak. zur Trockene gedampft. Man erhält 3,1 g (73,8%) **4** als gelbliche Kristalle.
**b.** 0,3 g (0,94 mmol) **4**, 0,16 ml (1,3 mmol) 2-Piperazin-1-ylethanol und 0,25 g (2,6 mmol) Phenol werden 30 min in der Mikrowelleapparatur auf 120° C erhitzt. Man versetzt das Reaktionsgemisch mit 50 ml Ethylacetat und extrahiert 2 x mit je 10 ml 1N NaOH. Dann wäscht man die organische Phase 3 x mit gesättigter Kochsalzlösung, trocknet sie über Na₂SO₄, filtriert ab und dampft sie i. Vak. Zur Trockene ein. Man erhält ein gelbes Öl, welches auskristallisiert: 0,3 g (77,4%) **5**.
**c.** 90 mg (0,22 mmol) **5** werden mit 10 ml 6N HCl in Methanol versetzt und 1 Stunde bei Raumtemperatur gerührt. Dann wird i. Vak. zur Trockene eingedampft. Man erhält 20 mg (29 %) 6 als gelbe amorphe Substanz.
**d.** 20 mg (0,064 mmol) **6**, 18 mg .(0,064 mmol) 2-Brom-N-(5-chlor-2-methoxy-phenyl)-acetamid und 62,6 mg (0,192 mmol) Cäsiumcarbonat werden in 8 ml DMF 18 Stunden gerührt. Dann wird der Ansatz mit Ethylacetat versetzt. Man wäscht einmal mit Wasser und 3 x mit gesättigter Kochsalzlösung, anschließend wird die organische Phase mit Na₂SO₄ getrocknet, abfiltriert und i. Vak. bis zur Trockene eingedampft. Der Rückstand wird mittels präparativer HPLC aufgereinigt. Nach anschließender Lyophilisation erhält man 17,7 mg (52%) 7 als braune Kristalle.

### Beispiel 3

### Synthese von 10-(2-Dimethylamino-ethylamino)-3,4-dihydro-1H-benzo[b][1,6]naphthyridine-2-carboxylic acid (5-chloro-2-methoxy-phenylcarbamoyl)-methyl ester

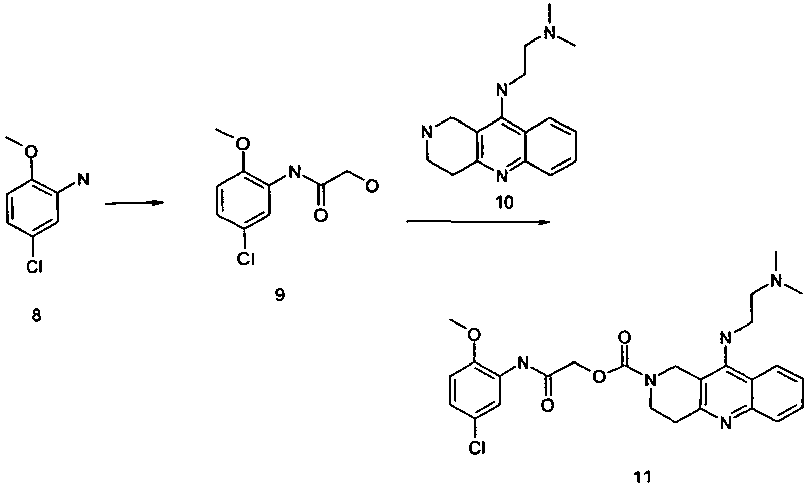

Zunächst bildet man aus Glycolsäure und 5-Chlor-2-methoxy-phenylamin **8** mit einem geeigneten Amidkupplungsverfahren, z.B. TBTU, HOBt (1-Hydroxybenzotriazolhydrat), NMM (4-Methylmorpholin) in DMF das Amid **9**. Dieses wird in einem geeigneten Lösungsmittel (z.B. DMF) mit 1,1'-Carbonyldiimidazol mehrere Stunden bei RT gerührt. Nach der anschliessenden Zugabe von **10** zum Reaktionsgemisch und erneutem Rühren für mehrere Stunden bei RT erhält man **11**.

Eine Übersicht weiterer analog synthetisierter Verbindungen der Erfindung einschließlich der physikalisch-chemischen Parameter aller Verbindungen der Erfindung wird in Tabelle 2 gegeben.

**Tabelle 2**

| **Compound No.** | **Chemical Name** | **Rt [min] (HPLC method)*** | **ESI [M+1]⁺** |
|---|---|---|---|
| **1** | N-(5-Chloro-2-methoxy-phenyl)-2-[10-(1-methyl-piperidin-4-ylamino)-3,4-dihydro-1H-benzo[b][1,6]naphthyridi n-2-yl]-acetamide | 2,16 (C) | 495 |
| **2** | 2-(10-Amino-3,4-dihydro-1H-benzo[b][1,6]naphthyridi n-2-yl)-N-(5-chloro-2-methoxy-phenyl)-acetamide | 2,43 (A) | 398 |
| **3** | 2-(10-Amino-3,4-dihydro-1H-benzo[b][1,6]naphthyridi n-2-yl)-N-(2,5-dichloro-phenyl)-acetamide | 2,46 (A) | 402 |
| **4** | N-(5-Chloro-2-methoxy-phenyl)-2-[10-(2-hydroxy-ethylamino)-3,4-dihydro-1H-benzo[b][1,6]naphthyridi n-2-yl]-acetamide | 2,43 (A) | 442 |
| **5** | N-(5-Chloro-2-methoxyphenyl)-2-[10-(2-dimethylamino-ethylamino)-3,4-dihydro-1H-benzo[b][1,6]naphthyridi n-2-yl]-acetamide | 2,15 (B) | 469 |
| **6** | 10-(2-Dimethylamino-ethylamino)-3,4-dihydro-1H-benzo[b][1,6]naphthyridi ne-2-carboxylic acid (5-chloro-2-methoxy-phenylcarbamoyl)-methyl ester | 2,11 (B) | 513 |
| **7** | 10-(2-Hydroxy-ethylamino)-3,4-dihydro-1H-benzo[b][1,6]naphthyridi ne-2-carboxylic acid (5-chloro-2-methoxy-phenylcarbamoyl)-methyl ester | 2,43 (A) | 486 |
| **8** | N-(5-Chloro-2-methoxyphenyl)-2-[10-(methylcarbamoylmethyl-amino)-3,4-dihydro-1H-benzo[b][1,6]naphthyridi n-2-yl]-acetamide | 2,67 (C) | 469 |
| **9** | N-(5-Chloro-2-methoxyphenyl)-2-[10-(dimethylcarbamoylmeth yl-amino)-3,4-dihydro-1H-benzo[b][1,6]naphthyridi n-2-yl]-acetamide | 2,83 (C) | 483 |
| **10** | N-(5-Chloro-2-methoxy-phenyl)-2-{10-[4-(2-hydroxy-ethyl)-piperazin-1-yl]-3,4-dihydro-1H-benzo[b][1,6]naphthyridi n-2-yl}-acetamide | 2,27 (A) | 511 |
| **11** | 2-{10-[(1H-Benzoimidazol-2-ylmethyl)-amino]-3,4-dihydro-1H-benzo[b][1,6]naphthyridi n-2-yl}-N-(5-chloro-2-methoxy-phenyl)-acetamide | 2,80 (C) | 527 |
| **12** | N-(5-Chloro-2-methoxy-phenyl)-2-[10-(2-oxo-2-pyrrolidin-1-yl-ethylamino)-3,4-dihydro-1H-benzo[b][1,6]naphthyridi n-2-yl]-acetamide | 2,91 (C) | 509 |
| **13** | N-(5-Chloro-2-methoxy-phenyl)-2-[10-(2-oxo-2-piperidin-1-yl-ethylamino)-3,4-dihydro-1H-benzo[b][1,6]naphthyridi n-2-yl]-acetamide | 3,15 (C) | 523 |
| **14** | N-(5-Chloro-2-methoxy-phenyl)-2-[10-(2-morpholin-4-yl-2-oxo-ethylamino)-3,4-dihydro-1H-benzo[b][1,6]naphthyridi n-2-yl]-acetamide | 2,80 (C) | 525 |
| **15** | N-(5-Chloro-2-methoxyphenyl)-2-[10-(cyclohexylcarbamoylmet hyl-amino)-3,4-dihydro-1H-benzo[b][1,6]naphthyridi n-2-yl]-acetamide | 3,20 (C) | 537 |
| **16** | N-(5-Chloro-2-methoxy-phenyl)-2-{10-[2-(4-methyl-piperazin-1-yl)-2-oxo-ethylamino]-3,4-dihydro-1H-benzo[b][1,6]naphthyridi n-2-yl}-acetamide | 2,29 (C) | 538 |
| **17** | N-(5-Chloro-2-methoxy-phenyl)-2-{10-[2-(4-isopropyl-piperazin-1-yl)-2-oxo-ethylamino]-3,4-dihydro-1H-benzo[b][1,6]naphthyridi n-2-yl}-acetamide | 2,32 (C) | 566 |
| **18** | N-(5-Chloro-2-methoxyphenyl)-2-[10-(diethylcarbamoylmethyl-amino)-3,4-dihydro-1H-benzo[b][1,6]naphthyridi n-2-yl]-acetamide | 3,15 (C) | 511 |
| **19** | 2-{10-[2-(4-Benzyl-piperazin-1-yl)-2-oxo-ethylamino]-3,4-dihydro-1H-benzo[b][1,6]naphthyridi n-2-yl}-N-(5-chloro-2-methoxy-phenyl)-acetamide | 2,59 (C) | 614 |
| **20** | N-(5-Chloro-2-methoxy-phenyl)-2-[10-(pyridin-4-ylamino)-3,4-dihydro-1H-benzo[b][1,6]naphthyridi n-2-yl]-acetamide | 2,53 (C) | 475 |
| **21** | N-(5-Chloro-2-methoxy-phenyl)-2-[10-(pyridin-3-ylamino)-3,4-dihydro-1H-benzo[b][1,6]naphthyridi n-2-yl]-acetamide | 2,72 (C) | 475 |
| **22** | N-(5-Chloro-2-methoxyphenyl)-2-(10-phenylamino-3,4-dihydro-1H-benzo[b][1,6]naphthyridi n-2-yl)-acetamide | 3,25 (C) | 474 |
| **23** | N-(5-Chloro-2-methoxyphenyl)-2-[10-(pyridin-2-ylamino)-3,4-dihydro-1H-benzo[b][1,6]naphthyridi n-2-yl]-acetamide | 2,96 (C) | 475 |
| **24** | N-(5-Chloro-2-methoxyphenyl)-2-[10-(4-methoxy-phenylamino)-3,4-dihydro-1H-benzo[b][1,6]naphthyridi n-2-yl]-acetamide | 3,25 (C) | 504 |

Nachstehend sind die ¹H-NMR-Daten für ausgewählte Verbindungen der Erfindung dargestellt:

### Compound 2

¹H NMR (500 MHz, DMSO) δ = 8.56 (d, *J*=8.5, 1 H), 8.20 (t, *J*=3.8, 1 H), 7.99 (t, *J*=7.7, 1H), 7.92 (d, *J*=8.4, 1 H), 7.72 (t, *J*=7.8, 1H), 7.22 (dd, *J*=8.8, 2.5, 1H), 7.14 (d, *J*=8.9, 1H), 4.54 (m, 4H), 3.90 (s, 3H), 3.84 (t, *J*=5.9, 2H), 3.46 (t, *J*=6.0, 2H)

### Compound 4

¹H NMR (400 MHz, DMSO) δ = 8.50 (t, *J*=13.4, 1H), 8.10 (d, *J*=2.2, 1H), 7.94 - 7.77 (m, 2H), 7.71 - 7.52 (m, 1 H), 7.20 - 6.93 (m, 2H), 4.91 - 4.49 (m, 4H), 4.04 - 3.62 (m, 9H), 3.17 (m, 2H).

### Compound 7

¹H NMR (400 MHz, DMSO) δ = 8.50 (t, *J*=13.4, 1H), 8.10 (d, *J*=2.2, 1 H), 7.94 - 7.77 (m, 2H), 7.71 - 7.52 (m, 1 H), 7.20 - 6.93 (m, 2H), 4.91 - 4.49 (m, 4H), 4.04 - 3.62 (m, 9H), 3.17 (m, 2H).

### Compound 8

¹H NMR (500 MHz, DMSO) ö = 8.40 (d, *J*=8.6, 1H), 8.20 (d, *J*=2.5, 1 H), 7.96 (m, 2H), 7.75 - 7.66 (m, 1H), 7.21 (dd, *J*=8.8, 2.6, 1 H), 7.17 - 7.10 (m, 1 H), 4.72 (s, 2H), 4.52 (t, *J*=16.1, 4H), 3.97 - 3.85 (2, 3H), 3.81 (t, *J*=6.2, 2H), 3.47 (m, 2H), 2.71 (s, 3H).

### Compound 9

¹H NMR (500 MHz, DMSO) δ = 8.45 (d, *J*=8.6, 1H), 8.18 (d, *J*=2.5, 1H), 8.01 (t, *J*=7.4, 1H), 7.94 (d, *J*=7.8, 1 H), 7.73 (t, *J*=7.8, 1 H), 7.22 (dd, *J*=8.8, 2.5, 1 H), 7.14 (d, *J*=8.9, 1H), 4.82 (s, 2H), 4.73 (s, 2H), 4.55 (s, 2H), 3.95 - 3.86 (m, 3H), 3.86 (s, 2H), 3.47 (dd, *J*=14.0, 7.6, 2H), 3.08 (d, *J*=11.5, 3H), 2.95 (d, *J*=11.5, 3H).

### Compound 11

¹H NMR (500 MHz, DMSO) δ = 8.44 (d, *J*=8.7, 1H), 8.10 - 7.93 (m, 3H), 7.76 (m, 2H), 7.69 (dd, *J*=11.0, 4.2, 1 H), 7.54 - 7.48 (m, 2H), 7.14 (dd, *J*=8.8, 2.6, 1H), 7.05 (d, *J*=8.9, 1H), 5.63 (s, 2H), 4.76 (s, 2H), 4.51 (d, *J*=19.6, 2H), 3.88 - 3.75 (m, 5H), 3.67 - 3.45 (m, 2H).

### Compound 12

¹H NMR (400 MHz, DMSO) δ = 8.47 (d, *J*=8.6, 1 H), 8.21 (d, *J*=2.6, 1H), 8.03 - 7.93 (m, 2H), 7.73 (ddd, *J*=8.4, 6.4, 1.8, 1H), 7.21 (dd, *J*=8.8, 2.5, 1H), 7.12 (d, *J*=8.9, 1 H), 4.80 (s, 2H), 4.75 (s, 2H), 4.59 (s, 2H), 3.90 (s, 3H), 3.86 (m, 2H), 3.52 (m, 4H), 3.41 (t, *J*=6.9, 2H), 2.01 - 1.89 (m, 2H), 1.89 - 1.76 (m, 2H).

### Compound 13

¹H NMR (400 MHz, DMSO) δ = 8.48 (d, *J*=8.7, 1H), 8.22 (m, 1H), 8.04 - 7.92 (m, 2H), 7.72 (ddd, *J*=8.4, 6.3, 1.9, 1 H), 7.21 (dd, *J*=8.8, 2.5, 1 H), 7.12 (d, *J*=8.9, 1H), 4.83 (2 s, 4H), 4.61 (s, 2H), 3.93 (s, 3H), 3.85 (m, 2H), 3.64 - 3.36 (m, 6H), 1.57 (m, 6H).

### Compound 14

¹H NMR (400 MHz, DMSO) δ = 8.41 (d, *J*=8.7, 1H), 8.12 (dd, *J*=11.0, 7.0, 1H), 7.97 - 7.86 (m, 2H), 7.71 - 7.61 (m, 1H), 7.13 (m, 1H), 7.04 (d, *J*=8.9, 1H), 4.82 (s, 2H), 4.72 (s, 2H), 4.53 (s, 2H), 3.83 (s, 3H), 3.81 (s, 2H), 3.67 - 3.40 (m, 10H).

### Compound 16

¹H NMR (500 MHz, DMSO) δ = 8.51 (d, *J*=8.7, 1H), 8.19 (d, *J*=3.8, 1H), 8.06 - 7.94 (m, 2H), 7.75 (ddd, *J*=8.4, 5.8, 2.4, 1 H), 7.21 (dd, *J*=8.8, 2.5, 1H), 7.12 (d, *J*=8.9, 1 H), 5.17 - 5.04 (m, 1 H), 4.97 - 4.81 (m, 3H), 4.65 (s, 2H), 4.49 (d, *J*=14.1, 1 H), 4.27 (d, *J*=13.4, 1H), 3.97 - 3.78 (m, 5H), 3.89 - 3.48 (m, 5H), 3.31 - 3.00 (m, 3H), 2.92 (d, *J*=5.1, 3H).

### Compound 17

¹H NMR (500 MHz, DMSO) δ = 8.49 (d, *J*=8.7, 1H), 8.20 (m, 1H), 7.99 (m, 2H), 7.73 (t, *J*=7.1, 1H), 7.21 (dd, *J*=8.8, 2.5, 1H), 7.11 (d, *J*=8.9, 1H), 5.04 (d, *J*=17.8, 1H), 4.90 (d, *J*=16.6, 1H), 4.77 (s, 2H), 4.59 (s, 3H), 4.25 (d, *J*=13.0, 1H), 3.90 (s, 3H), 3.87 (m, 2H), 3.67 - 3.39 (m, 6H), 3.18 (d, *J*=17.1, 2H), 3.00 (s, 1H), 1.42 - 1.22 (m, 6H).

### Compound 18

¹H NMR (400 MHz, DMSO) δ = 8.44 - 8.33 (m, 1H), 8.16 (d, *J*=2.5, 1H), 7.89 (m, 2H), 7.63 (m, 1H), 7.13 (dd, *J*=8.7, 2.3, 1H), 7.09 - 7.00 (m, 1 H), 4.86 - 4.66 (m, 4H), 4.61 - 4.47 (m, 2H), 3.91 - 3.71 (m, 5H), 3.37 (m, 6H), 1.24 - 1.10 (m, 3H), 1.02 (t, *J*=7.1, 3H).

### Compound 22

¹H NMR (500 MHz, DMSO) δ = 8.28 (d, *J*=8.6, 1H), 8.05 (d, *J*=3.8, 1H), 8.02 - 7.93 (m, 2H), 7.63 (ddd, *J*=8.4, 6.3, 1.8, 1 H), 7.46 - 7.38 (m, 2H), 7.28 (dd, *J*=13.9, 6.5, 1H), 7.21 (dd, *J*=13.3, 6.6, 2H), 7.18 - 7.11 (m, 1H), 7.04 (d, *J*=8.9, 1 H), 4.31 (s, 2H), 4.11 (s, 2H), 3.88 - 3.72 (m, 4H), 3.58 - 3.46 (m, 3H).

### Compound 23

¹H NMR (500 MHz, DMSO) δ = 8.24 (d, *J*=8.5, 1 H), 8.16 (d, *J*=4.2, 1 H), 8.08 (d, *J*=8.4, 1H), 8.05 - 7.98 (m, 2H), 7.91 - 7.82 (m, 1 H), 7.71 (t, *J*=7.6, 1H), 7.20 - 7.11 (m, 2H), 7.06 (m, 2H), 4.39 (d, *J*=13.5, 4H), 3.85 (t, *J*=8.3, 2H), 3.81 (s, 3H), 3.67 - 3.55 (m, 2H).

### Compound 24

¹H NMR (500 MHz, DMSO) δ = 8.26 (d, *J*=8.7, 1 H), 8.08 (m, 1 H), 7.95 - 7.84 (m, 2H), 7.55 (t, *J*=7.4, 1H), 7.17 (d, *J*=8.7, 2H), 7.12 - 7.06 (m, 1 H), 6.99 (d, *J*=8.9, 1 H), 6.94 (d, *J*=8.7, 2H), 4.31 (s, 2H), 4.06 (s, 2H), 3.79 (s, 3H), 3.75 (t, *J*=6.3, 2H), 3.70 (s, 3H), 3.47 (t, *J*=6.5, 2H).

Für die Bestimmung der oben dargestellten physikalisch-chemischen Parameter wurden die folgenden Analysenmethoden verwendet:
**ESI:** Elektrospray-Ionisation Massenspektrometrie (M+H)⁺
   ***(A): HPLC-Methode:** 1_100_2 Speed (Gerät: LaChrom)
      Säule: Chromolith Performance RP18e 100-3mm
      Flussrate: 2ml/min (Pumpe: L-7100)
      Solvent A: Wasser + 0.01% TFA
      Solvent B: Acetonitril + 0.01% TFA
      Wellenlänge: 220 nm (Detektor: L-7455)
      0-0,2 100% A, 0,2 - 3,7 auf 100% B, 3,7 - 4,4 100% B, 4,5 - 5,0 100% A
   **(B): HPLC-Methode:** polar.M (Gerät: Agilent 1100 Series)
      Säule: Chromolith Speed Rod RP18e-50-4.6
      Flussrate: 2.4ml/min
      Solvent A: Wasser + 0,05% HCOOH
      Solvent B: Acetonitril + 0,04% HCOOH
      WL: 220 nm
      Gradient: 0-2.8min: 4%B auf 100%B, 2.6-3.3min:100%B
   **(C): HPLC-Methode:** Säule: Chromolith SpeedROD, 50 x 4.6 mm2 (Best.Nr. ^1.51450.0001) von Merck
      Gradient: 5.0 min, t = 0 min, A:B = 95:5, t = 4.4 min: A:B = 25:75,
      t = 4.5 min bis t = 5.0 min: A:B = 0:100
      Fluß:3.00 ml/min, Laufmittel A: Wasser + 0,01% HCOOH (Ameisensäure)
      Laufmittel B: Acetonitril + 0,01% HCOOH, Wellenlänge: 220 nm

### II. Autotaxin Test (Enzym Test)

### Testbeschreibung

Die Autotaxin Aktivität wird indirekt mit dem Amplex Red Reagenz gemessen. Hierbei wird Amplex Red als fluorgenischem Indikatior für das entstandene H₂O₂ gemessen. Im Detail setzt Autotaxin das Substrat Lysophosphatidylcholin (LPC) zu Phosphocholin und Lysophosphatidylsäure (LPS) um. Nach dieser Umsetzung wird das Phosphocholin mit alkalischer Phosphatase zu inorganischem Phosphat und Cholin ungesetzt. Im nächsten Schritt wird Cholin durch Choline-Oxidase zu Betain oxidiert, wobei H₂O₂ entsteht. H₂O₂ reagiert in Gegenwart von Peroxidase (Horseradish peroxidase) mit dem Amplex Red Reagenz in eine 1:1 Stöchiometrie und bildet das hochfluoreszente Resorufin. Die Fluoreszenz wird in einem reaktionsabhängigen kinetischen Modus gemessen, damit dass fluoreszente Signale möglicher anderer fluoreszenter Stoffe, die nicht an der Reaktion beteiligt sind, herauskorrigiert werden kann.

### Testausführung

1,5 µl einer Standardlösung oder der Testsubstanzen (Verbindungen der Erfindung) in individuellen Konzentrationen gelöst in 20mM Hepes pH 7.2 mit maximal 7.7% DMSO werden zusammen mit 10 µl (16 ng) hochgereinigten rekombinanten Autotaxin in einer schwarzen mit 384 Vertiefungen versehenen Mikrotiterplatte für 30 min bei 22°C vorinkubiert. Danach wird die Reaktion durch Zugabe von 5µl L-α-Lysophosphatidylcholin (LPC) gestartet, wobei die Endkonzentration von LPC 75 µM beträgt. Die Mischung wird 90 min bei 37°C inkubiert. Nach der Inkubation wird Amplex Red Reagenz, Peroxidase (Horseradish peroxidase) und Cholin-Oxidase hinzugefügt und sofort die Fluoreszenz bei 612 nm bei einer Anregung von 485 nm in einem "Tecan Ultra multimode" Lesegerät gemessen. Die Aktivität von Autotaxin wird indirekt über den Nachweis des anfallenden H₂O₂ errechnet.

### Material:

| | |
|---|---|
| Microtiterplatte: | PS-Microplate, 384 Vertiefungen, kleines Volumen, schwarz Corning, Cat#3677 |
| Protein: | Rekombinantes Autotaxin (Baculovirale Hi5 Expression) |
| Substrat: | L-α-Lysophosphatidylcholin (Hühnerei)); Avanti Polar Lipids # 830071 P |
| Standard: | C14 LPA, Avanti Polar Lipids, Cat# 857120P |
| Nachweis Reagenz: | Amplex Red Reagenz ; Invitrogen # A12222; gelöst in 1.923 ml of DMSO Peroxidase Type VI-A (horseradish) von Sigma # P6782; gelöst in 7,45 ml Test Puffer, Choline-Oxidase ; Sigma # C5896; gelöst in 2,47 ml Test Puffer |
| Nachweis Reagenz Mix: | 1:100 Verdünnung von Amplex Red Regenzt in Test Puffer |
| Test Puffer: | 200 mM Tris-HCl, Merck, Cat # 1.08219, pH 7.9, 0.1 % BSA, lipidfrei, Roche Cat#775835 |

### Pharmakologische Daten

Autotaxin-Hemmung
(IC₅₀-Bereiche: A: < 100 nM, B: 100 nM - 1000 nM, C: > 1000 nM)

**Tabelle 3**

| **Erfindungsgemäße Verbindung** | **IC₅₀; % (ctrl 3E-5 M)** |
|---|---|
| N-(5-Chloro-2-methoxy-phenyl)-2-[10-(1-methyl-piperidin-4-ylamino)-3,4-dihydro-1H-benzo[b][1,6]naphthyridin-2-yl]-acetamide | B |
| 2-(10-Amino-3,4-dihydro-1H-benzo[b][1,6]naphthyridin-2-yl)-N-(5-chloro-2-methoxy-phenyl)-acetamide | C |
| 2-(10-Amino-3,4-dihydro-1H-benzo[b][1,6]naphthyridin-2-yl)-N-(2,5-dichloro-phenyl)-acetamide | 79% |
| N-(5-Chloro-2-methoxy-phenyl)-2-[10-(2-hydroxy-ethylamino)-3,4-dihydro-1H-benzo[b][1,6]naphthyridin-2-yl]-acetamide | B |
| N-(5-Chloro-2-methoxy-phenyl)-2-[10-(2-dimethylamino-ethylamino)-3,4-dihydro-1H-benzo[b][1,6]naphthyridin-2-yl]-acetamide | B |
| 10-(2-Dimethylamino-ethylamino)-3,4-dihydro-1H-benzo[b][1,6]naphthyridine-2-carboxylic acid (5-chloro-2-methoxy-phenylcarbamoyl)-methyl ester | -9% |
| 10-(2-Hydroxy-ethylamino)-3,4-dihydro-1H-benzo[b][1,6]naphthyridine-2-carboxylic acid (5-chloro-2-methoxy-phenylcarbamoyl)-methyl ester | C |
| N-(5-Chloro-2-methoxy-phenyl)-2-[10-(methylcarbamoylmethyl-amino)-3,4-dihydro-1H-benzo[b][1,6]naphthyridin-2-yl]-acetamide | B |
| N-(5-Chloro-2-methoxy-phenyl)-2-[10-(dimethylcarbamoylmethyl-amino)-3,4-dihydro-1H-benzo[b][1,6]naphthyridin-2-yl]-acetamide | B |
| N-(5-Chloro-2-methoxy-phenyl)-2-{10-[4-(2-hydroxy-ethyl)-piperazin-1-yl]-3,4-dihydro-1H-benzo[b][1,6]naphthyridin-2-yl}-acetamide | B |
| 2-{10-[(1H-Benzoimidazol-2-ylmethyl)-amino]-3,4-dihydro-1H-benzo[b][1,6]naphthyridin-2-yl}-N-(5-chloro-2-methoxy-phenyl)-acetamide | C |
| N-(5-Chloro-2-methoxy-phenyl)-2-[10-(2-oxo-2-pyrrolidin-1-yl-ethylamino)-3,4-dihydro-1H-benzo[b][1,6]naphthyridin-2-yl]-acetamide | B |
| N-(5-Chloro-2-methoxy-phenyl)-2-[10-(2-oxo-2-piperidin-1-yl-ethylamino)-3,4-dihydro-1H-benzo[b][1,6]naphthyridin-2-yl]-acetamide | B |
| N-(5-Chloro-2-methoxy-phenyl)-2-[10-(2-morpholin-4-yl-2-oxo-ethylamino)-3,4-dihydro-1H-benzo[b][1,6]naphthyridin-2-yl]-acetamide | B |
| N-(5-Chloro-2-methoxy-phenyl)-2-[10-(cyclohexylcarbamoylmethyl-amino)-3,4-dihydro-1H-benzo[b][1,6]naphthyridin-2-yl]-acetamide | C |
| N-(5-Chloro-2-methoxy-phenyl)-2-{10-[2-(4-methyl-piperazin-1-yl)-2-oxo-ethylamino]-3,4-dihydro-1H-benzo[b][1,6]naphthyridin-2-yl}-acetamide | B |
| N-(5-Chloro-2-methoxy-phenyl)-2-{10-[2-(4-isopropyl-piperazin-1-yl)-2-oxo-ethylamino]-3,4-dihydro-1H-benzo[b][1,6]naphthyridin-2-yl}-acetamide | B |
| N-(5-Chloro-2-methoxy-phenyl)-2-[10-(diethylcarbamoylmethyl-amino)-3,4-dihydro-1H-benzo[b][1,6]naphthyridin-2-yl]-acetamide | B |
| 2-{10-[2-(4-Benzyl-piperazin-1-yl)-2-oxo-ethylamino]-3,4-dihydro-1H-benzo[b][1,6]naphthyridin-2-yl}-N-(5-chloro-2-methoxy-phenyl)-acetamide | B |
| N-(5-Chloro-2-methoxy-phenyl)-2-[10-(pyridin-4-ylamino)-3,4-dihydro-1H-benzo[b][1,6]naphthyridin-2-yl]-acetamide | C |
| N-(5-Chloro-2-methoxy-phenyl)-2-[10-(pyridin-3-ylamino)-3,4-dihydro-1H-benzo[b][1,6]naphthyridin-2-yl]-acetamide | C |
| N-(5-Chloro-2-methoxy-phenyl)-2-(10-phenylamino-3,4-dihydro-1H-benzo[b][1,6]naphthyridin-2-yl)-acetamide | C |
| N-(5-Chloro-2-methoxy-phenyl)-2-[10-(pyridin-2-ylamino)-3,4-dihydro-1H-benzo[b][1,6]naphthyridin-2-yl]-acetamide | C |
| N-(5-Chloro-2-methoxy-phenyl)-2-[10-(4-methoxy-phenylamino)-3,4-dihydro-1H-benzo[b][1,6]naphthyridin-2-yl]-acetamide | C |

## Patentansprüche

1. Verbindungen der Formel (I) 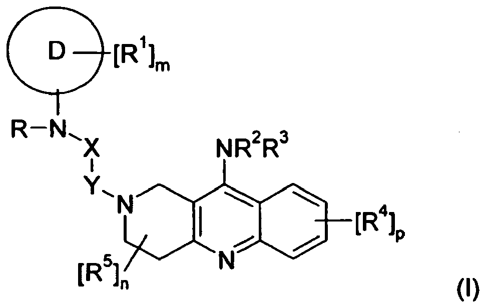 worin:
D Ar oder Het bedeutet,
Ar unsubstituiertes oder einfach oder mehrfach substituiertes Phenyl, Indanyl, Naphthyl oder Biphenyl bedeutet,
Het einen ein- oder zweikernigen gesättigten, partiell ungesättigten oder aromatischen Heterocyclus mit 1, 2, 3 oder 4 N-, O- und/oder S- Atomen, der unsubstituiert oder einfach oder mehrfach substituiert sein kann, bedeutet,
X, Y jeweils unabhängig voneinander fehlt, -CH₂-, -(CH₂)₂-, -C(O)-, -CHOH- oder -CH₂OC(O)- bedeuten, wobei nur einer der Reste X oder Y fehlen darf,
R jeweils unabhängig voneinander H, A, Cyc, (CH₂)_{q}Ar oder (CH₂)_{q}Het bedeuten und einfach oder mehrfach mit R⁶ substituiert sein können, wobei bei A und Cyc die C-Kette bzw. der C-Ring auch durch O unterbrochen sein kann,
R¹ jeweils unabhängig voneinander R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phenyl bedeuten und einfach oder mehrfach mit R⁶ substituiert sein können,
R², R³ jeweils unabhängig voneinander R bedeuten, wobei R² und R³ alternativ zusammen auch Cyc oder Het bilden können, welche wiederum einfach oder mehrfach mit R⁶ substituiert sein können,
R⁴ jeweils unabhängig voneinander R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phenyl bedeuten und einfach oder mehrfach mit R⁶ substituiert sein können,
R⁵ jeweils unabhängig voneinander R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phenyl bedeuten und einfach oder mehrfach mit R⁶ substituiert sein können,
R⁶ jeweils unabhängig voneinander R, F, Cl, Br, I, OH, =O, CN, NO₂, NRR, NHC(O)R, NHSO₂R,-OR, C(O)R, C(O)NRR, CF₃, OCF₃, SCF₃, SO₂CH₃, SO₃R, SO₂R, SO₂NR, SR, OA, A, phenyl bedeuten und, sofern eine Substitution chemisch möglich ist, einfach oder mehrfach mit R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phenyl substituiert sein können,
A linear oder verzweigtes Alkyl mit 1, 2, 3, 4, 5, 6, 7 oder 8 C-Atomen bedeutet, worin 1, 2, 3, 4, 5, 6 oder 7 H-Atome durch OR, CN, NRR, F und/oder CI ersetzt sein können und/oder worin eine oder zwei nicht-benachbarte CH₂-Gruppen durch O, NH, S, SO, SO₂ und/oder durch CH=CH-Gruppen ersetzt sein können,
Cyc cyclisches Alkyl mit 3, 4, 5, 6, 7, 8, 9, 10, 11, 12, 13 oder 14 C-Atomen bedeutet,
m 0, 1, 2, 3, 4, oder 5 bedeutet,
n 0, 1, 2, oder 3 bedeutet,
p 0, 1, 2, 3, oder 4 bedeutet,
q 0, 1, oder 2 bedeutet,
sowie ihre pharmazeutisch verwendbaren Salze, Solvate und Stereoisomere, einschließlich deren Mischungen in allen Verhältnissen.

2. Verbindungen gemäß Anspruch 1, worin
D Ar bedeutet,
Ar unsubstituiertes oder einfach oder mehrfach substituiertes Phenyl bedeutet, bevorzugt aber einfach oder mehrfach substituiertes Phenyl bedeutet,
X, Y jeweils unabhängig voneinander -CH₂-, -C(O)- oder - CH₂OC(O)- bedeuten, bevorzugt ist X = -C(O)- und Y = -CH₂-oder -CH₂OC(O)-,
R¹ jeweils unabhängig voneinander F, Cl, OA oder OCH₃ bedeuten,
R², R³ jeweils unabhängig voneinander H, Ar, Ar einfach substituiert mit OA, Het, Het einfach substituiert mit A, CH₂-Het, A, A einfach substituiert mit OH oder mit NRR oder mit CO-NRR oder mit Het oder mit CO-R bedeuten oder jeweils unabhängig voneinander 1-methyl-piperidin-4-yl, 2-hydroxy-ethyl, 2-dimethylamino-ethyl, methylcarbamoylmethyl, dimethylcarbamoylmethyl, 1H-Benzoimidazol-2-ylmethyl, 2-oxo-2-pyrrolidin-1-yl-ethyl, 2-oxo-2-piperidin-1-yl-ethyl, 2-morpholin-4-yl-2-oxo-ethyl, cyclohexylcarbamoylmethyl, 2-(4-methyl-piperazin-1-yl)-2-oxo-ethyl, 2-(4-isopropyl-piperazin-1-yl)-2-oxo-ethyl, diethylcarbamoylmethyl, 2-(4-Benzyl-piperazin-1-yl)-2-oxo-ethyl, 3-oxo-3-piperidin-1-yl-propyl, pyridin-4-yl, pyridin-3-yl, phenyl, pyridin-2-yl oder 4-methoxy-phenyl bedeuten, wobei R² und R³ alternativ zusammen auch 4-(2-hydroxy-ethyl)-piperazin-1-yl bilden können,
m 2 bedeutet,
n 0 bedeutet,
p 0 bedeutet,
q 0 oder 1 bedeutet,
Het, R, R⁶, A und Cyc die in Anspruch 1 angegebenen Bedeutungen haben,
sowie ihre pharmazeutisch verwendbaren Salze, Solvate und Stereoisomere, einschließlich deren Mischungen in allen Verhältnissen.

3. Verbindungen nach einem oder mehreren der Ansprüche 1 bis 2, ausgewählt aus der Gruppe bestehend aus:
| **Compound No.** | **Chemical Structure** | **Chemical Name** |
|---|---|---|
| **1** | 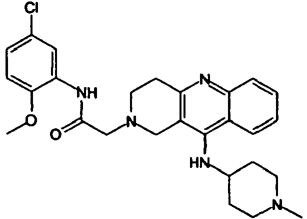 | N-(5-Chloro-2-methoxy-phenyl)-2-[10-(1-methyl-piperidin-4-ylamino)-3,4-dihydro-1H-benzo[b][1,6]naphthyri din-2-yl]-acetamide |
| **2** | 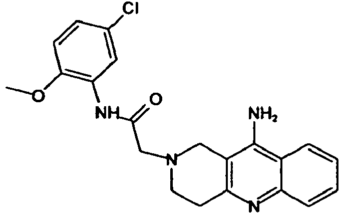 | 2-(10-Amino-3,4-dihydro-1H-benzo[b][1,6]naphthyri din-2-yl)-N-(5-chloro-2-methoxy-phenyl)-acetamide |
| **3** | 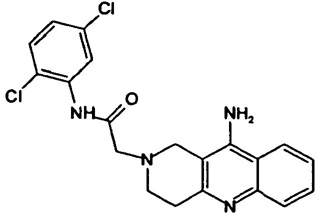 | 2-(10-Amino-3,4-dihydro-1H-benzo[b][1,6]naphthyri din-2-yl)-N-(2,5-dichloro-phenyl)-acetamide |
| **4** | 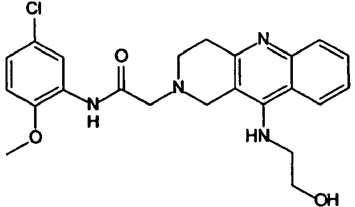 | N-(5-Chloro-2-methoxy-phenyl)-2-[10-(2-hydroxy-ethylamino)-3,4-dihydro-1H-benzo[b][1,6]naphthyri din-2-yl]-acetamide |
| **5** | 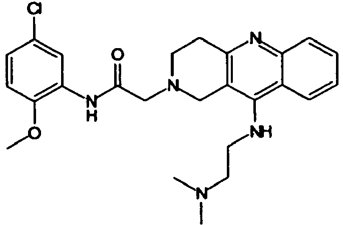 | N-(5-Chloro-2-methoxy-phenyl)-2-[10-(2-dimethylamino-ethylamino)-3,4-dihydro-1H-benzo[b][1,6]naphthyri din-2-yl]-acetamide |
| **6** | 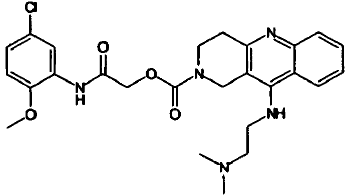 | 10-(2-Dimethylamino-ethylamino)-3,4-dihydro-1H-benzo[b][1,6]naphthyri dine-2-carboxylic acid (5-chloro-2-methoxy-phenylcarbamoyl)-methyl ester |
| **7** | 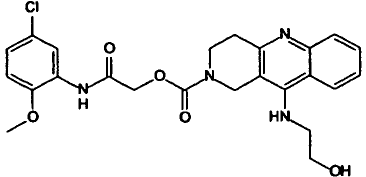 | 10-(2-Hydroxy-ethylamino)-3,4-dihydro-1H-benzo[b][1,6]naphthyri dine-2-carboxylic acid (5-chloro-2-methoxyphenylcarbamoyl)-methyl ester |
| **8** | 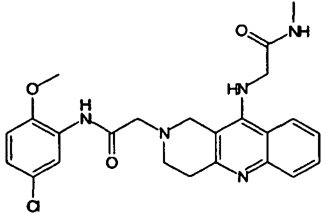 | N-(5-Chloro-2-methoxy-phenyl)-2-[10-(methylcarbamoylmet hyl-amino)-3,4-dihydro-1H-benzo[b][1,6]naphthyri din-2-yl]-acetamide |
| **9** | 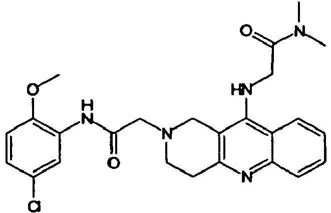 | N-(5-Chloro-2-methoxy-phenyl)-2-[10-(dimethylcarbamoylm ethyl-amino)-3,4-dihydro-1H-benzo[b][1,6]naphthyri din-2-yl]-acetamide |
| **10** | 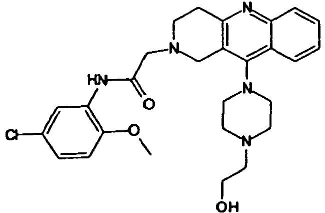 | N-(5-Chloro-2-methoxy-phenyl)-2-{10-[4-(2-hydroxy-ethyl)-piperazin-1-yl]-3,4-dihydro-1H-benzo[b][1,6]naphthyri din-2-yl}-acetamide |
| **11** | 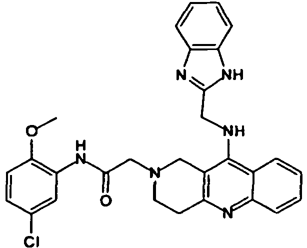 | 2-{10-[(1H-Benzoimidazol-2-ylmethyl)-amino]-3,4-dihydro-1H-benzo[b][1,6]naphthyri din-2-yl}-N-(5-chloro-2-methoxy-phenyl)-acetamide |
| **12** | 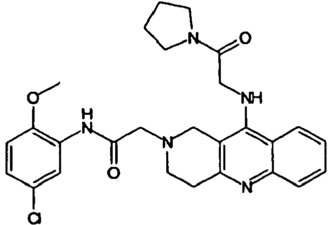 | N-(5-Chloro-2-methoxy-phenyl)-2-[10-(2-oxo-2-pyrrolidin-1-yl-ethylamino)-3,4-dihydro-1H-benzo[b][1,6]naphthyri din-2-yl]-acetamide |
| **13** | 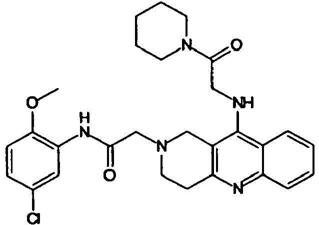 | N-(5-Chloro-2-methoxy-phenyl)-2-[10-(2-oxo-2-piperidin-1-yl-ethylamino)-3,4-dihydro-1H-benzo[b][1,6]naphthyri din-2-yl]-acetamide |
| **14** | 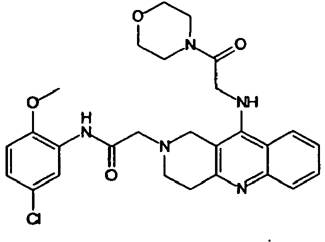 | N-(5-Chloro-2-methoxy-phenyl)-2-[10-(2-morpholin-4-yl-2-oxo-ethylamino)-3,4-dihydro-1H-benzo[b][1,6]naphthyri din-2-yl]-acetamide |
| **15** | 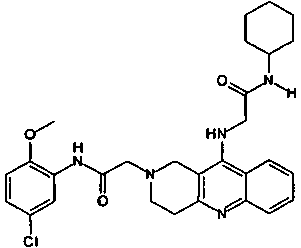 | N-(5-Chloro-2-methoxy-phenyl)-2-[10-(cyclohexylcarbamoyl methyl-amino)-3,4-dihydro-1H-benzo[b][1,6]naphthyri din-2-yl]-acetamide |
| **16** | 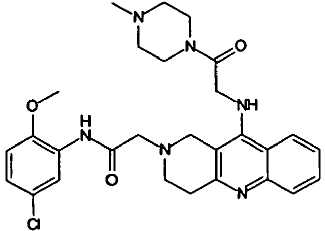 | N-(5-Chloro-2-methoxy-phenyl)-2-{10-[2-(4-methylpiperazin-1-yl)-2-oxo-ethylamino]-3,4-dihydro-1H-benzo[b][1,6]naphthyri din-2-yl}-acetamide |
| **17** | 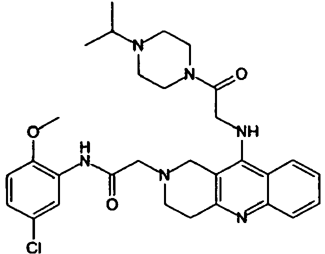 | N-(5-Chloro-2-methoxy-phenyl)-2-{10-[2-(4-isopropyl-piperazin-1-yl)-2-oxo-ethylamino]-3,4-dihydro-1H-benzo[b][1,6]naphthyri din-2-yl}-acetamide |
| **18** | 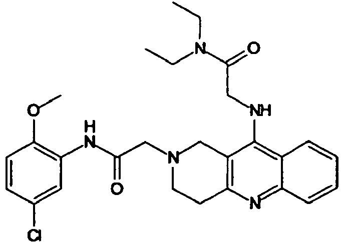 | N-(5-Chloro-2-methoxy-phenyl)-2-[10-(diethylcarbamoylmet hyl-amino)-3,4-dihydro-1H-benzo[b][1,6]naphthyri din-2-yl]-acetamide |
| **19** | 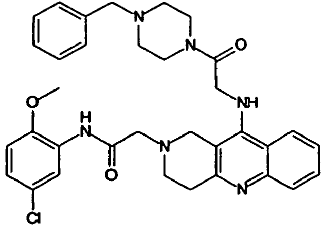 | 2-{10-[2-(4-Benzyl-piperazin-1-yl)-2-oxo-ethylamino]-3,4-dihydro-1H-benzo[b][1,6]naphthyri din-2-yl}-N-(5-chloro-2-methoxy-phenyl)-acetamide |
| **20** | 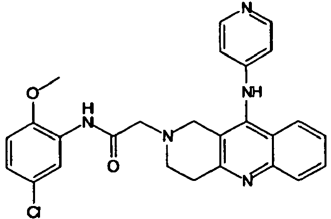 | N-(5-Chloro-2-methoxy-phenyl)-2-[10-(pyridin-4-ylamino)-3,4-dihydro-1H-benzo[b][1,6]naphthyri din-2-yl]-acetamide |
| **21** | 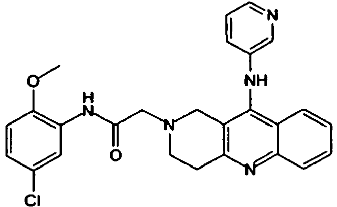 | N-(5-Chloro-2-methoxy-phenyl)-2-[10-(pyridin-3-ylamino)-3,4-dihydro-1H-benzo[b][1,6]naphthyri din-2-yl]-acetamide |
| **22** | 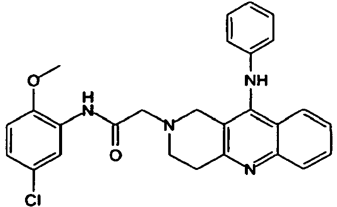 | N-(5-Chloro-2-methoxy-phenyl)-2-(10-phenylamino-3,4-dihydro-1H-benzo[b][1,6]naphthyri din-2-yl)-acetamide |
| **23** | 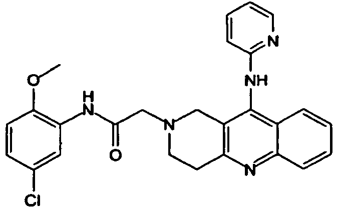 | N-(5-Chloro-2-methoxy-phenyl)-2-[10-(pyridin-2-ylamino)-3,4-dihydro-1H-benzo[b][1,6]naphthyri din-2-yl]-acetamide |
| **24** | 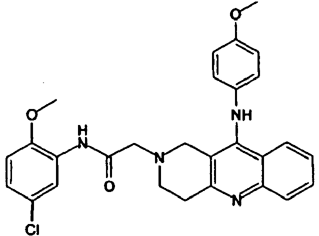 | N-(5-Chloro-2-methoxy-phenyl)-2-[10-(4-methoxy-phenylamino)-3,4-dihydro-1H-benzo[b][1,6]naphthyri din-2-yl]-acetamide |
sowie ihre pharmazeutisch verwendbaren Salze, Solvate und Stereoisomere, einschließlich deren Mischungen in allen Verhältnissen.

4. Verfahren zur Herstellung von Verbindungen der Formel (I) nach einem oder mehreren der Ansprüche 1 bis 3 sowie ihrer pharmazeutisch verwendbaren Salze, Solvate und Stereoisomere, **dadurch gekennzeichnet, dass** man eine Verbindung der Formel (II) 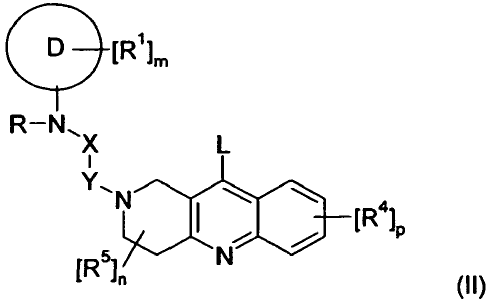 worin D, X, Y, R, R¹, R⁴, R⁵, m, n und p die in Anspruch 1 oder 2 angegebenen Bedeutungen haben und L ein Halogen, Tosylat, Mesylat oder Triflat ist,
mit einer Verbindung der Formel (III)
H-NR²R³ (III)
worin R², R³ die in Anspruch 1 oder 2 angegebenen Bedeutungen haben, umsetzt,
und/oder eine Base oder Säure der resultierenden Verbindungen der Formel (I) gemäß Anspruch 1 in eines ihrer Salze umwandelt,
oder
**dadurch gekennzeichnet, dass** man eine Verbindung der Formel (IV) 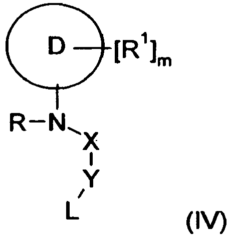 worin D, X, Y, R, R¹ und m die in Anspruch 1 oder 2 angegebenen Bedeutungen haben und L ein Halogen, Tosylat, Mesylat, Triflat oder eine freie oder reaktionsfähig abgewandelte OH-Gruppe, wie z.B. einen aktivierten Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy), darstellt,
mit einer Verbindung der Formel (V) 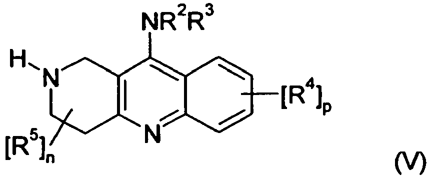 worin R², R³, R⁴, R⁵, n und p die in Anspruch 1 oder 2 angegebenen Bedeutungen haben, umsetzt,
und/oder eine Base oder Säure der resultierenden Verbindungen der Formel (I) gemäß Anspruch 1 in eines ihrer Salze umwandelt,
oder
**dadurch gekennzeichnet, dass** man eine Verbindung der Formel (VI) 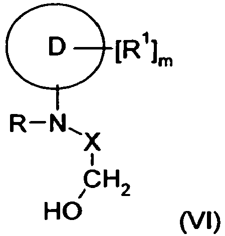 worin D, X, R, R¹ und m die in Anspruch 1 oder 2 angegebenen Bedeutungen haben,
zunächst mit einem Carbonylierungsmittel, bspw.1,1'-Carbonyldiimidazol, Phosgen, Diphosgen, Triphosgen, Harnstoff und Dialkylcarbonat, bevorzugt 1,1'-Carbonyldiimidazol, umsetzt,
und dann mit einer Verbindung der Formel (V) 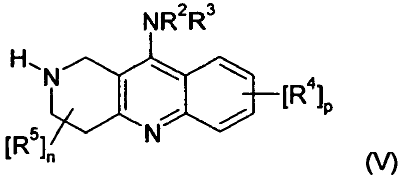 worin R², R³, R⁴, R⁵, n und p die in Anspruch 1 oder 2 angegebenen Bedeutungen haben, reagiert,
und/oder eine Base oder Säure der resultierenden Verbindungen der Formel (I) gemäß Anspruch 1 in eines ihrer Salze umwandelt.

5. Arzneimittel, enthaltend mindestens eine Verbindung der Formel (I) nach einem oder mehreren der Ansprüche 1 bis 3 und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

6. Arzneimittel, enthaltend mindestens eine Verbindung der Formel (I) nach einem oder mehreren der Ansprüche 1 bis 3 und/oder ihre pharmazeutisch verwendbaren Salze, Solvate und Stereoisomere, einschließlich deren Mischungen in allen Verhältnissen, zur Verwendung in der Behandlung und/oder Prophylaxe von physiologischen und/oder pathophysiologischen Zuständen, bei denen die Hemmung, Regulierung und/oder Modulation der Phosphodiesterase bzw. Lysophospholipase Autotaxin eine Rolle spielt.

7. Arzneimittel, enthaltend mindestens eine Verbindung der Formel (I) nach einem oder mehreren der Ansprüche 1 bis 3 und/oder ihre pharmazeutisch verwendbaren Salze, Solvate und Stereoisomere, einschließlich deren Mischungen in allen Verhältnissen, zur Verwendung in der Behandlung und/oder Prophylaxe von Krebs, Tumoren, malignen Tumoren, benignen Tumoren, festen Tumoren, Sarkoma, Karzinoma, hyperproliferativen Krankheiten, Karzinoiden, Ewing Sarkoma, Kaposi Sarkoma, Gehirntumoren, Tumoren ausgehend vom Gehirn und/oder Nervensystem und/oder Hirnhäuten, Glioma, Glioblastoma, Neuroblastoma, Magenkrebs, Nierenkrebs, Nierenzellkarzinoma, Prostatakrebs, Prostatakarzinoma, Bindegewebstumoren, Weichteilsarkoma, Bauchspeicheldrüsentumoren, Lebertumoren, Kopftumoren, Halstumoren, Kehlkopfkrebs, Speiseröhrenkrebs, Schilddrüsenkrebs, Osteosarkoma, Retinoblastoma, Thymusdrüsenkrebs, Hodenkrebs, Lungenkrebs, Lungenadenokarzinoma, kleinzellige Lungenkarzinoma, Bronchialkarzinoma, Brustkrebs, Mammakarzinoma, Darmkrebs, Kolorektaltumoren, Kolonkarzinoma, Rektumkarzinoma, gynäkologischen Tumoren, Eierstocktumoren, Gebärmutterkrebs, Gebärmutterhalskrebs, Gebärmutterhalskarzinoma, Korpuskarzinoma, Endometriumkarzinoma, Harnblasenkrebs, Urogenitaltraktkrebs, Blasenkrebs, Hautkrebs, Epitheltumoren, Plattenepithelkarzinoma, Basalioma, Spinalioma, Melanoma, intraokulären Melanoma, Leukämien, Monozytenleukämien, chronischen Leukämien, chronisch myelotischen Leukämien, chronisch lymphatischen Leukämien, akuten Leukämien, akuten myelotischen Leukämien, akuten lymphatischen Leukämien, Lymphomen, Angiogenese, Arteriosklerose, Atherosklerose, Augenerkrankungen, Uveititis, choroidale Neovascularisation, diabetische Retinopathie, Autoimmunerkrankungen, Entzündungskrankheiten, Asthma, chronisch obstruktive Lungenerkrankung (COPD), chronisch-entzündlichen Darmerkrankungen (Inflammatory Bowel Disease, IBD), Arthritis, Osteoporose, Osteoarthritis, Gicht, Gichtarthritis, Rheumatoide Spondylitis, allergische Rhinitis, Psoriasis, neurodegenerativen Erkrankungen, Restenose, Wundheilung, Transplantatabstoßung, Autoimmunenteropathie, Autoimmunhepatitis, Autoimmun-Polyendokrinopathie-Candidiasis-Ektodermaldystrophie-Syndrom Typ I (APECED), Bullöses Pemphigoid, Chronischer Gastritis, Churg-Strauss-Syndrom, Colitis ulcerosa, Dermatomyositis, Diabetes mellitus Typ 1, Dermatitis herpetiformis Duhring, Epidermolysis bullosa acquisita, Glomerulonephritis, Goodpasture-Syndrom, Guillain-Barre-Syndrom, Hashimoto-Thyreoiditis, Lichen sclerosus, Linearer IgA-Dermatose, Lupus erythematodes, Mikroskopischer Polyangütis, Morbus Adamantiades-Behçet, Morbus Basedow, Morbus Bechterew, Morbus Crohn, Multipler Sklerose, Myasthenia gravis, PANDAS (Pediatric Autoimmune Neuropsychiatric Disorders Associated with Streptococcal Infections), entzündlicher Becken-Krankheit (pelvic inflammatory disease, PID), Pemphigus foliaceus, Pemphigus seborrhoicus, Pemphigus vulgaris, Polychondritis, Polymyositis, Rheumatischem Fieber, Rheumatoider Arthritis, SAPHO-Syndrom, Sarkoidose (Morbus Boeck), Sjögren-Syndrom, Sklerodermie, Stiff-Man-Syndrom, Sympathischer Ophthalmie, Systemischen Lupus erythematodes, Vasculitis allergica, Vitiligo, Wegenerschen Granulomatose und/oder Zöliakie.

8. Arzneimittel nach einem oder mehreren der Ansprüche 5 bis 7, wobei das Arzneimittel mindestens eine zusätzliche pharmakologisch aktive Substanz umfasst.

9. Arzneimittel nach einem oder mehreren der Ansprüche 5 bis 7, wobei das Arzneimittel vor und/oder während und/oder nach der Behandlung mit mindestens einer zusätzlichen pharmakologisch aktiven Substanz verabreicht wird.

10. Pharmazeutische Zusammensetzung umfassend eine therapeutisch wirksame Menge mindestens einer Verbindung nach einem oder mehreren der Ansprüche 1 bis 3.

11. Pharmazeutische Zusammensetzung nach Anspruch 10 zusätzlich umfassend mindestens eine weitere Verbindung ausgewählt aus der Gruppe bestehend aus physiologisch verträglichen Träger- und/oder Hilfsstoffen und/oder einer zusätzlichen pharmakologisch aktiven Substanz, die nicht einer Verbindung nach einem oder mehreren der Ansprüche 1 bis 3 entspricht.

12. Kit umfassend eine therapeutisch wirksame Menge mindestens einer Verbindung nach einem oder mehreren der Ansprüche 1 bis 3 und/oder mindestens einer pharmazeutischen Zusammensetzung nach einem oder mehreren der Ansprüche 10 bis 11 und eine therapeutisch wirksame Menge mindestens einer zusätzlichen pharmakologisch aktiven Substanz, die nicht einer Verbindung nach einem oder mehreren der Ansprüche 1 bis 3 entspricht.

## Claims

1. Compounds of the formula (I) 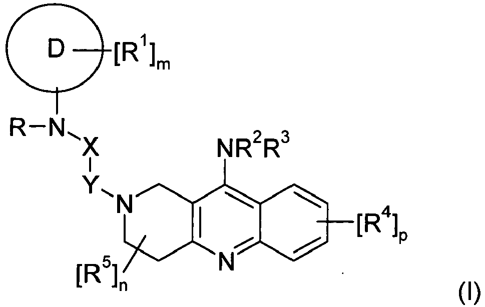 in which:
D denotes Ar or Het,
Ar denotes unsubstituted or mono- or polysubstituted phenyl, indanyl, naphthyl or biphenyl,
Het denotes a mono- or bicyclic saturated, partially unsaturated or aromatic heterocycle having 1, 2, 3 or 4 N, O and/or S atoms, which may be unsubstituted or mono- or polysubstituted,
X, Y are each, independently of one another, absent or denote -CH₂-, -(CH₂)₂-, -C(O)-, -CHOH- or -CH₂OC(O)-, where only one of the radicals X or Y may be absent,
R in each case, independently of one another, denotes H, A, Cyc, (CH₂)_{q}Ar or (CH₂)_{q}Het and may be mono- or polysubstituted by R⁶, where in A and Cyc, the C chain and C ring respectively may also be interrupted by O,
R¹ in each case, independently of one another, denotes R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phenyl and may be mono- or polysubstituted by R⁶,
R², R³ each, independently of one another, denote R, where R² and R³ may alternatively together also form Cyc or Het, each of which may in turn be mono- or polysubstituted by R⁶,
R⁴ in each case, independently of one another, denotes R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phenyl and may be mono- or polysubstituted by R⁶,
R⁵ in each case, independently of one another, denotes R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phenyl and may be mono- or polysubstituted by R⁶,
R⁶ in each case, independently of one another, denotes R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phenyl and, so long as a substitution is chemically possible, may be mono- or polysubstituted by R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phenyl,
A denotes linear or branched alkyl having 1, 2, 3, 4, 5, 6, 7 or 8 C atoms, in which 1, 2, 3, 4, 5, 6 or 7 H atoms may be replaced by OR, CN, NRR, F and/or CI and/or in which one or two non-adjacent CH₂ groups may be replaced by O, NH, S, SO, SO₂ and/or by CH=CH groups,
Cyc denotes cyclic alkyl having 3, 4, 5, 6, 7, 8, 9, 10, 11, 12, 13 or 14 C atoms,
m denotes 0, 1, 2, 3, 4 or 5,
n denotes 0, 1, 2 or 3,
p denotes 0, 1, 2, 3 or 4,
q denotes 0, 1, or 2,
and pharmaceutically usable salts, solvates and stereoisomers thereof, including mixtures thereof in all ratios.

2. Compounds according to Claim 1 in which
D denotes Ar,
Ar denotes unsubstituted or mono- or polysubstituted phenyl, but preferably denotes mono- or polysubstituted phenyl,
X, Y each, independently of one another, denote -CH₂-, -C(O)- or -CH₂OC(O)-, X is preferably = -C(O)- and Y is preferably = -CH₂- or -CH₂OC(O)-,
R¹ in each case, independently of one another, denotes F, CI, OA or OCH₃,
R², R³ each, independently of one another, denote H, Ar, Ar monosubstituted by OA, Het, Het monosubstituted by A, CH₂-Het, A, A monosubstituted by OH or by NRR or by CO-NRR or by Het or by CO-R, or each, independently of one another, denote 1-methylpiperidin-4-yl, 2-hydroxyethyl, 2-dimethylaminoethyl, methylcarbamoylmethyl, dimethylcarbamoylmethyl, 1 H-benzimidazol-2-ylmethyl, 2-oxo-2-pyrrolidin-1-yl-ethyl, 2-oxo-2-piperidin-1-ylethyl, 2-morpholin-4-yl-2-oxoethyl, cyclohexylcarbamoylmethyl, 2-(4-methylpiperazin-1-yl)-2-oxoethyl, 2-(4-isopropylpiperazin-1-yl)-2-oxoethyl, diethylcarbamoylmethyl, 2-(4-benzylpiperazin-1-yl)-2-oxoethyl, 3-oxo-3-piperidin-1-ylpropyl, pyridin-4-yl, pyridin-3-yl, phenyl, pyridin-2-yl or 4-methoxyphenyl, where R² and R³ may alternatively together also form 4-(2-hydroxyethyl)piperazin-1-yl,
m denotes 2,
n denotes 0,
p denotes 0,
q denotes 0 or 1,
Het, R, R⁶, A and Cyc have the meanings indicated in Claim 1,
and pharmaceutically usable salts, solvates and stereoisomers thereof, including mixtures thereof in all ratios.

3. Compounds according to one or more of Claims 1 to 2, selected from the group consisting of:
| **Compound No.** | **Chemical Structure** | **Chemical Name** |
|---|---|---|
| **1** | 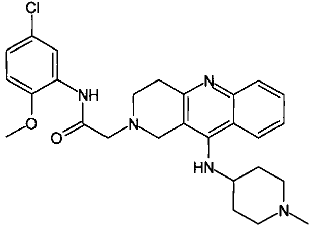 | N-(5-Chloro-2-methoxyphenyl)-2-[10-(1-methylpiperidin-4-ylamino)-3,4-dihydro-1H-benzo[b]-1,6-naphthyridin-2-yl]-acetamide |
| **2** | 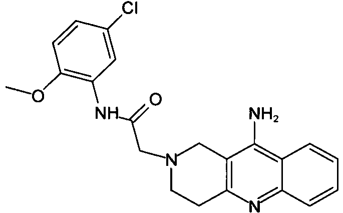 | 2-(10-Amino-3,4-dihydro-1H-benzo[b]-1,6-naphthyridin-2-yl)-N-(5-chloro-2-methoxyphenyl)-acetamide |
| **3** | 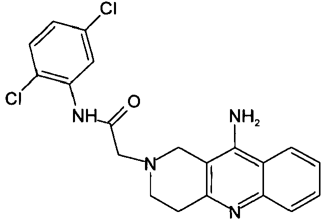 | 2-(10-Amino-3,4-dihydro-1H-benzo[b]-1,6-naphthyridin-2-yl)-N-(2,5-dichloro-phenyl)acetamide |
| **4** | 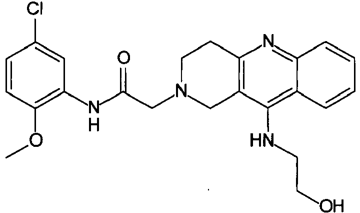 | N-(5-Chloro-2-methoxyphenyl)-2-[10-(2-hydroxyethyl-amino)-3,4-dihydro-1H-benzo[b]-1,6-naphthyridin-2-yl]-acetamide |
| **5** | 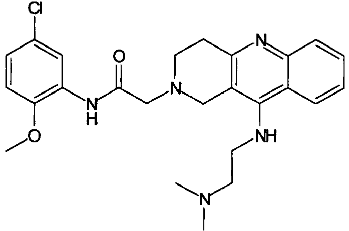 | N-(5-Chloro-2-methoxyphenyl)-2-[10-(2-dimethylamino-ethylamino)-3,4-dihydro-1H-benzo[b]-1,6-naphthyridin-2-yl]-acetamide |
| **6** | 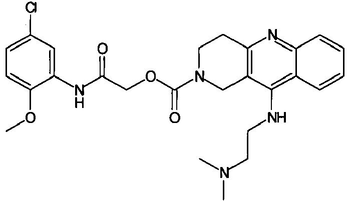 | 10-(2-Dimethylamino-ethylamino)-3,4-dihydro-1H-benzo[b]-1,6-naphthyridine-2-carboxylic acid (5-chloro-2-methoxy-phenylcarbamoyl)-methyl ester |
| **7** | 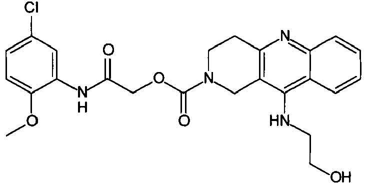 | 10-(2-Hydroxy-ethylamino)-3,4-dihydro-1H-benzo[b]-1,6-naphthyridine-2-carboxylic acid (5-chloro-2-methoxy-phenylcarbamoyl)-methyl ester |
| **8** | 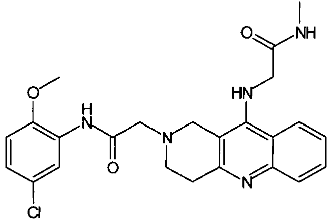 | N-(5-Chloro-2-methoxyphenyl)-2-[10-(methylcarbamoyl-methylamino)-3,4-dihydro-1H-benzo[b]-1,6-naphthyridin-2-yl]-acetamide |
| **9** | 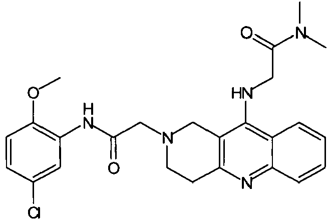 | N-(5-Chloro-2-methoxyphenyl)-2-[10-(dimethylcarbamoyl-methylamino)-3,4-dihydro-1H-benzo[b]-1,6-naphthyridin-2-yl]-acetamide |
| **10** | 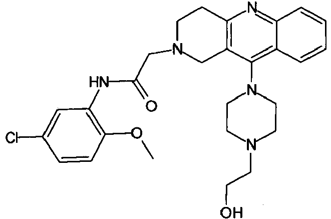 | N-(5-Chloro-2-methoxyphenyl)-2-{10-[4-(2-hydroxy-ethyl)piperazin-1-yl]-3,4-dihydro-1H-benzo-[b]-1,6-naphthyridin-2-yl}acetamide |
| **11** | 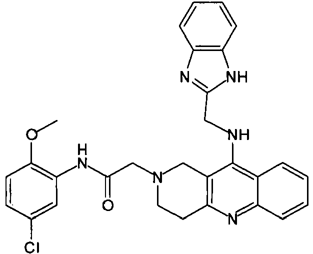 | 2-{10-[(1H-Benzimidazol-2-ylmethyl)amino]-3,4-dihydro-1H-benzo[b]-1,6-naphthyridin-2-yl}-N-(5-chloro-2-methoxyphenyl)-acetamide |
| **12** | 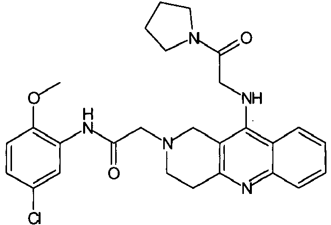 | N-(5-Chloro-2-methoxyphenyl)-2-[10-(2-oxo-2-pyrrolidin-1-ylethylamino)-3,4-dihydro-1H-benzo[b]-1,6-naphthyridin-2-yl]-acetamide |
| **13** | 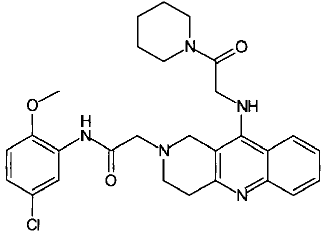 | N-(5-Chloro-2-methoxyphenyl)-2-[10-(2-oxo-2-piperidin-1-ylethylamino)-3,4-dihydro-1H-benzo[b]-1,6-naphthyridin-2-yl]-acetamide |
| **14** | 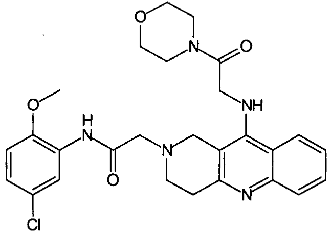 | N-(5-Chloro-2-methoxyphenyl)-2-[10-(2-morpholin-4-yl-2-oxoethylamino)-3,4-dihydro-1H-benzo[b]-1,6-naphthyridin-2-yl]-acetamide |
| **15** | 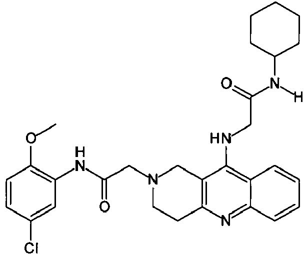 | N-(5-Chloro-2-methoxyphenyl)-2-[10-(cyclohexylcarbamoyl-methylamino)-3,4-dihydro-1H-benzo[b]-1,6-naphthyridin-2-yl]-acetamide |
| **16** | 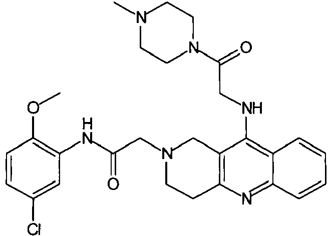 | N-(5-Chloro-2-methoxyphenyl)-2-{10-[2-(4-methyl-piperazin-1-yl)-2-oxo-ethylamino]-3,4-dihydro-1H-benzo[b]-1,6-naphthyridin-2-yl}-acetamide |
| **17** | 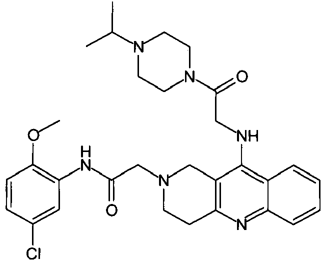 | N-(5-Chloro-2-methoxyphenyl)-2-{10-[2-(4-isopropyl-piperazin-1-yl)-2-oxo-ethylamino]-3,4-dihydro-1H-benzo[b]-1,6-naphthyridin-2-yl}-acetamide |
| **18** | 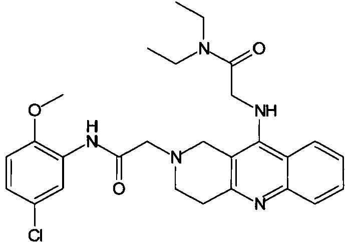 | N-(5-Chloro-2-methoxyphenyl)-2-[10-(diethylcarbamoyl-methylamino)-3,4-dihydro-1H-benzo[b]-1,6-naphthyridin-2-yl]-acetamide |
| **19** | 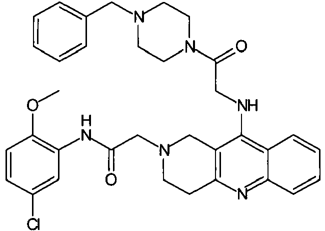 | 2-{10-[2-(4-Benzyl-piperazin-1-yl)-2-oxo-ethylamino]-3,4-dihydro-1H-benzo[b]-1,6-naphthyridin-2-yl}-N-(5-chloro-2-methoxyphenyl)-acetamide |
| **20** | 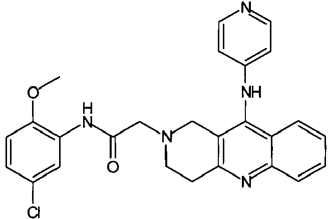 | N-(5-Chloro-2-methoxyphenyl)-2-[10-(pyridin-4-ylamino)-3,4-dihydro-1H-benzo-[b]-1,6-naphthyridin-2-yl]acetamide |
| **21** | 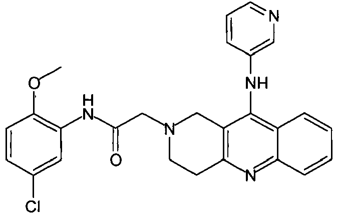 | N-(5-Chloro-2-methoxyphenyl)-2-[10-(pyridin-3-ylamino)-3,4-dihydro-1H-benzo[b]-1,6-naphthyridin-2-yl]-acetamide |
| **22** | 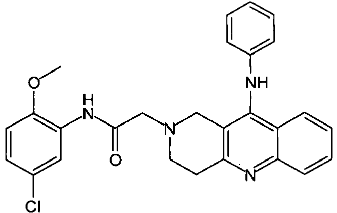 | N-(5-Chloro-2-methoxyphenyl)-2-(10-phenylamino-3,4-dihydro-1H-benzo[b]-1,6-naphthyridin-2-yl)-acetamide |
| **23** | 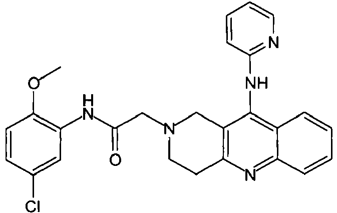 | N-(5-Chloro-2-methoxyphenyl)-2-[10-(pyridin-2-ylamino)-3,4-dihydro-1H-benzo[b]-1,6-naph-thyridin-2-yl]-acetamide |
| **24** | 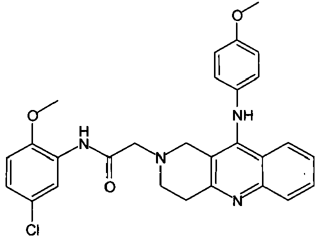 | N-(5-Chloro-2-methoxyphenyl)-2-[10-(4-methoxyphenyl-amino)-3,4-dihydro-1H-benzo[b]-1,6-naphthyridin-2-yl]-acetamide |
and pharmaceutically usable salts, solvates and stereoisomers thereof, including mixtures thereof in all ratios.

4. Process for the preparation of compounds of the formula (I) according to one or more of Claims 1 to 3 and pharmaceutically usable salts, solvates and stereoisomers thereof, **characterised in that** a compound of the formula (II) 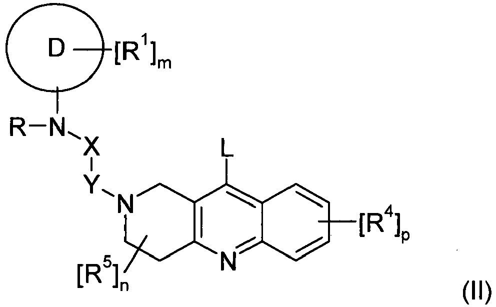 in which D, X, Y, R, R¹, R⁴, R⁵, m, n and p have the meanings indicated in Claim 1 or 2 and L is a halogen, tosylate, mesylate or triflate,
is reacted with a compound of the formula (III)
H-NR²R³ (III)
in which R², R³ have the meanings indicated in Claim 1 or 2,
and/or a base or acid of the resultant compounds of the formula (I) according to Claim 1 is converted into one of its salts,
or
**characterised in that** a compound of the formula (IV) 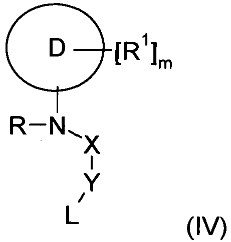 in which D, X, Y, R, R¹ and m have the meanings indicated in Claim 1 or 2 and L represents a halogen, tosylate, mesylate, triflate or a free or reactively modified OH group, such as, for example, an activated ester, an imidazolide or alkylsulfonyloxy having 1-6 C atoms (preferably methylsulfonyloxy or trifluoromethylsulfonyloxy) or arylsulfonyloxy having 6-10 C atoms (preferably phenyl- or p-tolylsulfonyloxy),
is reacted with a compound of the formula (V) 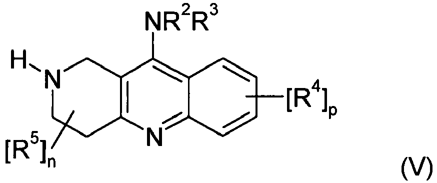 in which R², R³, R⁴, R⁵, n and p have the meanings indicated in Claim 1 or 2,
and/or a base or acid of the resultant compounds of the formula (I) according to Claim 1 is converted into one of its salts,
or
**characterised in that** a compound of the formula (VI) 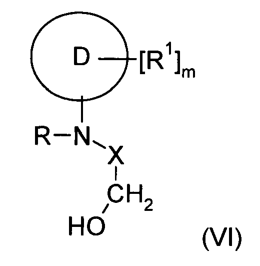 in which D, X, R, R¹ and m have the meanings indicated in Claim 1 or 2,
is firstly reacted with a carbonylation agent, for example 1,1'-carbonyl-diimidazole, phosgene, diphosgene, triphosgene, urea and dialkyl carbonate, preferably 1,1'-carbonyldiimidazole,
and then reacted with a compound of the formula (V) 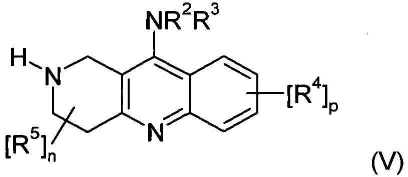 in which R², R³, R⁴, R⁵, n and p have the meanings indicated in Claim 1 or 2,
and/or a base or acid of the resultant compounds of the formula (I) according to Claim 1 is converted into one of its salts.

5. Medicament comprising at least one compound of the formula (I) according to one or more of Claims 1 to 3 and/or pharmaceutically usable salts, solvates and stereoisomers thereof, including mixtures thereof in all ratios, and optionally excipients and/or adjuvants.

6. Medicament comprising at least one compound of the formula (I) according to one or more of Claims 1 to 3 and/or pharmaceutically usable salts, solvates and stereoisomers thereof, including mixtures thereof in all ratios, for use in the treatment and/or prophylaxis of physiological and/or pathophysiological conditions in which the inhibition, regulation and/or modulation of phosphodiesterase or lysophospholipase autotaxin plays a role.

7. Medicament comprising at least one compound of the formula (I) according to one or more of Claims 1 to 3 and/or pharmaceutically usable salts, solvates and stereoisomers thereof, including mixtures thereof in all ratios, for use in the treatment and/or prophylaxis of cancer, tumours, malignant tumours, benign tumours, solid tumours, sarcoma, carcinoma, hyperproliferative diseases, carcinoids, Ewing's sarcoma, Kaposi's sarcoma, brain tumours, tumours originating from the brain and/or nervous system and/or meninges, glioma, glioblastoma, neuroblastoma, stomach cancer, kidney cancer, kidney cell carcinomas, prostate cancer, prostate carcinoma, connective tissue tumours, soft tissue sarcoma, pancreas tumours, liver tumours, head tumours, neck tumours, laryngeal cancer, oesophageal cancer, thyroid cancer, osteosarcoma, retinoblastomas, thymus gland cancer, testicular cancer, lung cancer, lung adenocarcinomas, small-cell lung carcinoma, bronchial carcinoma, breast cancer, mammary carcinoma, intestinal cancer, colorectal tumours, colon carcinoma, rectal carcinoma, gynaecological tumours, ovarian tumours, uterine cancer, cervical cancer, cervical carcinoma, corpus carcinoma, endometrial carcinoma, urinary bladder cancer, urogenital tract cancer, bladder cancer, skin cancer, epithelial tumours, squamous epithelial carcinoma, basaliomas, spinalioma, melanoma, intraocular melanomas, leukaemias, monocytic leukaemias, chronic leukaemias, chronic myeloid leukaemias, chronic lymphatic leukaemias, acute leukaemias, acute myeloid leukaemias, acute lymphatic leukaemias, lymphomas, angiogenesis, arteriosclerosis, atherosclerosis, eye diseases, uveitis, choroidal neovascularisation, diabetic retinopathy, autoimmune diseases, inflammatory diseases, asthma, chronic obstructive pulmonary disease (COPD), chronic inflammatory bowel disease (IBD), arthritis, osteoporosis, osteoarthritis, gout, gouty arthritis, rheumatoid spondylitis, allergic rhinitis, psoriasis, neurodegenerative diseases, restenosis, wound healing, transplant rejection, autoimmune enteropathy, autoimmune hepatitis, autoimmune polyendocrinopathy candidiasis ectodermal dystrophy syndrome type I (APECED), bullous pemphigoid, chronic gastritis, Churg-Strauss syndrome, ulcerative colitis, dermatomyositis, type 1 diabetes mellitus, Duhring's dermatitis herpetiformis, epidermolysis bullosa acquisita, glomerulonephritis, Goodpasture's syndrome, Guillain-Barré syndrome, Hashimoto's thyroiditis, lichen sclerosus, linear IgA dermatosis, lupus erythematosus, microscopic polyangiitis, Adama ntiades-Behçet's disease, Basedow's disease, Bechterew's disease, Crohn's disease, multiple sclerosis, myasthenia gravis, PANDAS (paediatric autoimmune neuropsychiatric disorders associated with streptococcal infections), pelvic inflammatory disease (PID), pemphigus foliaceus, pemphigus seborrhoicus, pemphigus vulgaris, polychondritis, polymyositis, rheumatic fever, rheumatoid arthritis, SAPHO syndrome, sarcoidosis (Boeck's disease), Sjögren's syndrome, dermatosclerosis, stiff person syndrome, sympathetic ophthalmia, systemic lupus erythematosus, allergic vasculitis, vitiligo, Wegener's granulomatosis and/or coeliac disease.

8. Medicament according to one or more of Claims 5 to 7, where the medicament comprises at least one additional pharmacologically active substance.

9. Medicament according to one or more of Claims 5 to 7, where the medicament is administered before and/or during and/or after the treatment with at least one additional pharmacologically active substance.

10. Pharmaceutical composition comprising a therapeutically effective amount of at least one compound according to one or more of Claims 1 to 3.

11. Pharmaceutical composition according to Claim 10 additionally comprising at least one further compound selected from the group consisting of physiologically tolerated excipients and/or adjuvants and/or an additional pharmacologically active substance which does not correspond to a compound according to one or more of Claims 1 to 3.

12. Kit comprising a therapeutically effective amount of at least one compound according to one or more of Claims 1 to 3 and/or at least one pharmaceutical composition according to one or more of Claims 10 to 11 and a therapeutically effective amount of at least one additional pharmacologically active substance which does not correspond to a compound according to one or more of Claims 1 to 3.

## Revendications

1. Composés de la formule (I) : 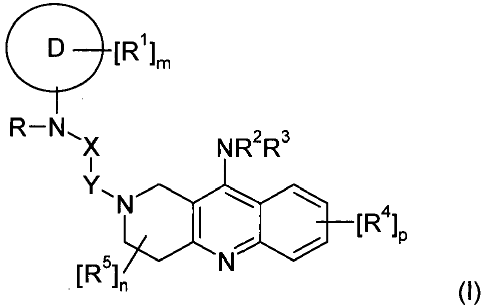 dans laquelle :
D représente Ar ou Het,
Ar représente phényle, indanyle, naphtyle ou biphényle non substitué, monosubstitué ou polysubstitué,
Het représente un hétérocycle saturé, partiellement non saturé ou aromatique monocyclique ou bicyclique comportant 1, 2, 3 ou 4 atomes de N, de O et/ou de S, lequel peut être non substitué ou monosubstitué ou polysubstitué,
X, Y sont, chacun indépendamment de l'autre, absents ou représentent -CH₂-, -(CH₂)₂-, -C(O)-, -CHOH- ou -CH₂OC(O)-, où seulement l'un des radicaux X ou Y peut être absent,
R représente, dans chaque cas indépendamment des autres, H, A, Cyc, (CH₂)_{q}Ar ou (CH₂)_{q}Het et peut être monosubstitué ou polysubstitué par R⁶, où dans A et Cyc, la chaîne C et le cycle C peuvent également être respectivement interrompus par O,
R¹ représente, dans chaque cas indépendamment des autres, R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phényle et peut être monosubstitué ou polysubstitué par R⁶,
R², R³ représentent, chacun indépendamment de l'autre, R, où R² et R³ peuvent alternativement également former ensemble Cyc ou Het, dont chacun peut à son tour être monosubstitué ou polysubstitué par R⁶,
R⁴ représente, dans chaque cas indépendamment des autres, R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phényle et peut être monosubstitué ou polysubstitué par R⁶,
R⁵ représente, dans chaque cas indépendamment des autres, R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phényle et peut être monosubstitué ou polysubstitué par R⁶,
R⁶ représente, dans chaque cas indépendamment des autres, R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phényle et, pour autant qu'une substitution est chimiquement possible, peut être monosubstitué ou polysubstitué par R, F, Cl, Br, I, OH, =O, CN, NO₂, NRR, NHC(O)R, NHSO₂R, OR, C(O)R, C(O)NRR, CF₃, OCF₃, SCF₃, SO₂CH₃, SO₃R, SO₂R, SO₂NR, SR, OA, A, phényle,
A représente alkyle linéaire ou ramifié comportant 1, 2, 3, 4, 5, 6, 7 ou 8 atome(s) de C, où 1, 2, 3, 4, 5, 6 ou 7 atome(s) de H peut/peuvent être remplacé(s) par OR, CN, NRR, F et/ou Cl et/ou où un ou deux groupe(s) CH₂ non adjacents peut/ peuvent être remplacé(s) par O, NH, S, SO, SO₂ et/ou par des groupes CH=CH,
Cyc représente alkyle cyclique comportant 3, 4, 5, 6, 7, 8, 9, 10, 11, 12, 13 ou 14 atomes de C,
m représente 0, 1, 2, 3, 4 ou 5,
n représente 0, 1, 2 ou 3,
p représente 0, 1, 2, 3 ou 4,
q représente 0, 1, ou 2,
et leurs sels, solvates et stéréo-isomères pharmaceutiquement utilisables, y compris des mélanges afférents selon tous rapports.

2. Composés selon la revendication 1, dans lesquels :
D représente Ar,
Ar représente phényle non substitué ou monosubstitué ou polysubstitué mais de façon préférable, représente phényle monosubstitué ou polysubstitué,
X, Y représentent, chacun indépendamment de l'autre, -CH₂-, -C(O)- ou -CH₂OC(O)-, X est de façon préférable = -C(O)- et Y est de façon préférable = -CH₂- ou -CH₂OC(O)-,
R¹ représente, dans chaque cas indépendamment des autres, F, CI, OA ou OCH₃,
R², R³ représentent, chacun indépendamment de l'autre, H, Ar, Ar monosubstitué par OA, Het, Het monosubstitué par A, CH₂-Het, A, A monosubstitué par OH ou par NRR ou par CO-NRR ou par Het ou par CO-R, ou représentent, chacun indépendamment de l'autre, 1-méthylpipéridin-4-yle, 2-hydroxyéthyle, 2-diméthylaminoéthyle, méthylcarbamoylméthyle, diméthylcarbamoylméthyle, 1 H-benzimidazol-2-ylméthyle, 2-oxo-2-pyrrolidin-1-yléthyle, 2-oxo-2-pipéridin-1-yléthyle, 2-morpholin-4-yl-2-oxoéthyle, cyclohexylcarbamoylméthyle, 2-(4-méthylpipérazin-1-yl)-2-oxoéthyle, 2-(4-isopropylpipérazin-1-yl)-2-oxoéthyle, diéthylcarbamoylméthyle, 2-(4-benzylpipérazin-1-yl)-2-oxoéthyle, 3-oxo-3-pipéridin-1-ylpropyle, pyridin-4-yle, pyridin-3-yle, phényle, pyridin-2-yle ou 4-méthoxyphényle, où R² et R³ peuvent alternativement également former ensemble 4-(2-hydroxyéthyl)pipérazin-1-yle,
m représente 2,
n représente 0,
p représente 0,
q représente 0 ou 1,
Het, R, R⁶, A et Cyc présentent les significations indiquées selon la revendication 1
et leurs sels, solvates et stéréo-isomères pharmaceutiquement utilisables, y compris des mélanges afférents selon tous rapports.

3. Composés selon une ou plusieurs des revendications 1 et 2, choisis parmi le groupe constitué par :
| **No. de Composé** | **Structure Chimique** | **Nom Chimique** |
|---|---|---|
| **1** | 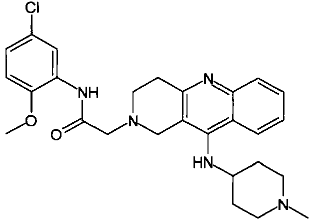 | N-(5-Chloro-2-méthoxyphényl)-2-[10-(1-méthylpipéridin-4-ylamino)-3,4-dihydro-1H-benzo[b]-1,6-naphtyridin-2-yl]-acétamide |
| **2** | 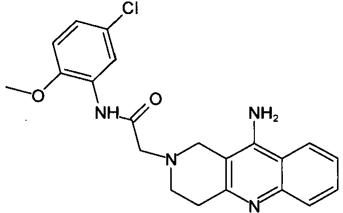 | 2-(10-Amino-3,4-dihydro-1H-benzo[b]-1,6-naphtyridin-2-yl)-N-(5-chloro-2-méthoxyphényl)-acétamide |
| **3** | 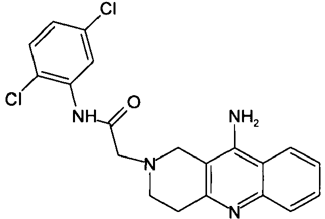 | 2-(10-Amino-3,4-dihydro-1H-benzo[b]-1,6-naphtyridin-2-yl)-N-(2,5-dichloro-phényl)-acétamide |
| **4** | 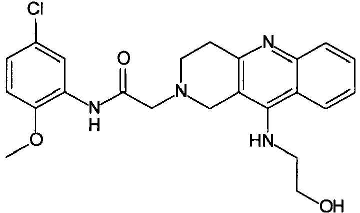 | N-(5-Chloro-2-méthoxyphényl)-2-[10-(2-hydroxyéthyl-amino)-3,4-dihydro-1H-benzo[b]-1,6-naphtyridin-2-yl]-acétamide |
| **5** | 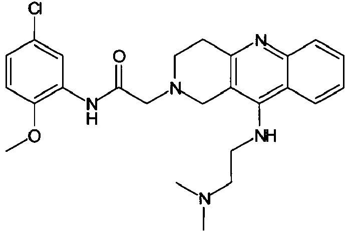 | N-(5-Chloro-2-méthoxyphényl)-2-[10-(2-diméthylamino-éthylamino)-3,4-dihydro-1H-benzo[b]-1,6-naphtyridin-2-yl]-acétamide |
| **6** | 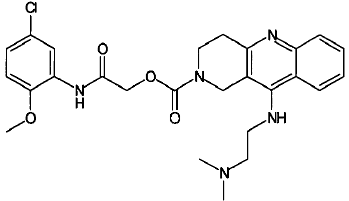 | 10-(2-Diméthylamino-éthylamino)-3,4-dihydro-1H-benzo[b]-1,6-naphtyridin-2-carboxylate de (5-chloro-2-méthoxy-phénylcarbamoyl)-méthyle |
| **7** | 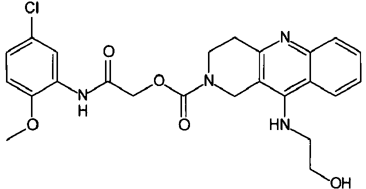 | 10-(2-Hydroxy-éthylamino)-3,4-dihydro-1H-benzo[b]-1,6-naphtyridin-2-carboxylate de (5-chloro-2-méthoxy-phénylcarbamoyl)-méthyle |
| **8** | 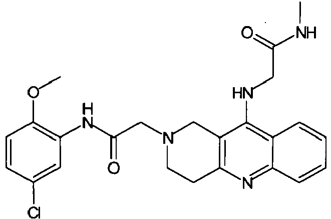 | N-(5-Chloro-2-méthoxyphényl)-2-[10-(méthylcarbamoyl-méthylamino)-3,4-dihydro-1H-benzo[b]-1,6-naphtyridin-2-yl]-acétamide |
| **9** | 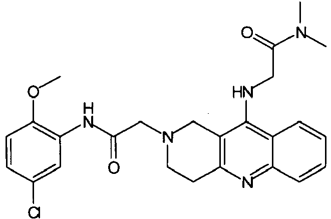 | N-(5-Chloro-2-méthoxyphényl)-2-[10-(diméthylcarbamoyl-méthylamino)-3,4-dihydro-1H-benzo[b]-1,6-naphtyridin-2-yl]-acétamide |
| **10** | 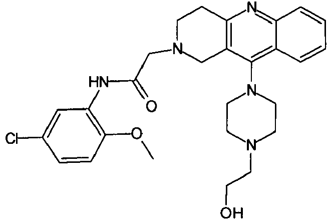 | N-(5-Chloro-2-méthoxyphényl)-2-{10-[4-(2-hydroxy-éthyl)pipérazin-1-yl]-3,4-dihydro-1H-benzo-[b]-1,6-naphtyridin-2-yl}acétamide |
| **11** | 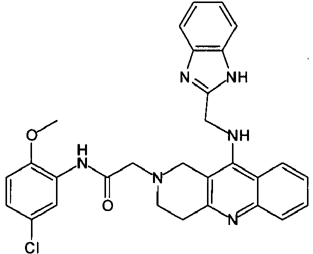 | 2-{10-[(1H-Benzimidazol-2-ylméthyl)amino]-3,4-dihydro-1H-benzo[b]-1,6-naphtyridin-2-yl}-N-(5-chloro-2-méthoxyphényl)-acétamide |
| **12** | 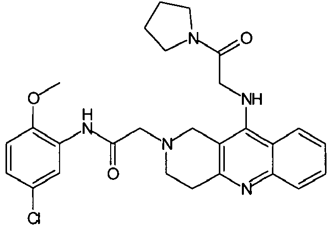 | N-(5-Chloro-2-méthoxyphényl)-2-[10-(2-oxo-2-pyrrolid in-1-yléthylamino)-3,4-dihydro-1H-benzo[b]-1,6-naphtyridin-2-yl]-acétamide |
| **13** | 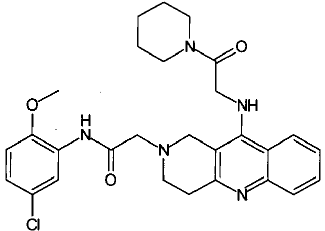 | N-(5-Chloro-2-méthoxyphényl)-2-[10-(2-oxo-2-pipéridin-1-yléthylamino)-3,4-dihydro-1H-benzo[b]-1,6-naphtyridin-2-yl]-aceétamide |
| **14** | 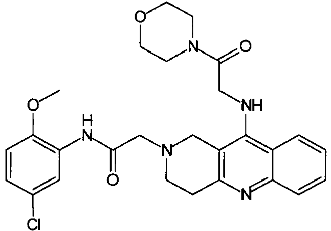 | N-(5-Chloro-2-méthoxyphényl)-2-[10-(2-morpholin-4-yl-2-oxoéthylamino)-3,4-dihydro-1H-benzo[b]-1,6-naphtyridin-2-yl]-acétamide |
| **15** | 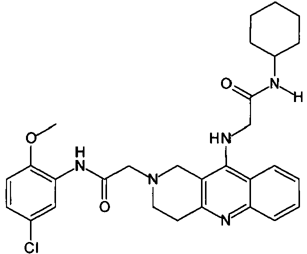 | N-(5-Chloro-2-méthoxyphényl)-2-[10-(cyclohexylcarbamoyl-méthylamino)-3,4-dihydro-1H-benzo[b]-1,6-naphtyridin-2-yl]acétamide |
| **16** | 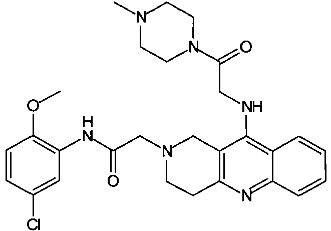 | N-(5-Chloro-2-méthoxyphényl)-2-{10-[2-(4-méthyl-pipérazin-1-yl)-2-oxo-éthylamino]-3,4-dihydro-1H-benzo[b]-1,6-naphtyridin-2-yl}-acétamide |
| **17** | 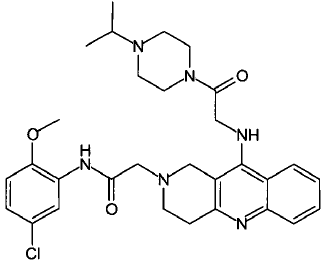 | N-(5-Chloro-2-méthoxyphényl)-2-{10-[2-(4-isopropyl-pipérazin-1-yl)-2-oxo-éthylamino]-3,4-dihydro-1H-benzo[b]-1,6-naphtyridin-2-yl}-acétamide |
| **18** | 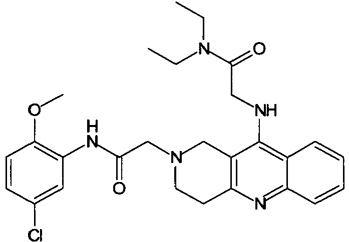 | N-(5-Chloro-2-méthoxyphényl)-2-[10-(diéthylcarbamoyl-méthylamino)-3,4-dihydro-1H-benzo[b]-1,6-naphtyridin-2-yl]-acétamide |
| **19** | 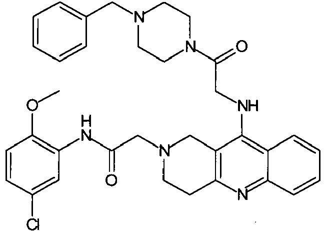 | 2-{10-[2-(4-Benzyl-pipérazin-1-yl)-2-oxo-éthylamino]-3,4-dihydro-1H-benzo[b]-1,6-naphtyridin-2-yl}-N-(5-chloro-2-méthoxyphényl)-acétamide |
| **20** | 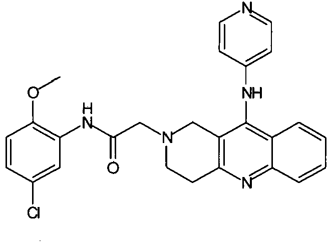 | N-(5-Chloro-2-méthoxyphényl)-2-[10-(pyridin-4-ylamino)-3,4-dihydro-1H-benzo[b]-1,6-naphtyridin-2-yl]-acétamide |
| **21** | 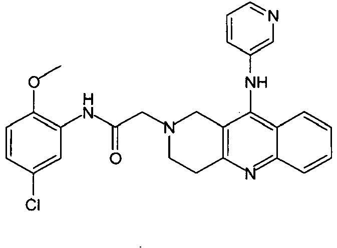 | N-(5-Chloro-2-méthoxyphényl)-2-[10-(pyridin-3-ylamino)-3,4-dihydro-1H-benzo[b]-1,6-naphtyridin-2-yl]acétamide |
| **22** | 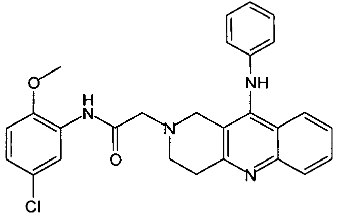 | N-(5-Chloro-2-méthoxyphényl)-2-(10-phénylamino-3,4-dihydro-1H-benzo[b]-1,6-naphtyridin-2-yl)-acétamide |
| **23** | 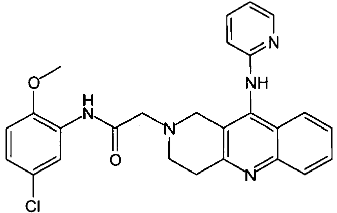 | N-(5-Chloro-2-méthoxyphényl)-2-[10-(pyridin-2-ylamino)-3,4-dihydro-1H-benzo[b]-1,6-naphtyridin-2-yl]acétamide |
| **24** | 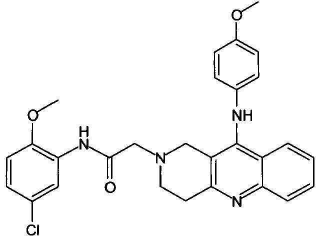 | N-(5-Chloro-2-méthoxyphényl)-2-[10-(4-méthoxyphényl-amino)-3,4-dihydro-1H-benzo[b]-1,6-naphtyridin-2-yl]-acétamide |
et leurs sels, solvates et stéréo-isomères pharmaceutiquement utilisables, y compris des mélanges afférents selon tous rapports.

4. Procédé pour la préparation de composés de la formule (I) selon une ou plusieurs des revendications 1 à 3 et de leurs sels, solvates et stéréoisomères pharmaceutiquement utilisables, **caractérisé en ce qu'**un composé de la formule (II) : 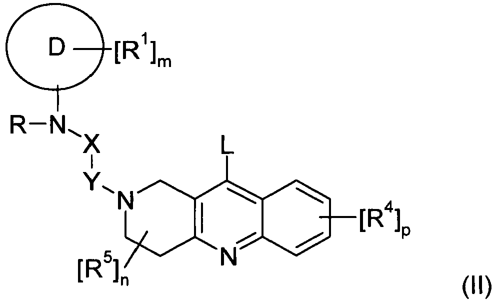 dans laquelle D, X, Y, R, R¹, R⁴, R⁵, m, n et p présentent les significations indiquées selon la revendication 1 ou 2 et L est un halogène, un tosylate, un mésylate ou un triflate,
est amené à réagir avec un composé de la formule (III) :
H-NR²R³ (III)
dans laquelle R², R³ présentent les significations indiquées selon la revendication 1 ou 2,
et/ou une base ou un acide des composés résultants de la formule (I) selon la revendication 1 est converti€ selon l'un de ses sels,
ou
**caractérisé en ce qu'**un composé de la formule (IV) : 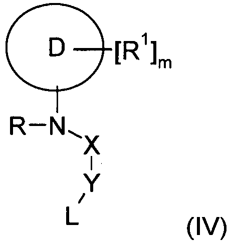 dans laquelle D, X, Y, R, R¹ et m présentent les significations indiquées selon la revendication 1 ou 2 et L représente un halogène, un tosylate, un mésylate, un triflate ou un groupe OH libre ou modifié par réaction, tel que, par exemple, un ester activé, un imidazolide ou un alkylsulfonyloxy comportant 1-6 atome(s) de C (de façon préférable méthylsulfonyloxy ou trifluorométhylsulfonyloxy) ou un arylsulfonyloxy comportant 6-10 atomes de C (de façon préférable phényl- ou p-tolyl-sulfonyloxy),
est amené à réagir avec un composé de la formule (V) : 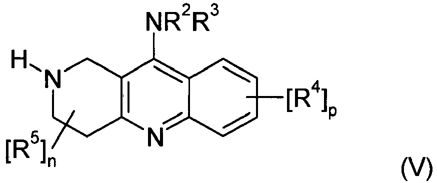 dans laquelle R², R³, R⁴, R⁵, n et p présentent les significations indiquées selon la revendication 1 ou 2,
et/ou une base ou un acide des composés résultants de la formule (I) selon la revendication 1 est converti(e) selon l'un de ses sels,
ou
**caractérisé en ce qu'**un composé de la formule (VI) : 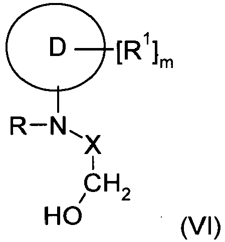 dans laquelle D, X, R, R¹ et m présentent les significations indiquées selon la revendication 1 ou 2,
est en premier lieu amené à réagir avec un agent de carbonylation, par exemple 1,1'-carbonyldiimidazole, phosgène, diphosgène, triphosgène, urée et carbonate de dialkyle, de façon préférable 1,1'-carbonyldiimidazole,
puis est amené à réagir avec un composé de la formule (V) : 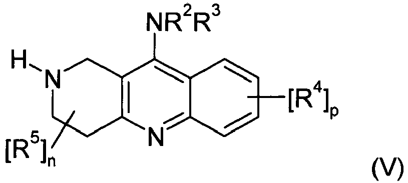 dans laquelle R², R³, R⁴, R⁵, n et p présentent les significations indiquées selon la revendication 1 ou 2,
et/ou une base ou un acide des composés résultants de la formule (I) selon la revendication 1 est converti(e) selon l'un de ses sels.

5. Médicament comprenant au moins un composé de la formule (I) selon une ou plusieurs des revendications 1 à 3 et/ou et leurs sels, solvates et stéréo-isomères pharmaceutiquement utilisables, y compris des mélanges afférents selon tous rapports, et en option des excipients et/ou adjuvants.

6. Médicament comprenant au moins un composé de la formule (I) selon une ou plusieurs des revendications 1 à 3 et/ou leurs sels, solvates et stéréo-isomères pharmaceutiquement utilisables, y compris des mélanges afférents selon tous rapports, pour une utilisation au niveau du traitement et/ou de la prophylaxie de conditions physiologiques et/ou pathophysiologiques au niveau desquelles l'inhibition, la régulation et/ou la modulation de la phosphodiestérase ou de la lysophospholipase autotaxine joue(nt) un rôle.

7. Médicament comprenant au moins un composé de la formule (I) selon une ou plusieurs des revendications 1 à 3 et/ou leurs sels, solvates et stéréo-isomères pharmaceutiquement utilisables, y compris des mélanges afférents selon tous rapports, pour une utilisation au niveau du traitement et/ou de la prophylaxie du cancers, des tumeurs, des tumeurs malignes, des tumeurs bénignes, des tumeurs solides, des sarcomes, des carcinomes, des maladies hyper-prolifératives, des carcinoïdes, du sarcome d'Ewing, du sarcome de Kaposi, des tumeurs du cerveau, des tumeurs prenant leur origine au niveau du cerveau et/ou du système nerveux et/ou des méninges, des gliomes, des glioblastomes, des neuroblastomes, du cancer de l'estomac, du cancer du rein, des carcinomes des cellules du rein, du cancer de la prostate, du carcinome de la prostate, des tumeurs des tissus connectifs, du sarcome des tissus mous, des tumeurs du pancréas, des tumeurs du foie, des tumeurs de la tête, des tumeurs du cou, du cancer du larynx, du cancer de l'oesophage, du cancer de la thyroïde, de l'ostéosarcome, des rétinoblastomes, du cancer de la glande thymus, du cancer des testicules, du cancer des poumons, des adénocarcinomes du poumon, du carcinome du poumon à petites cellules, du carcinome des bronches, du cancer du sein, du carcinome mammaire, du cancer de l'intestin, des tumeurs colorectales, du carcinome du côlon, du carcinome rectal, des tumeurs gynécologiques, des tumeurs ovariennes, du cancer de l'utérus, du cancer cervical, du carcinome cervical, du carcinome du corps, du carcinome endométrial, du cancer de la vessie urinaire, du cancer des voies urogénitales, du cancer de la vessie, du cancer de la des basaliomes, du spinaliome, du mélanome, des mélanomes intraoculaires, des leucémies, des leucémies monocytaires, des leucémies chroniques, des leucémies myéloïdes chroniques, des leucémies lymphatiques chroniques, des leucémies aiguës, des leucémies myéloïdes aiguës, des leucémies lymphatiques aiguës, des lymphomes, de l'angiogénèse, de l'artériosclérose, de l'athérosclérose, des maladies des yeux, de l'uvéite, de la néovascularisation choroïdale, de la rétinopathie diabétique, des maladies auto-immunes, des maladies inflammatoires, de l'asthme, de la maladie pulmonaire obstructive chronique (COPD), de la maladie du bol inflammatoire chronique (IBD), de l'arthrite, de l'ostéoporose, de l'ostéo-arthrite, de la goutte, de l'arthrite gouteuse, de la spondylite rhumatoïde, de la rhinite allergique, du psoriasis, des maladies neurodégénératives, de la resténose, de la cicatrisation des plaies, du rejet des transplants, de l'entéropathie auto-immune, de l'hépatite auto-immune, du syndrome de polyendocrinopathie auto-immune de type I (syndrome APECED), de la pemphigoïde bulleuse, de la gastrite chronique, du syndrome de Churg-Strauss, de la colite ulcérative, de la dermatomyosite, du diabète sucré de type 1, de la dermatite herpétiforme de Duhring, de l'épidermolyse bulleuse acquise, de la glomérulonéphrite, du syndrome de Goodpasture, du syndrome de Guillain-Barré, de la thyroïdite de Hashimoto, du lichen scléreux, de la dermatose IgA linéaire, du lupus érythémateux, de la polyangéite microscopique, de la maladie de Adamantiades-Behçet, de la maladie de Basedow, de la maladie de Bechterew, de la maladie de Crohn, de la sclérose en plaques, de la myasthénie, des PANDAS (troubles neuropsychiatriques auto-immunes pédiatriques associés à des infections streptococcales), de la maladie inflammatoire pelvienne (PID), du pemphigus foliaceus, du pemphigus séborrhéique, du pemphigus vulgaire, de la polychondrite, de la polymyosite, de la fièvre rhumatique, de l'arthrite rhumatoïde, du syndrome de SAPHO, de la sarcoïdose (maladie de Boeck), du syndrome de Sjögren, de la der-matosclérose, du syndrome de l'homme raide, de l'ophtalmie sympathique, du lupus érythémateux systémique, de la vasculite allergique, du vitiligo, de la granulomatose de Wegener et/ou de la maladie coe-liaque.

8. Médicament selon une ou plusieurs des revendications 5 à 7, où le médicament comprend au moins une substance pharmacologiquement active additionnelle.

9. Médicament selon une ou plusieurs des revendications 5 à 7, où le médicament est administré avant et/ou pendant et/ou après le traitement à l'aide d'au moins une substance pharmacologiquement active additionnelle.

10. Composition pharmaceutique comprenant une quantité thérapeutiquement efficace d'au moins un composé selon une ou plusieurs des revendications 1 à 3.

11. Composition pharmaceutique selon la revendication 10, comprenant additionnellement au moins un autre composé choisi parmi le groupe constitué par des excipients et/ou des adjuvants physiologiquement tolérés et/ou une substance pharmacologiquement active additionnelle qui ne correspond pas à un composé selon une ou plusieurs des revendications 1 à 3.

12. Kit comprenant une quantité thérapeutiquement efficace d'au moins un composé selon une ou plusieurs des revendications 1 à 3 et/ou d'au moins une composition pharmaceutique selon une ou plusieurs des revendications 10 et 11 et une quantité thérapeutiquement efficace d'au moins une substance pharmacologiquement active additionnelle qui ne correspond pas à un composé selon une ou plusieurs des revendications 1 à 3
